(19) Europäisches Patentamt
European Patent Office
Office européen des brevets

(11) EP 4 582 420 A1

# (12) EUROPEAN PATENT APPLICATION

published in accordance with Art. 153(4) EPC

(43) Date of publication:
**09.07.2025 Bulletin 2025/28**

(21) Application number: **23890737.2**

(22) Date of filing: **13.11.2023**

(51) International Patent Classification (IPC):
***C07D 403/06*** *(2006.01)* ***A61K 31/4178*** *(2006.01)*
***A61P 35/00*** *(2006.01)*

(52) Cooperative Patent Classification (CPC):
**A61K 31/4178; A61K 31/675; A61P 11/00; A61P 35/00; A61P 35/02; C07D 403/06; C07F 9/6558**

(86) International application number:
**PCT/CN2023/131362**

(87) International publication number:
**WO 2024/104304 (23.05.2024 Gazette 2024/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.11.2022 CN 202211419976**

(71) Applicant: **Global Health Drug Discovery Institute**
**Beijing 100192 (CN)**

(72) Inventors:
- **LI, Zizhong**
  **Beijing 100192 (CN)**
- **WANG, Ting**
  **Beijing 100192 (CN)**
- **CHANG, Lei**
  **Beijing 100192 (CN)**
- **GAO, Hui**
  **Beijing 100192 (CN)**

(74) Representative: **Ipside**
**7-9 Allée Haussmann**
**33300 Bordeaux Cedex (FR)**

## (54) MDM2/MDMX DOUBLE-TARGET INHIBITOR COMPOUND, PRODRUG, PHARMACEUTICAL COMPOSITION, AND PREPARATION METHOD THEREFOR AND USE THEREOF

(57) The invention provides a compound shown in formula (I) and a prodrug thereof, which have a good MDM2 and/or MDMX inhibiting effect and may be used for treating MDM2 and/or MDMX-mediated symptoms and/or diseases such as neoplastic diseases and/or idiopathic pulmonary fibrosis and preparing drugs for such symptoms or diseases. In particular, a compound containing a phosphoric acid (phosphate) structure has higher solubility. As the prodrug, the compound can release an MDM2 and/or MDMX double-target inhibitor compound with high activity in an animal body, which solves the problem that such double-target inhibitors are hardly prepared into drugs.



Processed by Luminess, 75001 PARIS (FR)

## Description

The invention claims priority of a priori application filed with China National Intellectual Property Administration on 14, November, 2022 and titled "MDM2/MDMX double-target inhibitor compound, prodrug, pharmaceutical composition, and preparation method therefor and use thereof" with application No. 202211419976.3. The entire content of the priori application is incorporated into the invention by reference.

## Technical Field

The invention belongs to the field of medicines, and specifically relates to an MDM2/MDMX double-target inhibitor compound, a prodrug, a pharmaceutical composition, and a preparation method therefor and use thereof.

## Background

Members of murine double minute (MDM) family, MDM2 and MDMX, are genes closely related to occurrence and development of malignant tumors and primary negative regulators for p53. MDM2, the member of MDM family, is one of the strongest apoptosis inhibitory factors discovered so far. It is a ubiquitin-protein ligase taking part in identification of a target protein by ubiquitin, which results in that the target protein is degraded by proteasome. Its encoded protein may bind with p53 protein to degrade the p53 protein in a ubiquitylated manner and reduce its activity. The p53 protein, in return, binds with its gene promoter sequence to inhibit its transcriptional activity and reduce expression of MDM2, so as to form a negative feedback loop, so that the ability of the cell which is transformed, proliferated, and malignantly transformed is enhanced. The homologous protein MDMX of MDM2 is another important binding factor for p53, which is similar to MDM2 in inhibition function on p53 protein. The difference is that MDMX does not have E3 ligase activity.

p53 may be divided into two types: wild p53 and mutant p53. The wild p53 is a tumor suppressor gene which is studies mostly deeply at present, which may induce cell apoptosis rapidly and prevent generation of cells with a potential canceration probability, so as to effectively exert the cancer inhibition effect. The wild p53 accounts for about 50% of all cancers. In these cancers, overexpression of MDM2/X proteins may be observed in most cases. Based on 28 different types of tumors, analysis of about 4000 tumor samples shows that the MDM2 gene is amplified in about 7% of tumor samples averagely, with the amplitude of amplification of 2-10 times. Over 80% of liposarcoma has amplification of the MDM2 gene and overexpression of the protein. In addition, overexpression of MDM2 is also common in osteosarcoma, esophagus cancer, and breast cancer. In many human cancers, MDMX is overexpressed, including glioma, soft tissue sarcoma, melanoma, retinoblastoma, breast cancer, and hematological malignancies. Through comparative study of data published in the cancer genome atlas (TCGA), scientists may recognize the expression of MDMX in patients with different cancer types. It is worth noting that in AML patients, the expression of MDMX is considerably higher than the expression of MDMX in other most tumor types. It is worth noting that compared with other tumor cells, malignant tumors originated from the blood system also have highly expressed MDMX.

Studies show that in fibrotic lesion of human idiopathic pulmonary fibrosis (IPF) and a pulmonary fibrosis animal model of an aged mouse, the MDMX is overexpressed. The MDMX is a matrix stiffness regulated p53 endogenous inhibitor. Reduction of matrix stiffness will down-regulate expression of the MDMX, resulting in that p53 in a primary lung myofibroblast separated from an IPF patient is activated. Increase of p53 function activates a gene program which can sensitize the lung myofibroblast to a process of cell apoptosis and promote clearance of apoptotic lung myofibroblast by macrophages. Clearance of the MDMX in a fibrotic lung matrix by a targeted non-enzymatic cross-linking or gene knockout method may activate an MDMX-p53 channel, so as to eliminate pulmonary fibrosis of the mouse induced by agedness. These discoveries show that mechanically sensitive MDMX is a molecular target which has a therapeutic potential and fights against senescence-associated persistent pulmonary fibrosis.

At present, MDM2-p53 interacting small-molecule inhibitors under clinical studies are as follows: AMG232 (KRT-232) from Kartos, APG-115 from Ascentage Pharma, and DS-3032b from Daiichi Sankyo Company Limited are under clinical studies in different stages. These compounds have low affinity to the MDMX and only inhibit MDM2-p53 protein interaction. Since the MDMX has a compensation effect on the MDM2, when p53 is activated by using the MDM2 selective inhibitor, MDMX-p53 interaction will reduce the p53 activating effect generated due to MDM2 inhibition. This phenomenon may be a reason that the clinical efficacy of the MDM2 selective inhibitor is non-significant. So far, there have been no MDMX selective inhibitors with high activity. ALRN-6924 is a unique p53-MDM2/MDMX double-inhibitor entering clinical practice, which is a "binding peptide" capable of blocking the interaction between p53 and MDM2 and MDMX. Clinical study results show that its safety index is superior to that of the MDM2 selective inhibitor, but its micromole level cell activity limits clinical widespread application.

## Summary

To improve the above technical problem, the invention provides a compound shown in formula (I), and a raceme, a stereoisomer, a tautomer, an isotopic label, a solvate, a polymorph, a pharmaceutically acceptable salt or a prodrug compound thereof:

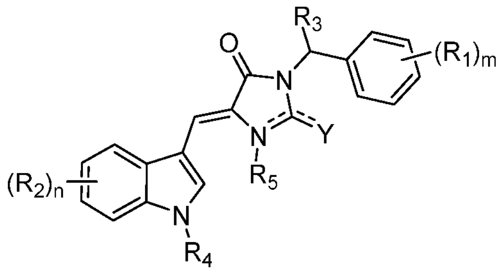

(I)

where

- $R_1$ is selected from H, halogen, CN, $C_{1-6}$ alkyl, $C_{2-6}$ alkenyl, $C_{2-6}$ alkynyl, $C_{1-6}$ alkoxyl, halogenated $C_{1-6}$ alkyl, and halogenated $C_{1-6}$ alkoxyl;
- $R_2$ is selected from H, $C_{1-6}$ alkyl, halogen, $C_{1-6}$ alkoxyl, $C_{1-6}$ alkylthio, halogenated $C_{1-6}$ alkyl, and halogenated $C_{1-6}$ alkoxyl;
- $R_3$ is selected from H, $R_{3a}$-O-$C_{1-6}$ alkyl, -CH(=O), and -C(=O)-NH-$R_{3b}$; $R_{3a}$ is selected from H or -P(=O)($OR_{31}$)($OR_{32}$); $R_{3b}$ is selected from the following radicals which are unsubstituted or optionally substituted with one, two, three or more $R_{3c}$: $C_{1-6}$ alkyl, $C_{3-8}$ cycloalkyl, and $C_{3-8}$ cycloalkyl-$C_{1-6}$ alkyl; $R_{3c}$ is selected from OH, $C_{1-6}$ alkyl-NH-, ($C_{1-6}$ alkyl)$_2$-N-, and -O-P(=O)($OR_{31}$)($OR_{32}$); and $R_{31}$ and $R_{32}$ are same or different, and are independently selected from H and $C_{1-6}$ alkyl;
- $R_4$ is selected from H and -P(=O)($OR_{41}$)($OR_{42}$); and $R_{41}$ and $R_{42}$ are same or different, and are independently selected from H and $C_{1-6}$ alkyl;
- $R_5$ is selected from H and -P(=O)($OR_{51}$)($OR_{52}$); and $R_{51}$ and $R_{52}$ are same or different, and are independently selected from H and $C_{1-6}$ alkyl;
- Y is O or $OR_6$; $R_6$ is selected from H and -P(=O)($OR_{61}$)($OR_{62}$); and $R_{61}$ and $R_{62}$ are same or different, and are independently selected from H and $C_{1-6}$ alkyl;
- 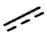 is selected from a single bond or a double bond;
- m is selected from 0, 1, 2, 3, 4 or 5; and
- n is selected from 0, 1, 2, 3 or 4.

According to an embodiment of the invention, $R_1$ is selected from H, halogen, CN, $C_{1-3}$ alkyl, $C_{2-3}$ alkenyl, $C_{1-3}$ alkoxyl, halogenated $C_{1-3}$ alkyl, and halogenated $C_{1-3}$ alkoxyl;

according to an embodiment of the invention, $R_1$ is selected from H, F, Cl, CN, acetenyl, methyl, ethyl, and propyl.
according to an embodiment of the invention, $R_2$ is selected from H, $C_{1-3}$ alkyl, halogen, $C_{1-3}$ alkoxyl, $C_{1-3}$ alkylthio, and halogenated $C_{1-3}$ alkoxyl;
according to an embodiment of the invention, $R_2$ is selected from H, F, Cl, Br, methyl, methoxyl, fluoromethoxyl, and methylthio.

According to an embodiment of the invention, $R_3$ is selected from H, $R_{3a}$-O-$C_{1-3}$ alkyl, -CH(=O), and -C(=O)-NH-$R_{3b}$; $R_{3a}$ is selected from H or -P(=O)(OH) (OH); $R_{3b}$ is selected from the following radicals which are unsubstituted or optionally substituted with one, two, three or more $R_{3c}$: $C_{1-3}$ alkyl, $C_{3-8}$ cycloalkyl, and $C_{3-8}$ cycloalkyl-$C_{1-3}$ alkyl; $R_{3c}$ is selected from OH, $C_{1-3}$ alkyl-NH-, ($C_{1-3}$ alkyl)$_2$-N-, and -OP(=O)(OH)(OH).

According to an embodiment of the invention, $R_3$ is selected from H, methyl,

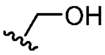, 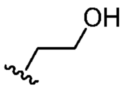, 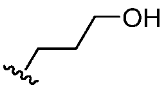, 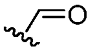, 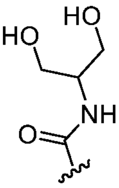, 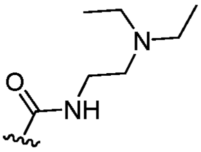,

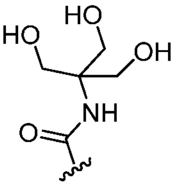, 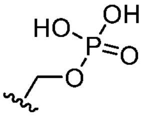, 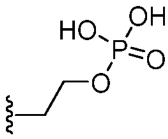, 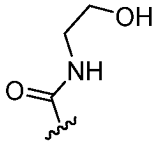, 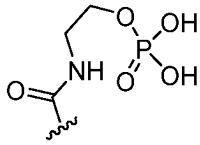,

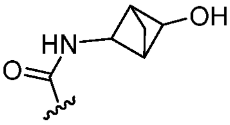, 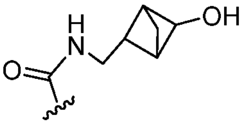, 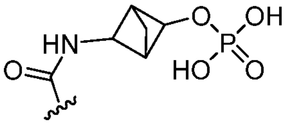, 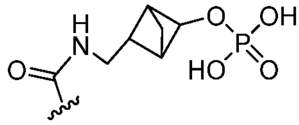,

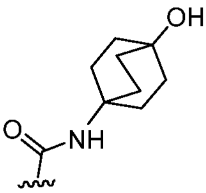, 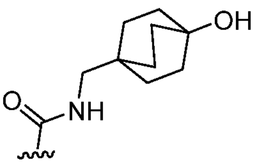, 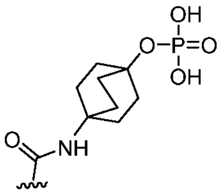, 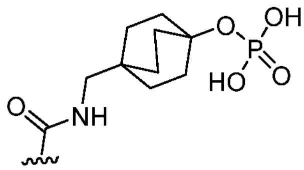,

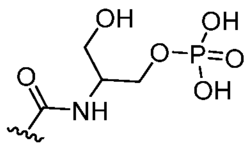, and 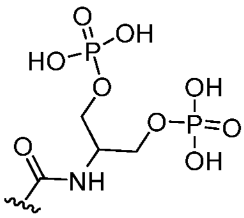;

for example, selected from

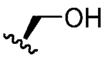,

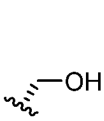, 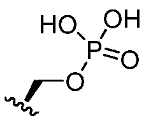, and 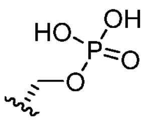.

According to an embodiment of the invention, $R_4$ is selected from H and - P(=O)($OR_{41}$)($OR_{42}$); and $R_{41}$ and $R_{42}$ are same or different, and are independently selected from H and $C_{1-3}$ alkyl;

according to an embodiment of the invention, $R_4$ selected from H and - P(=O)(OH)(OH);
according to an embodiment of the invention, $R_5$ is selected from H and - P(=O)($OR_{51}$)($OR_{52}$); and $R_{51}$ and $R_{52}$ are same or different, and are independently selected from H and $C_{1-3}$ alkyl;
according to an embodiment of the invention, $R_5$ selected from H and-P(=O)(OH)(OH);
according to an embodiment of the invention, Y is O or $OR_6$; $R_6$ is selected from H and -P(=O)($OR_{61}$)($OR_{62}$); and $R_{61}$ and $R_{62}$ are same or different, and are independently selected from H and $C_{1-3}$ alkyl;
according to an embodiment of the invention, $R_6$ selected from H and-P(=O)(OH)(OH);
according to an embodiment of the invention, m is selected from 1, 2 or 3.

According to an embodiment of the invention, n is selected from 1 or 2.

According to an embodiment of the invention, the prodrug of the compound shown in formula (I) has a structure shown in formula II:

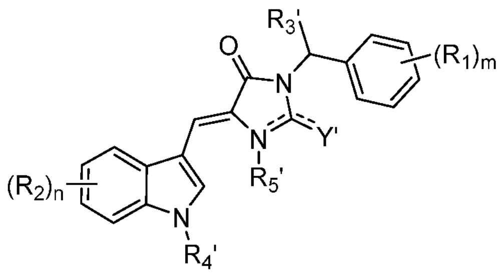

(II)

where $R_1$, $R_2$, m, and n independently have definitions described above;

$R_3$' has a definition of $R_3$;

$R_4$' is selected from H and -P(=O)($OR_{41}$' )($OR_{42}$' ); and $R_{41}$' and $R_{42}$' are same or different, and are independently selected from H and $C_{1-6}$ alkyl;

$R_5$' is selected from H and -P(=O)($OR_{51}$' )($OR_{52}$' ); and $R_{51}$' and $R_{52}$' are same or different, and are independently selected from H and $C_{1-6}$ alkyl;

Y' is O or $OR_6$' ; $R_6$' is selected from H and -P(=O)($OR_{61}$' ) ($OR_{62}$' ); and $R_{61}$' and $R_{62}$' are same or different, and are independently selected from H and $C_{1-6}$ alkyl; and

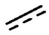 is selected from a single bond or a double bond.

According to the technical solution of the invention:

$R_3$' is, for example, selected from H, $R_{3a}$-O-$C_{1-6}$ alkyl, -CH(=O), and-C(=O)-NH-$R_{3b}$; $R_{3a}$ is selected from H or -P(=O)(OH)(OH); $R_{3b}$ is selected from the following radicals which are unsubstituted or optionally substituted with one, two, three or more $R_{3c}$: $C_{1-6}$ alkyl, $C_{3-8}$ cycloalkyl, and $C_{3-8}$ cycloalkyl-$C_{1-6}$ alkyl; $R_{3c}$ is selected from OH, $C_{1-6}$ alkyl-NH-, ($C_{1-6}$ alkyl)$_2$-N-, and -O-P(=O)(OH)(OH);

$R_4$' is selected from H and -P(=O)($OR_{41}$' )($OR_{42}$' ); and $R_{41}$' and $R_{42}$' are same or different, and are independently selected from H and $C_{1-3}$ alkyl; preferably, $R_4$' is selected from H and -P(=O)(OH)(OH);

$R_5$' is selected from H and -P(=O)($OR_{51}$' )($OR_{52}$' ); and $R_{51}$' and $R_{52}$' are same or different, and are independently selected from H and $C_{1-3}$ alkyl; preferably, $R_5$' is selected from H and -P(=O)(OH)(OH);

Y' is O or $OR_6$' ; $R_6$' is selected from H and -P(=O)($OR_{61}$' )($OR_{62}$' ); and $R_{61}$' and $R_{62}$' are same or different, and are independently selected from H and $C_{1-3}$ alkyl; preferably, $R_6$' is selected from H and -P(=O)(OH)(OH);

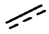 is selected from a single bond or a double bond; and

at least one of $R_4$' , $R_5$' , $R_6$' , $R_{3a}$, and $R_{3c}$ is -P(=O)(OH)(OH).

According to an embodiment of the invention, the compound is selected from the following structures:

| Number | Structure | Number | Structure |
|---|---|---|---|
| 1 | 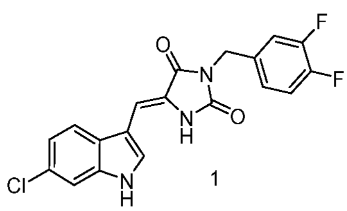 1 | 48 | 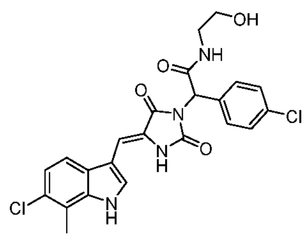 |

(continued)

| Number | Structure | Number | Structure |
|---|---|---|---|
| 2 | 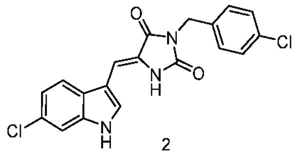 2 | 49 | 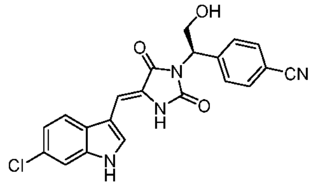 |
| 3 | 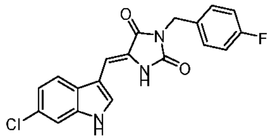 | 50 | 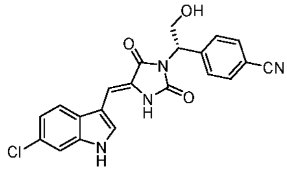 |
| 4 | 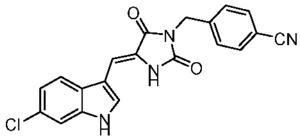 | 51 | 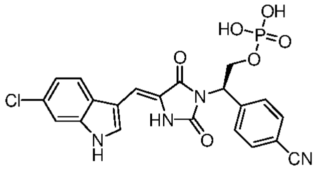 |
| 5 | 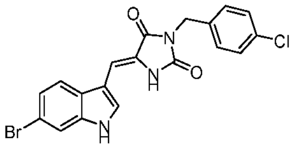 | 52 | 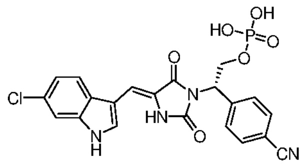 |
| 6 | 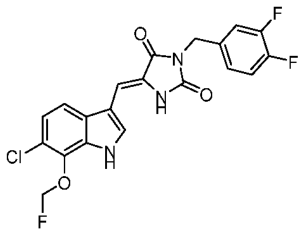 | 53 | 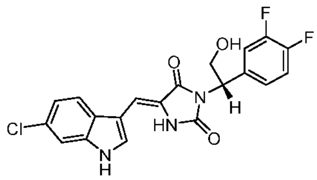 |
| 7 | 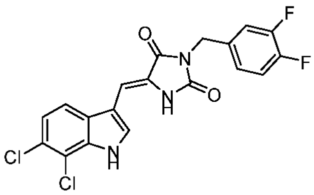 | 54 | 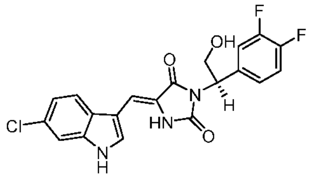 |
| 8 | 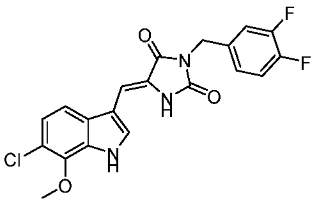 | 55 | 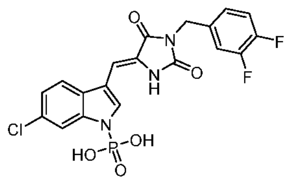 |
| 9 | 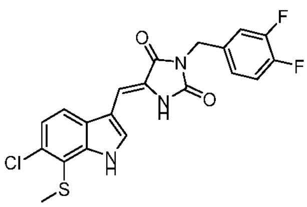 | 56 | 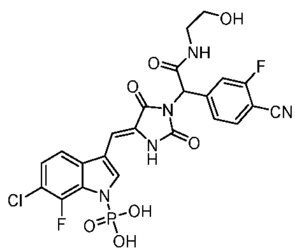 |

(continued)

| Number | Structure | Number | Structure |
|---|---|---|---|
| 10 | 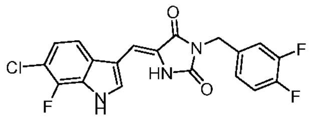 | 57 | 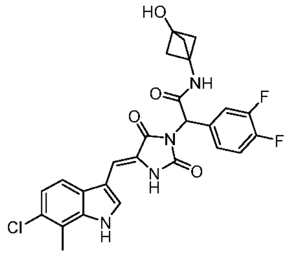 |
| 11 | 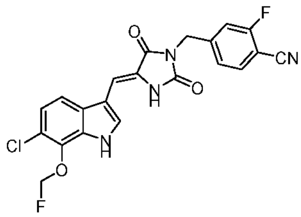 | 58 | 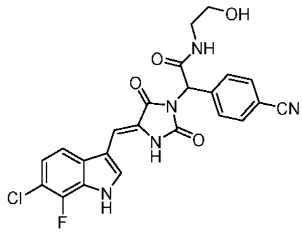 |
| 12 | 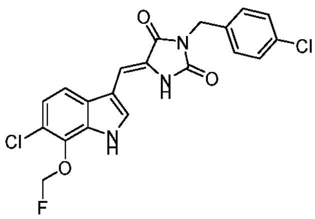 | 59 | 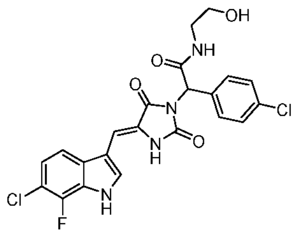 |
| 13 | 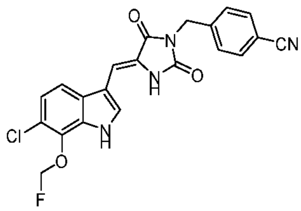 | 60 | 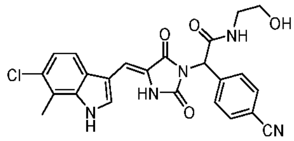 |
| 14 | 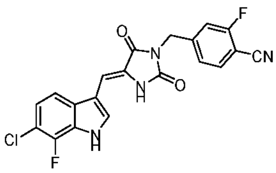 | 61 | 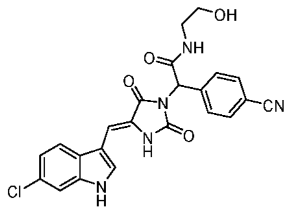 |
| 15 | 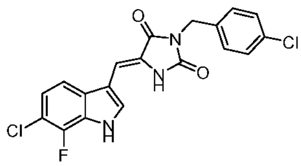 | 62 | 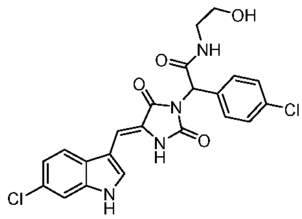 |
| 16 | 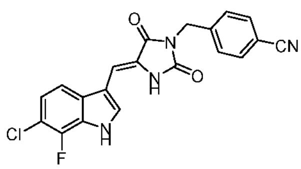 | 63 | 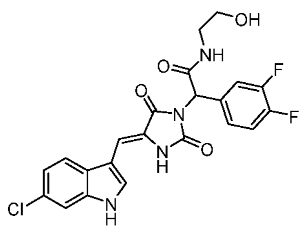 |

(continued)

| Number | Structure | Number | Structure |
|---|---|---|---|
| 17 | 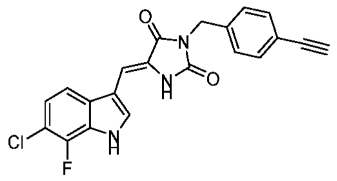 | 64 | 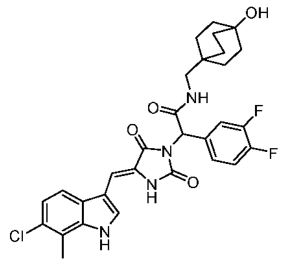 |
| 18 | 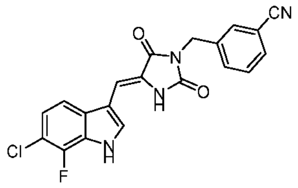 | 65 | 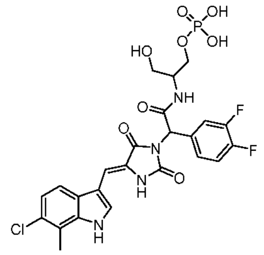 |
| 19 | 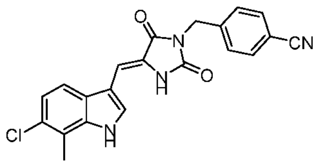 | 66 | 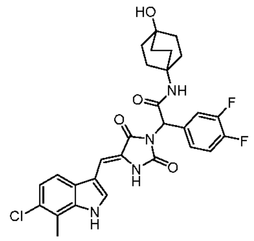 |
| 20 | 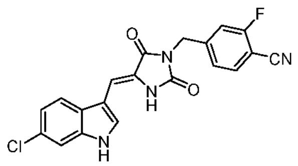 | 67 | 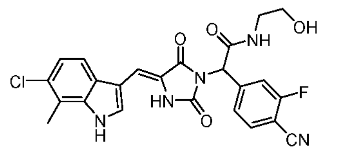 |
| 21 | 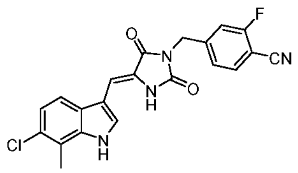 | 68 | 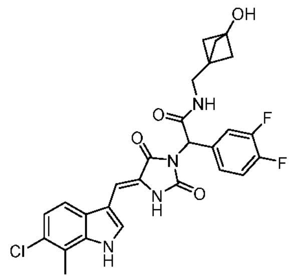 |
| 22 | 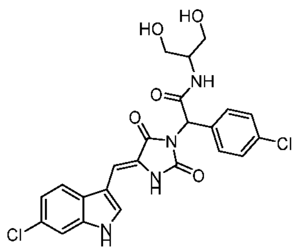 | 69 | 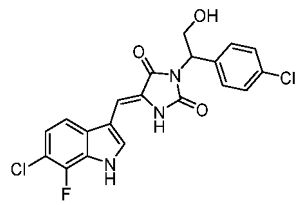 |

(continued)

| Number | Structure | Number | Structure |
|---|---|---|---|
| 23 | 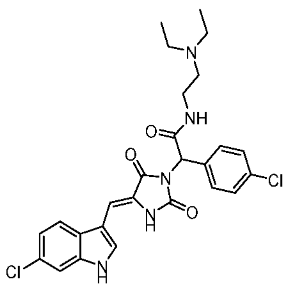 | 70 | 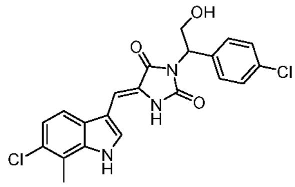 |
| 24 | 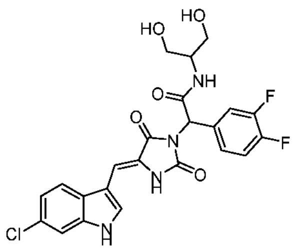 | 71 | 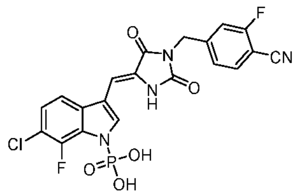 |
| 25 | 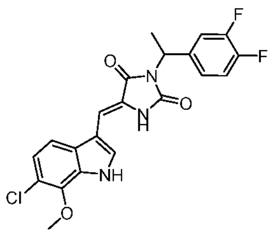 | 72 | 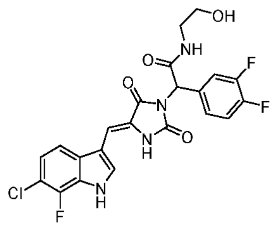 |
| 26 | 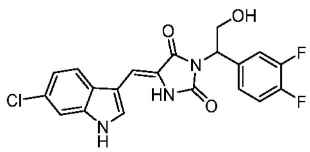 | 73 | 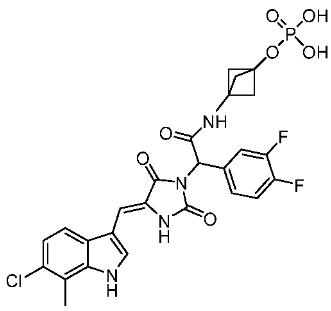 |
| 27 | 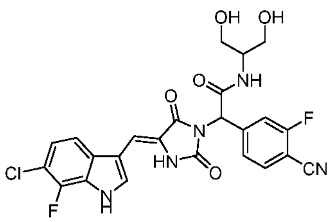 | 74 | 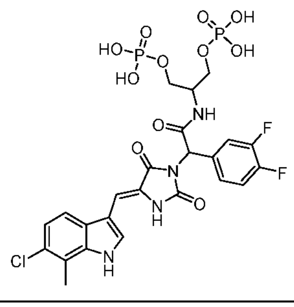 |

(continued)

| Number | Structure | Number | Structure |
|---|---|---|---|
| 28 | 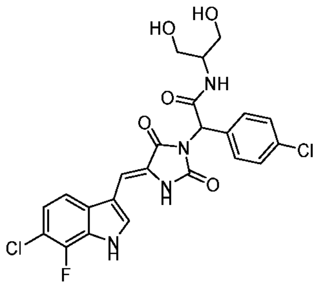 | 75 | 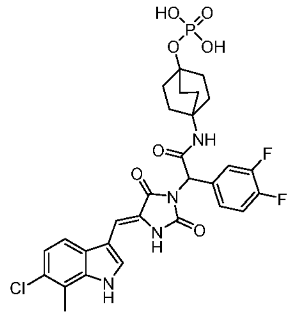 |
| 29 | 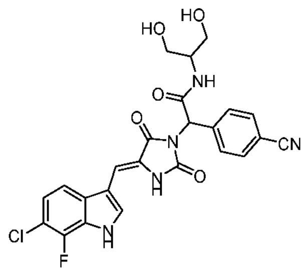 | 76 | 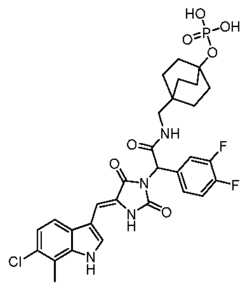 |
| 30 | 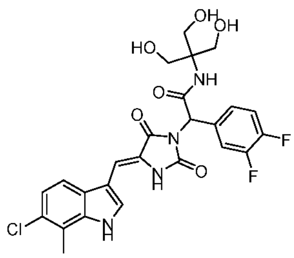 | 77 | 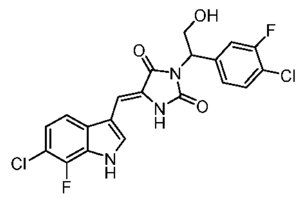 |
| 31 | 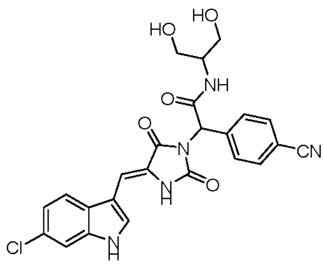 | 78 | 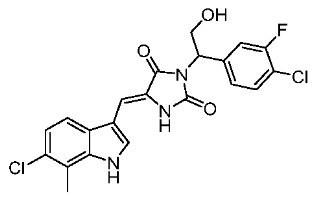 |
| 32 | 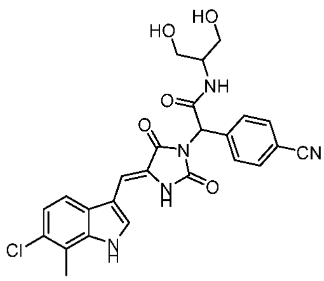 | 79 | 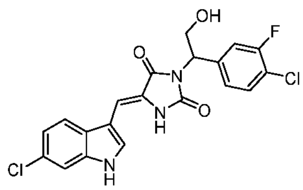 |
| 33 | 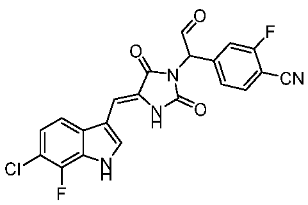 | 80 | 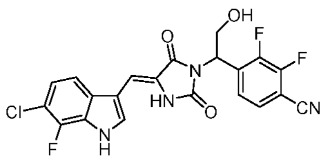 |

(continued)

| Number | Structure | Number | Structure |
|---|---|---|---|
| 34 | 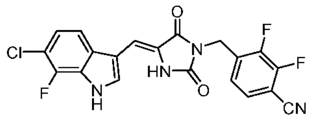 | 81 | 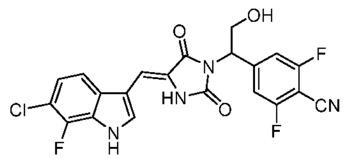 |
| 35 | 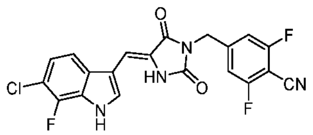 | 82 | 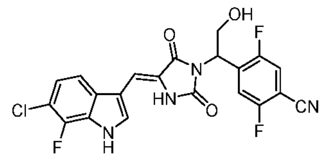 |
| 36 | 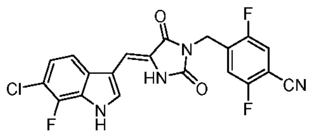 | 83 | 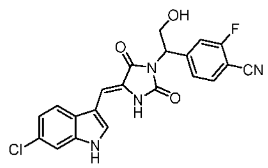 |
| 37 | 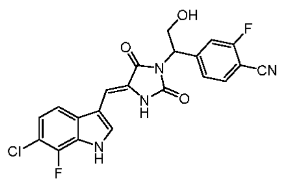 | 84 | 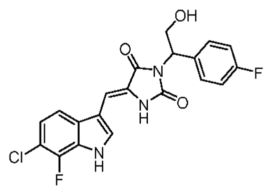 |
| 38 | 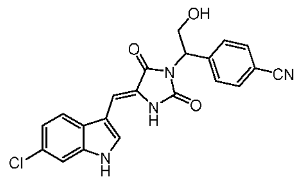 | 85 | 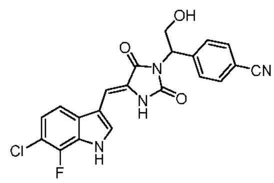 |
| 39 | 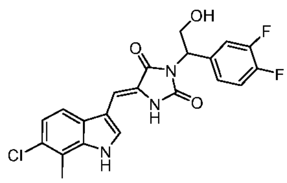 | 86 | 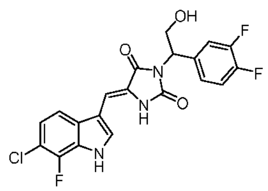 |
| 40 | 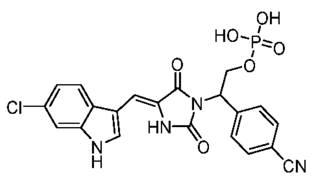 | 87 | 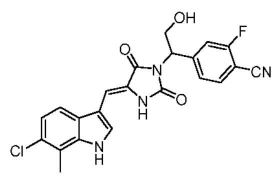 |
| 41 | 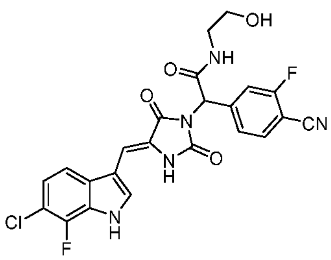 | 88 | 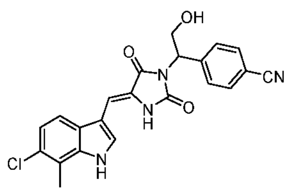 |

(continued)

| Number | Structure | Number | Structure |
|---|---|---|---|
| 42 | 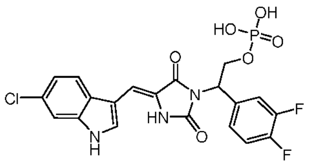 | 89 | 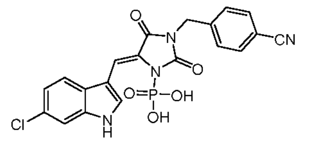 |
| 43 | 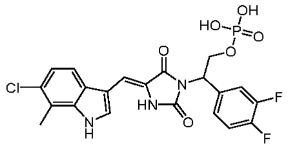 | 90 | 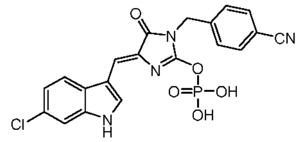 |
| 44 | 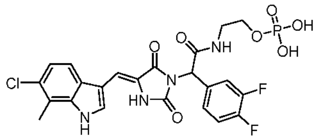 | 91 | 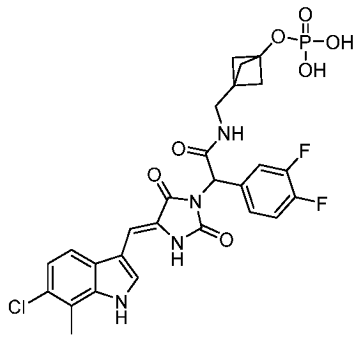 |
| 45 | 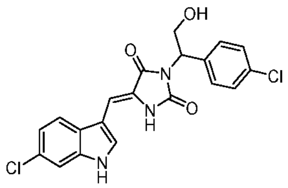 | 92 | 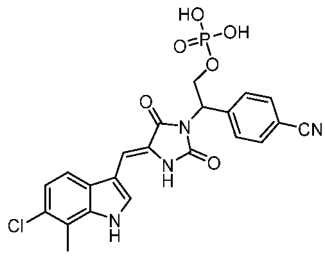 |
| 46 | 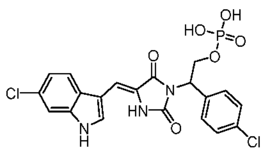 | 93 | 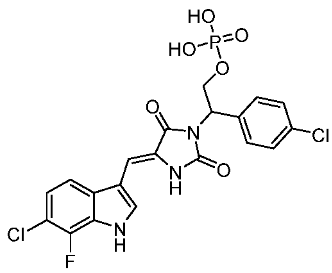 |
| 47 | 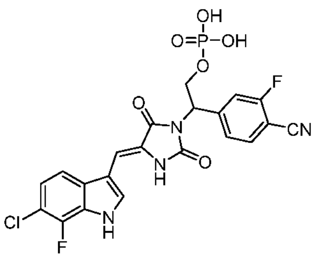 | 94 | 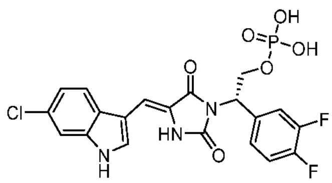 |

(continued)

| Number | Structure | Number | Structure |
|---|---|---|---|
| 95 | 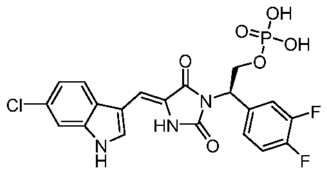 | 96 | 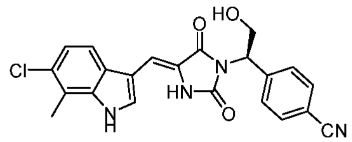 |
| 97 | 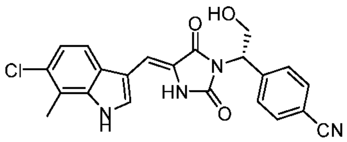 | 98 | 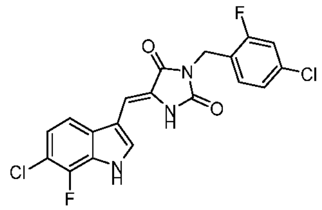 |
| 99 | 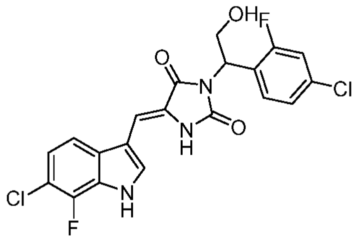 | 100 | 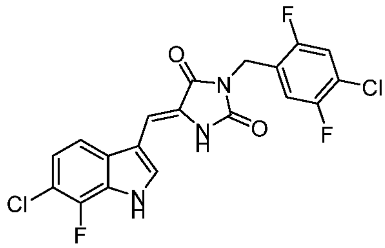 |
| 101 | 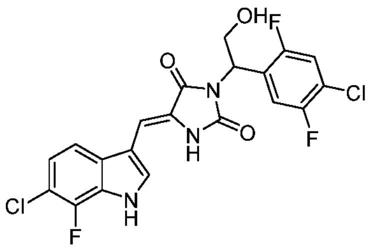 | 102 | 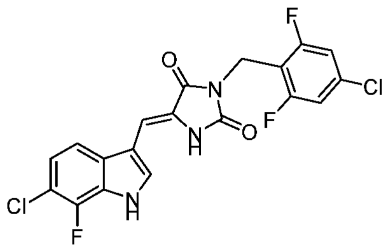 |
| 103 | 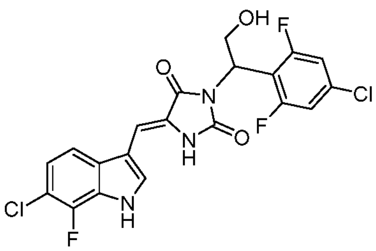 | 104 | 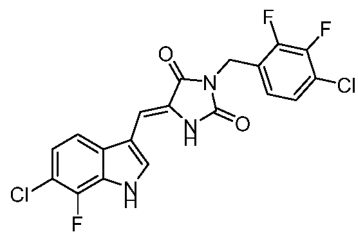 |
| 105 | 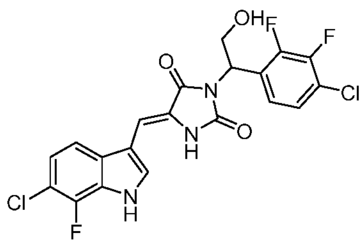 | 106 | 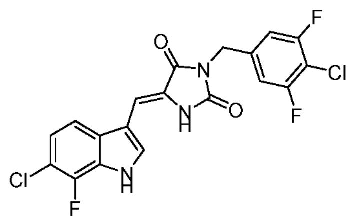 |

(continued)

| Number | Structure | Number | Structure |
|---|---|---|---|
| 107 | 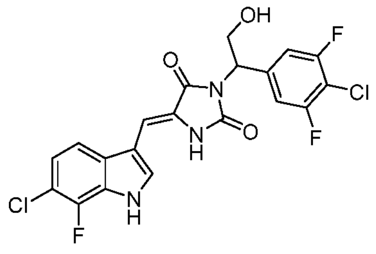 | 108 | 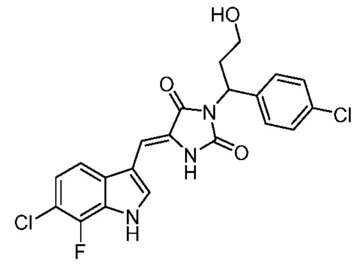 |
| 109 | 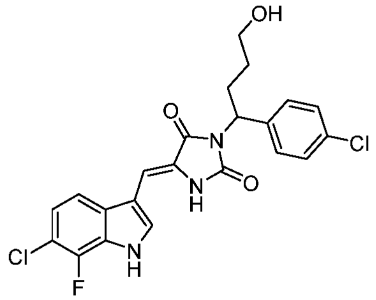 | 110 | 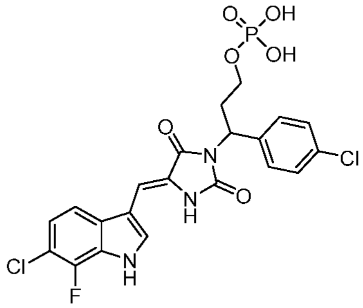 |

The invention further provides a method for preparing the compound shown in formula (I), including a method I and/or a method II:
method I: performing a reaction on a compound a and a compound b to obtain the compound shown in formula (I);

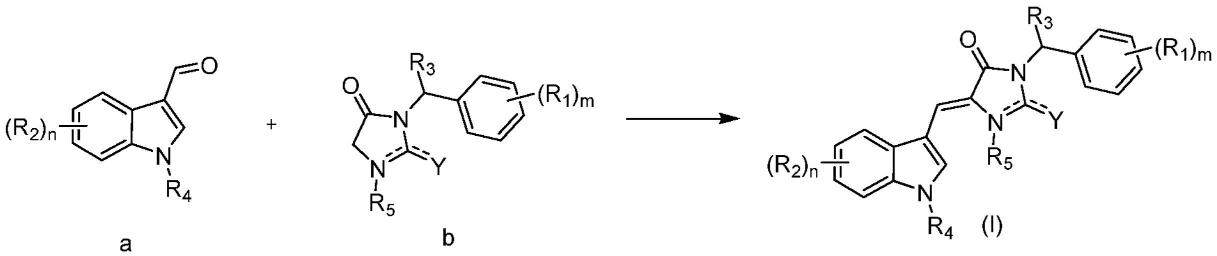

where $R_1$, $R_2$, $R_3$, $R_4$, $R_5$, Y, m, and n independently have definitions described above;
method II: performing a reaction on a compound c and $R_{3b}$-$NH_2$ to obtain the compound shown in formula (I);

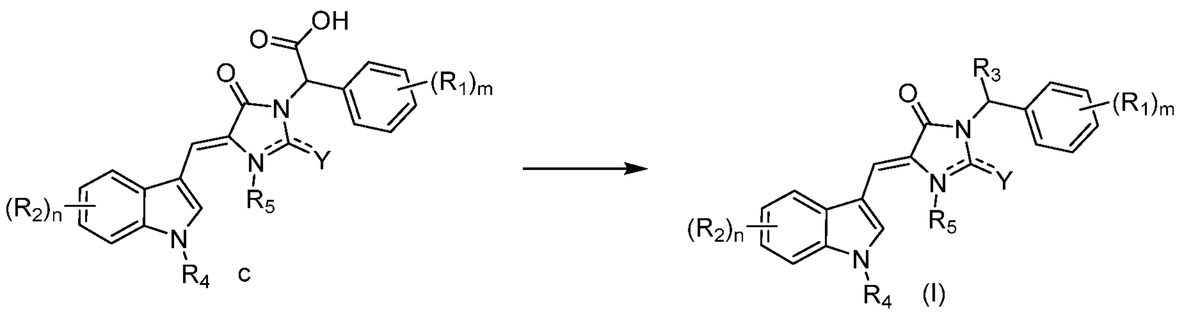

where $R_1$, $R_2$, $R_3$, $R_4$, $R_5$, $R_{3b}$, Y, m, and n independently have definitions described above.

The invention further provides a pharmaceutical composition, including a therapeutically effective amount of at least one of the compound shown in formula (I), and the raceme, the stereoisomer, the tautomer, the isotopic label, the solvate, the polymorph, the pharmaceutically acceptable salt or the prodrug compound thereof.
According to an embodiment of the invention, the pharmaceutical composition further includes one or more pharmaceutically acceptable accessories.

According to an embodiment of the invention, the pharmaceutical composition may further include one or more extra therapeutic agents.

The invention further provides a method for treating a neoplastic disease and/or idiopathic pulmonary fibrosis, including administrating to a patient a prophylactically or therapeutically effective amount of at least one of the compound shown in formula (I), and the raceme, the stereoisomer, the tautomer, the isotopic label, the solvate, the polymorph, the pharmaceutically acceptable salt or the prodrug compound thereof.

The invention further provides a method for treating a neoplastic disease and/or idiopathic pulmonary fibrosis, including administrating to a patient a prophylactically or therapeutically effective amount of the pharmaceutical composition.

According to an embodiment of the invention, the neoplastic disease may be a neoplastic disease induced by overexpression of MDM2 and MDMX.

According to an embodiment of the invention, the neoplastic disease includes at least one of leukemia, chronic myeloid leukemia, acute myelocytic leukemia, lymphocytic leukemia, hairy cell leukemia, T lymphoblastic leukemia, lymphoma, B-cell lymphoma, burkitt lymphoma, hodgkin lymphoma, chondrosarcoma, fibrosarcoma, ewing sarcoma, rhabdomyosarcoma, small cell lung cancer, non-small cell lung cancer, myeloma, glioma, prostatic cancer, breast cancer, bone cancer, neuroblastoma, gastric cancer, ovarian cancer, intestinal cancer, renal cancer, medulloblastoma, pancreatic cancer, thyroid cancer, mesothelioma, cervical cancer, bladder cancer, liver cancer, skin cancer, and tumors in the central system or the peripheral nervous system.

In some embodiments, the patient includes a mammal, preferably, a person.

The invention further provides at least one of a compound shown in formula (I), and a raceme, a stereoisomer, a tautomer, an isotopic label, a solvate, a polymorph, a pharmaceutically acceptable salt or a prodrug compound thereof, or a pharmaceutical composition thereof for treating a neoplastic disease and/or idiopathic pulmonary fibrosis.

The invention further provides use of at least one of a compound shown in formula (I), and a raceme, a stereoisomer, a tautomer, an isotopic label, a solvate, a polymorph, a pharmaceutically acceptable salt or a prodrug compound thereof in preparation of a drug.

According to an embodiment of the invention, the use may be use in preparation of a drug for treating an MDM2 and/or MDMX-mediated symptom and/or disease, for example, use in preparation of an MDM2 and/or MDMX inhibitor drug.

According to an embodiment of the invention, the use may be use in preparation of a drug for treating idiopathic pulmonary fibrosis.

According to an embodiment of the invention, the disease is, for example, a neoplastic disease. The neoplastic disease includes at least one of leukemia, chronic myeloid leukemia, acute myelocytic leukemia, lymphocytic leukemia, hairy cell leukemia, T lymphoblastic leukemia, lymphoma, B-cell lymphoma, burkitt lymphoma, hodgkin lymphoma, chondrosarcoma, fibrosarcoma, ewing sarcoma, rhabdomyosarcoma, small cell lung cancer, non-small cell lung cancer, myeloma, glioma, prostatic cancer, breast cancer, bone cancer, neuroblastoma, gastric cancer, ovarian cancer, intestinal cancer, renal cancer, medulloblastoma, pancreatic cancer, thyroid cancer, mesothelioma, cervical cancer, bladder cancer, liver cancer, skin cancer, and tumors in the central system or the peripheral nervous system.

## Beneficial effects

The compound provided by the invention has a good MDM2 and/or MDMX inhibiting effect and may be used for treating MDM2 and/or MDMX-mediated symptoms and/or diseases such as neoplastic diseases and/or idiopathic pulmonary fibrosis and preparing drugs for such symptoms or diseases. In particular, a compound containing a phosphoric acid (phosphate) structure has higher solubility. As the prodrug, the compound can release an MDM2 and/or MDMX double-target inhibitor compound with high activity in an animal body. Moreover, the preferred compound provided by the invention also has preferred pk activity. It solves the problem that such double-target inhibitors are hardly prepared into drugs.

## Definitions and descriptions of terms

Unless otherwise specified, radical and term definitions recorded in the description and claims of this application include definitions thereof as examples, exemplary definitions, preferred definitions, definitions recorded in a table, definitions of specific compounds in the examples, and the like, which may be arbitrarily combined and incorporated with each other. Such combined and incorporated radial definitions and compound structures should be construed as falling with the scope recorded by the description and/or claims of this application.

Unless otherwise specified, the numerical ranges recorded in the description and claims at least equivalently record each specific integer value. For example, the numerical value "1-8" equivalently records each integer value in the numerical value "1-8" , i.e., 1, 2, 3, 4, 5, 6, 7 or 8.

The term "optional" (or "optionally" , "option" ) in the definitions of the general formula in this application means that the radical is substituted with zero, one or more substituents. For example, "optionally substituted with one, two or more R" means that the radial may not be substituted with R (unsubstituted) or may be selectively substituted with one, two or more R.

"More" represents three or more than three.

The term "$C_{1-6}$ alkyl" should be construed as representing straight-chain alkyl and branched alkyl having 1, 2, 3, 4, 5 or 6 carbon atoms. The alkyl is, for example, methyl, ethyl, propyl, butyl, amyl, hexyl, isopropyl, isobutyl, sec-butyl, tertiary butyl, isoamyl, 2-methyl butyl, 1-methyl butyl, 1-ethyl propyl, 1,2-dimethyl propyl, neopentyl, 1, 1-dimethyl propyl, 4-methyl amyl, 3-methyl amyl, 2-methyl amyl, 1-methyl amyl, 2-ethyl butyl, 1-ethyl butyl, 3,3-dimethyl butyl, 2,2-dimethyl butyl, 1, 1-dimethyl butyl, 2,3-dimethyl ethyl, 1,3-dimethyl butyl or 1,2-dimethyl butyl, and the like or their isomers.

The term "$C_{2-6}$ alkenyl" should be construed as representing monovalent hydrocarbyl of a straight chain or a branched chain, including one or more double bonds and having 2-6 carbon atoms, for example, 2 or 3 carbon atoms (i.e., $C_{2-3}$ alkenyl). It should be understood that the alkenyl includes more than one double bond, and the double bonds may be separated from each other or may be conjugated. The alkenyl is, for example, vinyl, allyl, (E)-2-methyl vinyl, (Z)-2-methyl vinyl, (E)-2-butenyl, (Z)-2-butenyl, (E)-1-butenyl, (Z)-1-butenyl, 4-pentenyl, (E)-3-pentenyl, (Z)-3-pentenyl, (E)-2-pentenyl, (Z)-2-pentenyl, (E)-1-pentenyl, (Z)-1-pentenyl, 5-hexenyl, (E)-4-hexenyl, (Z)-4-hexenyl, (E)-3-hexenyl, (Z)-3-hexenyl, (E)-2-hexenyl, (Z)-2-hexenyl, (E)-1-hexenyl, (Z)-1-hexenyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1, 1-dimethylprop-2-enyl, 1-ethylprop-1-enyl, 1-propylvinyl, and 1-isopropylvinyl.

The term "$C_{2-6}$ alkynyl" should be construed as representing monovalent hydrocarbyl of a straight chain or a branched chain, including one or more triple bonds and having 2-6 carbon atoms, for example, 2 or 3 carbon atoms (i.e., $C_{2-3}$ alkynyl). The alkynyl is, for example, acetenyl, 1-propinyl, 2-propinyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or 3,3-dimethylbut-1-ynyl.

The term "$C_{3-8}$ cycloalkyl" should be construed as representing a saturated monovalent monocyclic, bicyclic hydrocarbon ring (for example, a condensed ring, a bridge ring, and a spiral ring) or tricyclic alkane, having 3-8 carbon atoms. The $C_{3-8}$ cycloalkyl may be monocyclic hydrocarbyl such as cyclopropyl, cyclobutyl, cyclopentyl, cyclohexyl, cycloheptyl or cyclooctyl, or may be bridged hydrocarbyl such as bicyclo[2. 1. 1]hexyl, bicyclo[2. 2. 1]heptyl, bicyclo[2. 2. 1]heptenyl, 6, 6-dimethyl-bicyclo[3. 1. 1]heptyl, 2,6,6-trimethyl bicycle[3. 1. 1]heptyl, bicycle[2. 2. 2]octyl, 2, 7-diazaspiro[3, 5]nonyl, and 2, 6-diazaspiro[3, 4]octyl.

Persons skilled in the art may understand that the compound shown in formula (I) may exist in the form of various pharmaceutically acceptable salts. If these compounds have alkaline centers, they may form acid addition salts; if these compounds have acidic centers, they may form alkali addition salts; and if these compounds not only include the acidic centers (for example, carboxyl) but also include the alkaline centers (for example, amino), they also may form inner salts.

The compound provided by the invention may exist in the form of the solvate (for example, a hydrate). The compound provided by the invention includes a polar solvent as a structural element of the compound lattices, particularly, for example, water, methanol or ethanol. The amount of the polar solvent, particularly, water may exist in the form of a stoichiometric ratio or a non-stoichiometric ratio.

According to its molecular structure, the compound provided in the invention may be chiral. Therefore, various enantiomer forms may exist. Therefore, these compounds may exist in the form of racemes or in an optical activity form. The compound provided by the invention covers various isomers with chiral carbon in an R or S configuration, a mixture thereof, and racemes. The compound provided by the invention or an intermediate thereof may be separated as enantiomer compounds by chemical or physical methods known to persons skilled in the art or may be used for synthesis in this form. In a case of amine of the raceme, a diastereoisomers is prepared from the mixture through a reaction with an optically active splitting reagent. An example of a proper splitting reagent is an optically active acid, for example, tartaric acid, diacetyl tartaric acid, dibenzoyltartaric acid, mandelic acid, malic acid, and lactic acid in R and S forms, proper N-protected amino acids (for example, N-benzoyl proline or N-phenyl sulfonyl proline) or various optically active camphorsulfonic acids. Chromatographic enantiomers may also be favorably split by virtue of the optically active splitting reagents (for example, dinitrobenzoylphenyl glycine, cellulose triacetate or derivatives of other carbohydrates or chirally derivatized methacrylic ester polymers fixed on a silica gel). A proper eluent for this purpose is a water or alcohol-containing solvent mixture, for example, hexane/isopropanol/acetonitrile.

Corresponding stable isomers may be separated according to known methods such as extraction, filtering or column chromatography.

The term "patient" refers to any animal including mammals, preferably, a mouse, a rat, another rodent, a rabbit, a dog, a cat, a pig, a cow, a goat, a horse or a primate, and most preferably a person.

The term "therapeutically effective amount" refers to a dose of an active compound or a drug inducing a biological or medical response sought by researchers, veterinarians, physicians or other clinical physicians in tissues, systems, animals, individuals or persons, including the following one or more: (1) preventing diseases: for example, preventing diseases, disorders or symptoms in individuals susceptible to diseases, disorders or symptoms without experiencing or having disease pathology or symptoms; (2) suppressing diseases: for example, suppressing diseases, disorders or symptoms (i.e., preventing further development of pathology and/or symptoms) in individuals experiencing or having pathology and/or symptoms of diseases, disorders or symptoms; and(3) relieving diseases: for example, relieving diseases, disorders or symptoms (i.e., reversing pathology and/or symptoms) in individuals experiencing or having pathology and/or symptoms of diseases, disorders or symptoms.

## Detailed Description

The technical solutions of the invention will be further described in detail below in conjunction with specific examples. It should be understood that the examples below merely describe and explain the invention exemplarily and should not be explained as a limitation to the protection scope of the invention. Technologies realized based on the above content of the invention should fall within the scope to be protected by the invention.

Unless otherwise specified, raw materials and reagents used in the examples below all are marketed commodities or may be prepared by known methods.

### Intermediates

Intermediate I-1: Synthesis of 3-(3,4-difluorobenzyl)imidazolidine-2,4-dione

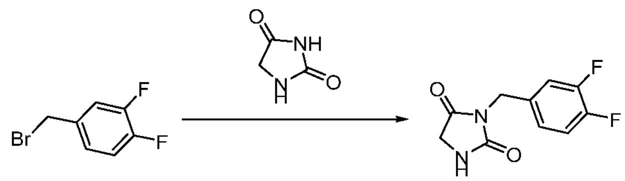

I-1

At room temperature, 4-(bromomethyl)-1,2-difluorobenzene (2 g, 9.178 mmol) and potassium carbonate (3.17 g, 22.945 mmol) are dissolved in N,N-dimethylformamide (20 mL). Hydantoin (1.35 g, 12.849 mmol) is added in batches into a mixed solution while the mixed solution is stirred. After being stirred at room temperature for 16 h, the mixture is poured into water (30 mL) and extracted with ethyl acetate (30 mL) three times, and organic phases are combined and washed with saturated saline. After being dried with anhydrous sodium sulfate, the organic phases are filtered, a filtrate is concentrated in vacuum to obtain a white solid crude product intermediate I-1 (1 g, 41.72%), and the crude product is not further purified and is directly used for a next reaction.

LC-MS m/z (ESI): 227.15 $[M+H]^+$.

### Intermediate I-2: Synthesis of 3-(4-chlorobenzyl)imidazolidine-2,4-dione

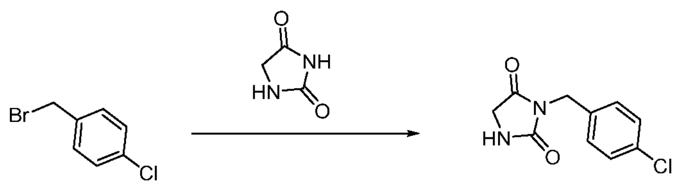

I-2

By using the method for the intermediate I-1, the white solid crude product intermediate I-2 (500 mg, 92.78%) is prepared by using 1-(bromomethyl)-4-chlorobenzene and hydantoin, and the crude product is not further purified and is

directly used for a next reaction.

**Intermediate I-3: Synthesis of 4-((2,5-dioxoimidazolidin-1-yl)methyl)benzonitrile**

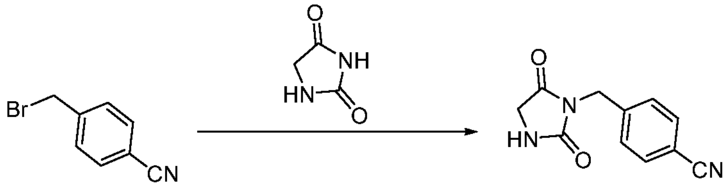

I-3

By using the method for the intermediate **I-1**, the white solid crude product intermediate **I-3** (2.26 g, 84%) is prepared by using 4-(bromomethyl)benzonitrile and hydantoin, and the crude product is not further purified and is directly used for a next reaction.

**Intermediate I-4: Synthesis of 4-((2,5-dioxoimidazolidin-1-yl)methyl)-2-fluorobenzonitrile**

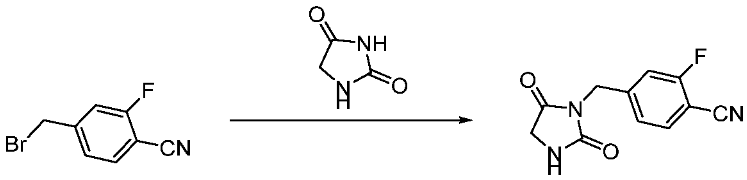

I-4

By using the method for the intermediate I-1, the white solid crude product intermediate **I-4** (500 mg, 81%) is prepared by using 1-(bromomethyl)-4-chlorobenzene and hydantoin, and the crude product is not further purified and is directly used for a next reaction.

LC-MS m/z (ESI): 232.00 $[M-H]^-$.

Intermediate **I-5: Synthesis of 6-chloro-7-methoxyl-1H-indole-3-**formaldehyde

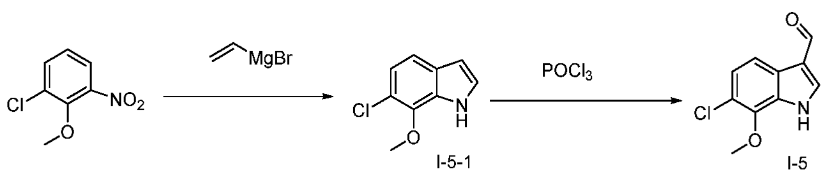


I-5-1

I-5

Intermediate **I-5-1: 6-chloro-7-methoxyl-1H-indole**

At -20° C, a bromovinyl magnesium bromide-tetrahydrofuran (1N, 160 mL) solution is slowly dropwise added into a solution of 1-chloro-2-methoxyl-3-nitrobenzene (10 g, 53.31 mmol) in tetrahydrofuran (200 mL). After being stirred at room temperature for 3 h, the mixture is poured into ice water (200 ml) and extracted with ethyl acetate (500 ml) twice. Organic phases are combined, washed with saturated saline, dried with anhydrous sodium sulfate, and concentrated in vacuum. The mixture is purified by flash chromatography to obtain the white solid I-5-1 (2.1 g, 19.52%).

LC-MS m/z (ESI): 182.1 $[M+H]^+$.

$^1H$ NMR (400 MHz, $CDCl_3$) δ 8.39 (s, 1H), 7.30 (dd, J = 8.4, 0.5 Hz, 1H), 7.19 (dd, J = 3.1, 2.5 Hz, 1H), 7.07 (d, J = 8.4 Hz, 1H), 6.53 (dd, J = 3.1, 2.2 Hz, 1H), 4.02 (s, 3H).

**I-5: 6-chloro-7-methoxyl-1H-indole-3-formaldehyde**

At 0° C, phosphorus oxychloride (2.12 g, 13.87 mmol) is added into dimethylformamide (20 ml). The mixture is stirred at 0° C for 10 min. Then, a solution of 6-chloro-7-methoxyl-1H-indole (2.1 g, 11.56 mmol) in dichloromethane (5 mL) is added. A mixed solution is stirred at room temperature for 1 h, sodium hydroxide (1N, 30 ml) is added into the mixed solution, and the mixed solution is stirred for 30 min. The mixed solution is extracted with ethyl acetate (30 ml) three times. Organic phases are combined, washed with anhydrous sodium sulfate, and concentrated in vacuum. The mixture is purified by flash chromatography to obtain the white solid **I-5** (0.9 g, 37.1%).

LC-MS m/z (ESI): 210.1 $[M+H]^+$. $^1H$ NMR (400 MHz, DMSO-d6) δ 12.57 (s, 1H), 9.94 (s, 1H), 8.35 (s, 1H), 7.82 (d, J = 8.4 Hz, 1H), 7.26 (d, J = 8.4 Hz, 1H), 3.94 (s, 3H).

**Intermediate I-6: Synthesis of 3-[(4-acetenyl phenyl)methyl]imidazolidine-2,4-dione**

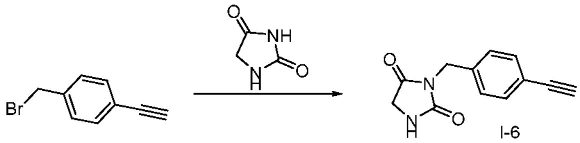

I-6

By using the method for the intermediate I-1, the white solid intermediate **I-6** (122 mg, 26%) is prepared by using 1-(bromomethyl)-4-acetylenylbenzene and hydantoin.

LC-MS m/z (ESI): 215.1 $[M+H]^+$.

**Intermediate I-7: Synthesis of 3-[4-(8-amino octyl)-1-oxo-3H-isoindol-2-yl]piperidine-2,6-dione**

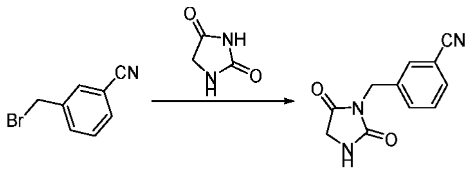

I-7

By using the method for the intermediate **I-1**, the brown solid intermediate **I-7** (129 mg, 29%) is prepared by using 3-(bromomethyl)benzonitrile and hydantoin.

LC-MS m/z (ESI): 214.0 $[M-H]^-$.

Intermediate **I-8: Synthesis of 6-chloro-7-methyl-1H-indole-3-formaldehyde**

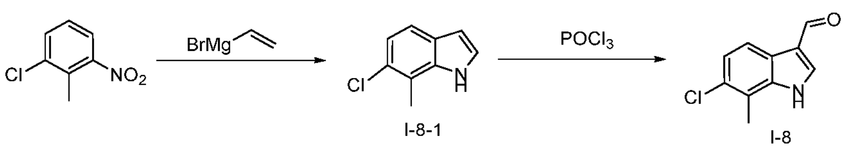


I-8-1 I-8

**I-8-1: 6-chloro-7-methyl-1H-indole**

Under nitrogen protection, 1-chloro-2-methyl-3-nitrobenzene (2.0 g, 11.656 mmol) is dissolved in a tetrahydrofuran (20 ml) solution, the temperature of the solution is decreased to -78° C, vinyl magnesium bromide (1N, 46.6 ml, 46.626 mmol) is slowly dropwise added, and the mixture is continuously stirred for 2 h. A saturated ammonium chloride (100 ml) solution is slowly added into the mixture and the temperature of the system is maintained not higher than -60° C. A reaction is performed to raise the temperature to room temperature, the mixture is extracted with ethyl acetate (100 ml) three times, and combined organic phases are washed with saturated saline (100 ml) and dried with anhydrous sodium

sulfate. After the organic phases are filtered, a filtrate is concentrated in vacuum. A residue is purified by silica gel column chromatography and eluted with petroleum ether/ethyl acetate (5:1) to obtain the brown oily **I-8-1** (1.08 g, 56%).
LC-MS m/z (ESI): 166.2 $[M+H]^+$.

**I-8: 6-chloro-7-methyl-1H-indole-3-formaldehyde**

At 0° C, phosphorus oxychloride (2.4 g, 16.094 mol) is dropwise added into a dimethylformamide (2.5 ml) solution. The obtained mixed solution is continuously stirred under nitrogen protection for 30 min and maintained at 0° C. A solution of 6-chloro-7-methyl-1H-indole (540 mg, 3.260 mmol) in dimethylformamide (2.5 ml) is added into the solution. After being stirred at 40° C for 1 h, the mixture is cooled to room temperature and water (20 ml) is added to quench the reaction. The pH value of the system is adjusted to 8 with a sodium hydroxide (30%) solution. The obtained mixture is extracted with ethyl acetate (50 ml) three times, and combined organic phases are washed with saturated saline (50 ml) and dried with anhydrous sodium sulfate. After the organic phases are filtered, a filtrate is concentrated in vacuum. A residue is purified by silica gel column chromatography and eluted with petroleum ether/ethyl acetate (1:1) to obtain the brown oily **I-8** (623 mg, 96%).
LC-MS m/z (ESI): 194.1 $[M+H]^+$.

**Intermediate I-9: Synthesis of 6-chloro-7-(methyl sulfonyl)-1H-indole-3-formaldehyde**

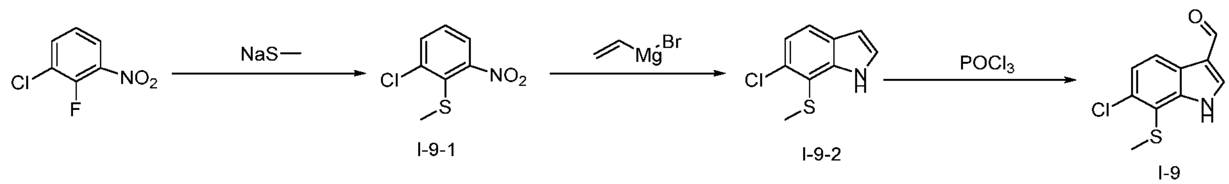


**I-9-1: 1-chloro-2-methylthio-3-nitrobenzene**

Under nitrogen protection at room temperature, 1-chloro-2-fluoro-3-nitrobenzene (2 g, 10.824 mmol) is dissolved in a DMF (40 mL) solution, sodium thiomethoxide (0.88 g, 11. 906 mmol) is added into the solution, and the mixed solution is maintained stirred. The mixture is heated to 120° C and stirred for 12 h, and after the reaction is completed, the mixture is cooled to room temperature. The obtained mixed solution is concentrated in vacuum. A residue is purified by silica gel column chromatography and eluted with petroleum ether/ethyl acetate (5/95) to obtain the reddish brown oily **I-9-1** (770 mg, 29%).
$^1H$ NMR (300 MHz, $CDCl_3$) δ 7. 68-7. 60 (m, 1H), 7. 55-7. 47 (m, 1H), 7.41-7.38 (m, 1H), 2.46 (s, 3H).

**I-9-2: 6-chloro-7-(methyl sulfonyl)-1H-indole**

Under nitrogen protection, 1-chloro-2-methylthio-3-nitrobenzene (230 mg, 0.932 mol) is dissolved in a tetrahydrofuran (5 ml) solution, the temperature of the solution is decreased to -78° C, vinyl magnesium bromide (1N, 3.7 ml, 3.7 mmol) is slowly dropwise added, and the mixture is continuously stirred for 3 h. A saturated ammonium chloride (50 ml) solution is slowly added into the mixture and the temperature of the system is maintained not higher than -60° C. A reaction is performed to raise the temperature to room temperature, the mixture is extracted with ethyl acetate (50 ml) twice, and combined organic phases are washed with saturated saline (50 ml) and dried with anhydrous sodium sulfate. After the organic phases are filtered, a filtrate is concentrated in vacuum. A residue is purified by preparative high performance liquid chromatography to obtain the brown oily **I-9-2** (180 mg, 81%).
LC-MS m/z (ESI): 198.1 $[M+H]^+$.

**I-9: 6-chloro-7-(methylsulfonyl)-1H-indole-3-formaldehyde**

At 0° C, phosphorus oxychloride (0.178 g, 1.115 mol) is dropwise added into a dimethylformamide (1.5 ml) solution. The obtained mixed solution is continuously stirred under nitrogen protection for 30 min and maintained at 0° C. A solution of 6-chloro-7-(methylsulfonyl)-1H-indole (180 mg, 0.76 mmol) in dimethylformamide (1.5 ml) is added into the solution. After being stirred at 40° C for 1 h, the mixture is cooled to room temperature and water (5 ml) is added to quench the reaction. The pH value of the system is adjusted to 8 with a sodium hydroxide (30%) solution. The obtained mixture is extracted with ethyl acetate (20 ml) three times, and combined organic phases are washed with saturated saline (30 ml)

and dried with anhydrous sodium sulfate. After the organic phases are filtered, a filtrate is concentrated in vacuum to obtain the light yellow solid I-9 (160 mg, 89%). A crude product is directly left for a next step without further purification.

LC-MS m/z (ESI): 226.0 $[M+H]^+$.

**Intermediate I-10: Synthesis of 6-chloro-7-fluoro-1H-indole-3-formaldehyde**

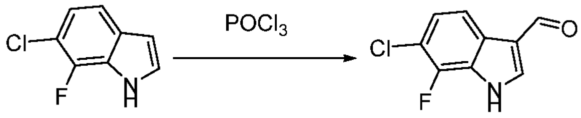


I-10

At 0° C, phosphorus oxychloride (0.18 ml, 1.117 mol) is dropwise added into a dimethylformamide (1.5 ml) solution. The obtained mixed solution is continuously stirred under nitrogen protection for 30 min and maintained at 0° C. A solution of 6-chloro-7-fluoro-1H-indole (75 mg, 0.44 mmol) in dimethylformamide (1.5 ml) is added into the solution. After being stirred at 40° C for 1 h, the mixture is cooled to room temperature and water (5 ml) is added to quench the reaction. The pH value of the system is adjusted to 8 with a sodium hydroxide (30%) solution. The obtained mixture is extracted with ethyl acetate (50 ml) three times, and combined organic phases are washed with saturated saline (30 ml) and dried with anhydrous sodium sulfate. After the organic phases are filtered, a filtrate is concentrated in vacuum to obtain the white-like solid **I-10** (75 mg, 44.07%). A crude product is directly left for a next step without further purification.

**Intermediate I-11: Synthesis of 6-chloro-7-(fluoromethoxyl)-1H-indole-3-formaldehyde**

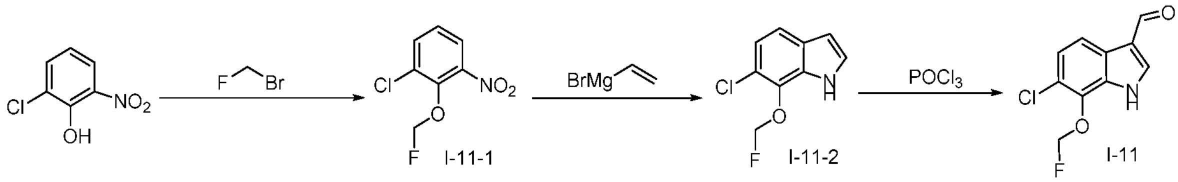


**I-11-1: 1-chloro-2-(fluoromethoxyl)-3-nitrobenzene**

At room temperature, 6-chloro-2-nitrophenol (1 g, 5.474 mmol) and potassium carbonate (1.59 g, 10.929 mmol) are added into a dimethylformamide (15 ml) solution and are maintained stirred, and bromofluoromethane (6.51 g, 54.764 mmol) is added into the mixture. The mixture is stirred overnight under nitrogen protection at room temperature, and after the reaction is completed, the mixed solution is poured into water (50 ml). The obtained mixture is extracted with ethyl acetate (30 ml) three times and washed with saturated saline (20 ml), and organic phases are combined and dried with anhydrous sodium sulfate. After the organic phases are filtered, a filtrate is concentrated in vacuum. A residue is purified by silica gel column chromatography and eluted with petroleum ether/ethyl acetate (5:1) to obtain the yellow oily **I-11-1** (430 mg, 38.14%).

$^1$H NMR (300 MHz, DMSO-$d_6$) $\delta$ 8.02-7.95 (m, 2H), 7. 52 (d, J = 8. 1 Hz, 1H), 5.87 (s, 1H), 5.70 (s, 1H).

**I-11-2: 6-chloro-7-(fluoromethoxyl)-1H-indole**

Under nitrogen protection, 1-chloro-2-(fluoromethoxyl)-3-nitrobenzene (590 mg, 2.864 mol) is dissolved in a tetrahydrofuran (8 ml) solution, the temperature of the solution is decreased to -78° C, vinyl magnesium bromide (1N, 8 ml, 8 mmol) is slowly dropwise added, and the mixture is continuously stirred for 3 h. A saturated ammonium chloride (50 ml) solution is slowly added into the mixture and the temperature of the system is maintained not higher than -60° C. A reaction is performed to raise the temperature to room temperature, the mixture is extracted with ethyl acetate (50 ml) twice, and combined organic phases are washed with saturated saline (50 ml) and dried with anhydrous sodium sulfate. After the organic phases are filtered, a filtrate is concentrated in vacuum. A residue is purified by preparative high performance liquid chromatography to obtain the gray oily I-11-2 (690 mg, 81%).

LC-MS m/z (ESI): 198.0 $[M-H]^-$.

**I-11: 6-chloro-7-(fluoromethoxyl)-1H-indole-3-formaldehyde**

At 0° C, phosphorus oxychloride (1.25 g, 7.848 mol) is dropwise added into a dimethylformamide (13 ml) solution. The obtained mixed solution is continuously stirred under nitrogen protection for 30 min and maintained at 0° C. A solution of 6-chloro-7-(fluoromethoxyl)-1H-indole (640 mg, 3.2 mmol) in dimethylformamide (13 ml) is added into the solution. After being stirred at 40° C for 1 h, the mixture is cooled to room temperature and water (4 ml) is added to quench the reaction. The pH value of the system is adjusted to 8 with a sodium hydroxide (30%) solution. The obtained mixture is extracted with ethyl acetate (30 ml) three times, and combined organic phases are washed with saturated saline (30 ml) and dried with anhydrous sodium sulfate. After the organic phases are filtered, a filtrate is concentrated in vacuum to obtain the brown oily I-11 (380 mg, 51.56%). A crude product is directly left for a next step without further purification.
LC-MS m/z (ESI): 228.2 [M+H]+.

**Intermediate I-12: Synthesis of 2-(4-chlorophenyl)-2-(2, 5-dioxoimidazolidine-1-yl)acetic acid**

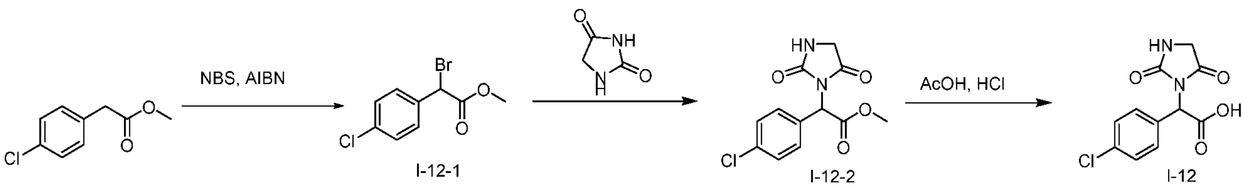


I-12-1 I-12-2 I-12

**I-12-1: Methyl 2-bromo-2(4-chlorophenyl)acetate**

N-bromosuccinimide (1.93 g, 10.83 mmol) and azodiisobutyronitrile (1.78 g, 10.83 mmol) are added into a solution of methyl 2-(4-chlorophenyl)acetate (2 g, 10.83 mmol) in carbon tetrachloride (20 mL). A mixed solution is stirred at 80° C for 5 h. The mixture is dried in vacuum to obtain the yellow oily **I-12-1** (1.7 g, 56%).
$^1$H NMR (300 MHz, $CDCl_3$) δ 7. 50-7. 47 (m, 2H), 7. 36-7. 33 (m, 2H), 5.32 (s, 1H), 3. 79 (s, 3H).

**I-12-2: Methyl 2-(4-chlorophenyl)-2-(2, 5-dioxoimidazolidin-1-yl)acetate**

At room temperature, hydantoin (0.89 g, 8.55 mmol) and potassium carbonate (1.63 g, 11.8 mmol) are respectively added into a solution of methyl 2-bromo-2(4-chlorophenyl)acetate (1.56 g, 5.9 mmol) in dimethylformamide (20 mL). A mixture is stirred at 25° C for 16 h. After the reaction is finished, the mixture is diluted with water and extracted with ethyl acetate (50 mL) three times. Organic phases are combined, washed with saturated saline, dried with anhydrous sodium sulfate, filtered and concentrated in vacuum. The organic phases are purified by liquid chromatography to obtain the yellow solid **I-12-2** (1.85 g, 88%).
$^1$H NMR (300 MHz, $CDCl_3$) δ 7.45 (d, J = 8.5 Hz, 2H), 7. 34-7. 26 (m, 2H), 5. 75 (s, 1H), 5. 71 (s, 1H), 4.01 (s, 2H), 3.80 (s, 3H).

**I-12: (4-chlorophenyl)(2,5-dioxoimidazolidin-1-yl)acetic acid**

At room temperature, methyl 2-(4-chlorophenyl)-2-(2, 5-dioxoimidazolidin-1-yl)acetate (1.17 g, 4.139 mmol) is added into 2.5 mL of concentrated hydrochloric acid and maintained stirred, and acetic acid (7.5 mL) is dropwise added into a mixed solution. The obtained mixture is heated to 120° C and then stirred for 3 h. After the reaction is finished, the reaction mixture is cooled to room temperature. The obtained mixture is concentrated at a reduced pressure to obtain a crude product **I-12** (1.1 g, 86.7%) in a white solid form. The purified crude product is used for a next step.
LC-MS m/z (ESI): 269.0 [M+H]+.

**Intermediate I-13: Synthesis of (Z)-2-(4-((6-chloro-1H-indol-3-yl)methylene)-2,5-dioxoimidazolidin-1-yl)-2-(4-chlorophenyl)acetic acid**

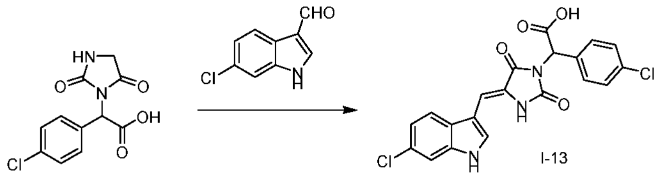

I-12

I-13

methyl 2-(4-chlorophenyl)-2-(2, 5-dioxoimidazolidin-1-yl)acetate (1.56 g, 5.5 mmol) is added into an ethyl acetate (10 mL) solution, and then concentrated hydrochloric acid (3 mL) is then added. The obtained mixture is reflowed till it is completely dissolved. The mixture is concentrated to obtain an intermediate. The intermediate is dissolved in ethyl acetate (15 mL) and piperidine (2 mL) for being further reflowed for 2 h, and then 6-chloro-1H-indole-3-formaldehyde (0.99 g, 5.5 mmol) is added. The mixture is stirred and reflowed for 16 h. After the reaction is finished, the reaction mixture is cooled to room temperature. The mixture is filtered to collect a yellow precipitant, so as to obtain a crude product in a piperidine salt form. The crude product is dissociated with hydrochloric acid to obtain the I-13 (0.56 g, 21.82%). The purified crude product is used for a next step.

LC-MS m/z (ESI): 429.9 [M+H]+.

**Intermediate I-14: Synthesis of [(4Z)-4-[(6-chloro-7-fluoro-1H-indol-3-yl)methylene]-2,5-dioxoimidazolidin-1-yl] (4-chlorophenyl)acetic acid**

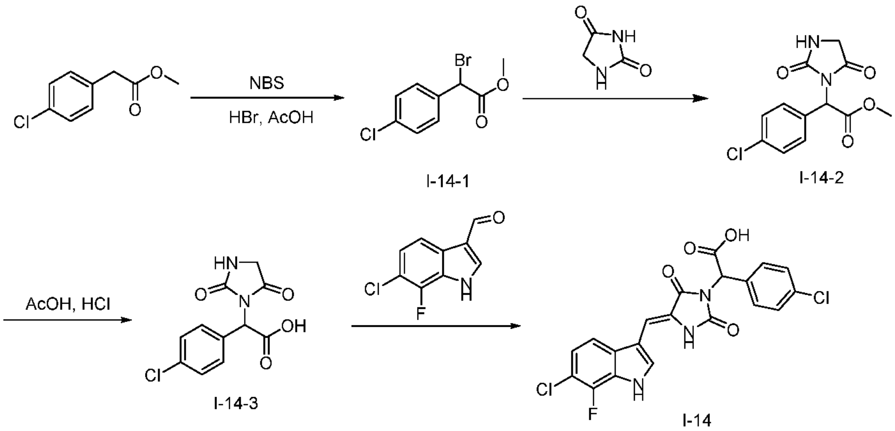


I-14-1

I-14-2

I-14-3

I-14

**I-14-1: Methyl 2-bromo-2-(4-chlorophenyl)acetate**

At room temperature, hydrobromic acid in acetic acid (7.04 g, 35.208 mmol) is dropwise added into a stirred solution of methyl 2-(4-chlorophenyl)acetate (5 g, 27.083 mmol) and NBS (6.27 g, 35.208 mmol) in DCE (50 mL). The obtained mixture is then stirred at 85° C for 12 h. After being cooled to room temperature, the obtained mixture is concentrated in vacuum to obtain a white solid crude product **I-14-1** (8 g), and the crude product is directly used for a next step without further purification.

**I-14-2: Methyl 2-(4-chlorophenyl)-2-(2,5-dioxoimidazolidin-1-yl)**acetate

At room temperature, methyl 2-bromo-2-(4-chlorophenyl)acetate (2 g) and potassium carbonate (13.1 g, 9.488 mmol) are respectively added into a DMF (15 mL) solution, and then hydantoin (570 mg, 5.692 mmol) is added. The obtained mixture is then stirred at room temperature for 12 h. The obtained mixture is concentrated in vacuum. A residue is purified by silica gel column chromatography and eluted with petroleum ether/ethyl acetate (5:1) to obtain the **I-14-2** (1.17 g, 93.45%) in a white solid form.

LC-MS m/z (ESI): 283.1 [M+H]+.

**I-14-3: (4-chlorophenyl)(2,5-dioxoimidazolidin-1-yl)acetic acid**

At room temperature, methyl 2-(4-chlorophenyl)-2-(2,5-dioxoimidazolidin-1-yl)acetate (1.17 g, 4.139 mmol) is

added into 2.5 mL of concentrated hydrochloric acid and maintained stirred, and acetic acid (7.5 mL) is continuously dropwise added into a mixed solution. The obtained mixture is heated to 120° C and then stirred for 3 h. A product is cooled to room temperature. The obtained mixture is concentrated in vacuum to obtain a white solid crude product **I-14-3** (1.1 g), and the crude product is directly used for a next step without further purification.

LC-MS m/z (ESI): 269 $[M+H]^+$.

**I-14: [(4Z)-4-[(6-chloro-7-fluoro-1H-indol-3-yl)methylene]-2,5-dioxoimidazolidin-1-yl](4-chlorophenyl)acetic acid**

At room temperature, (4-chlorophenyl)(2,5-dioxoimidazolidin-1-yl)acetic acid (400 mg, 1.487 mmol) and 6-chloro-7-fluoro-1H-indole-3-formaldehyde (294 mg, 1.489 mmol) are respectively added into ethanol (6 mL), and then piperidine (0.54 mL) is added into a mixed solution. The obtained mixture is heated to 100° C and stirred for 12 h. After the product is cooled to room temperature, a residue is purified by silica gel column chromatography and eluted with petroleum ether/ethyl acetate (2:1) to obtain the yellow solid **I-14** (196 mg, 31%).

LC-MS m/z (ESI): 446.0 $[M-H]^-$.

**Intermediate I-15: Synthesis of (4-cyanophenyl)(2,5-dioxypyrimidin-1-yl)acetic acid**

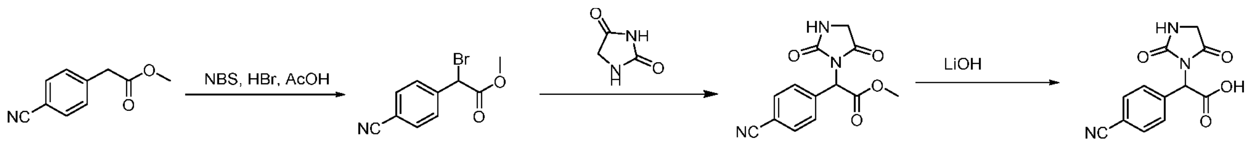

I-15-1 I-15-2 I-15

**I-15-1: Methyl 2-bromo-2(4-cyanophenyl)acetate**

At room temperature, methyl 2-(4-cyanophenyl)acetate (1200 mg, 6.507 mmol) and NBS (1552 mg, 8.719 mmol) are respectively added into a DCE (12 mL) solution, and a hydrobromic acid solution (0.27 mL, 8.784 mmol) is continuously dropwise added into a mixed system and maintained stirred. After the obtained mixture is heated to 85° C and stirred for 12 h, the mixture is cooled to room temperature. The obtained mixture is concentrated in vacuum. A residue is dissolved in ethyl acetate (50 mL). The residue is washed with saturated saline (30 mL) three times and dried with anhydrous sodium sulfate. After the residue is filtered, a filtrate is concentrated at a reduced pressure to obtain a light yellow solid crude product **I-15-1** (1.46 g), and the crude product may be used for a next step without purification.

**I-15-2: Methyl 2-(4-cyanophenyl)-2-(2,5-dioxoimidazolidin-1-yl)acetate**

At room temperature, methyl 2-bromo-2-(4-cyanophenyl)acetate (1.46 g) and hydantoin (1.52 g, 14.429 mmol) are respectively added into a dimethylformamide (20 mL) solution, and potassium carbonate (2.10 g, 14.471 mmol) is continuously added into the solution. A mixture is stirred under nitrogen protection at room temperature for 12 h, reacted, and poured into a saturated ammonium chloride solution (150 mL). The mixture is extracted with ethyl acetate (100) three times. Organic phases are combined, washed with saturated saline (100 mL) twice, and dried with anhydrous sodium sulfate. After the organic phases are filtered, a filtrate is concentrated at a reduced pressure. A residue is purified by reversed-phase chromatography to obtain the **I-15-2** (400 mg, 24.3%) in a light yellow solid form.

LC-MS m/z (ESI): 274.2 $[M+H]^+$.

**I-15: (4-cyanophenyl)(2,5-dioxoimidazolidin-1-yl)acetic acid**

methyl 2-(4-cyanophenyl)-2-(2, 5-dioxoimidazolidin-1-yl)acetate (400 mg, 1421 mmol) is dissolved in a mixed solution of tetrahydrofuran (15 mL) and water (15 mL), and a lithium hydroxide aqueous solution (2.1 mL, 2.100 mmol) is dropwise added into the solution. The obtained mixture is then stirred at room temperature under nitrogen protection for 0.5 h. A reside is acidized with hydrochloric acid till the pH value is equal to 5. The obtained mixture is concentrated in vacuum. A residue is purified by reversed-phase chromatography to obtain the **I-15** (380 mg, 42%) in a white solid form.

LC-MS m/z (ESI): 260.1 $[M+H]^+$.

**Intermediate I-16: Synthesis of [(4Z)-4-[(6-chloro-7-fluoro-1H-indol-3-yl)methylene]-2,5-dioxoimidazolidin-1-yl](4-cyanophenyl)acetic acid**

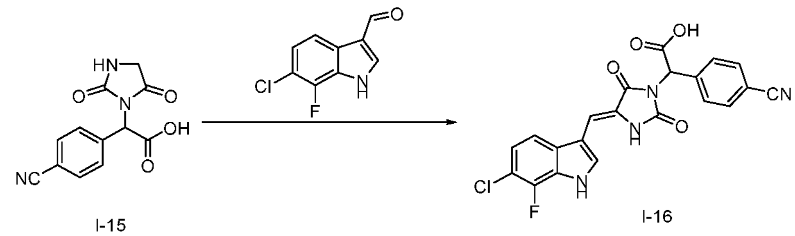

I-15 I-16

At room temperature, (4-cyanophenyl)(2,5-dioxoimidazolidin-1-yl)acetic acid (200 mg, 0.734 mmol) is added into ethanol (4 mL), and piperidine (263 mg, 2.936 mmol) is continuously added into a solution. The obtained mixture is heated to 80° C under nitrogen protection and stirred for 0.5 h. The reaction is reduced to room temperature, 6-chloro-7-fluoro-1H-indole-3-formaldehyde (137 mg, 0.661 mmol) is added in batches into the mixture, and the obtained mixture is heated to 80° C and then stirred for 12 h. The mixture is concentrated at a reduced pressure. A residue is purified by reversed-phase chromatography to obtain the yellow solid I-16 (60 mg, 24%).

LC-MS m/z (ESI): 439.1 [M+H]$^+$.

**Intermediate I-17: Synthesis of [(4Z)-4-[(6-chloro-7-methyl-1H-indol-3-yl)methylene]-2,5-dioxoimidazolidin-1-yl](3,4-difluorophenyl)acetic acid**

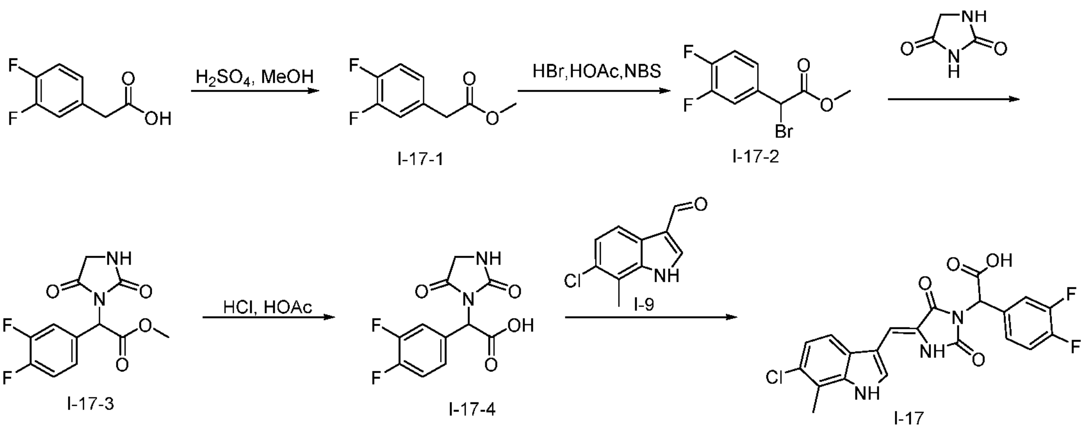


I-17-1 I-17-2 I-17-3 I-17-4 I-17

**I-17-1: Methyl 2-(3,4-difluorophenyl)acetate**

(3, 4-difluorophenyl)acetic acid (10 g, 58 mmol) is added into a methanol (100 mL) solution, and sulfuric acid (10 mL) is slowly dropwise added into the mixed solution. The mixed solution is reacted and reflowed overnight, cooled to room temperature, and poured into ice water (100 mL). The mixture is extracted with ethyl acetate (100 mL) three times. Organic phases are combined and dried with anhydrous $Na_2SO_4$, filtered, and concentrated in vacuum to obtain a crude product. The crude product is purified by column chromatography (petroleum ether/ethyl acetate) to obtain the yellow oily **I-17-1** (10 g, 88%).

LC-MS m/z (ESI): 187.0 [M+H]$^+$.

**I-17-2: Methyl 2-bromo-2-(3,4-difluorophenyl)acetate**

methyl 2-(3, 4-difluorophenyl)acetate (3.0 g, 16 mmol) is added into a carbon tetrachloride (30 mL) solution, and NBS (2.6 g, 14 mmol) and 12 drops of a solution of hydrobromic acid in acetic acid are added into the mixed solution. A reaction mixture is heated to 70° C and stirred for 10 min. The temperature of the reaction is raised to 85° C and the mixture is continuously stirred for 2. 5 h till thin-layer chromatographic tracking shows that raw materials are consumed completely. After being cooled to room temperature, the mixture is concentrated in vacuum to obtain a crude product. The crude

product is purified by column chromatography (petroleum ether/ethyl acetate) to obtain the yellow oily I-17-2 (1.1 g, 72%).
$^1$H NMR (400 MHz, $CDCl_3$) δ 7. 49-7. 44 (m, 1H), 7. 28-7. 24 (m, 1H), 7.18-7. 11 (m, 1H), 5.31 (s, 1H), 3.80 (s, 3H).

**I-17-3: Methyl 2-(3, 4-difluorophenyl)-2-(2, 5-dioxoimidazolidin-1-yl)acetate**

Imidazolidine-2, 4-dione (1.71 g, 17 mmol) and potassium carbonate (3.93 g, 28.4 mmol) are added into a solution of methyl 2-bromo-2-(3,4-difluorophenyl)acetate (3.8 g, 14 mmol) in dimethylformamide (37 mL). A reaction mixture is then stirred at room temperature for 14 h. After the reaction is completed, the reaction mixture is diluted with water (100 mL) and extracted with ethyl acetate (50 mL) three times. Organic phases are combined, washed with anhydrous sodium sulfate, filtered and concentrated in vacuum. A crude product is purified by column chromatography (petroleum ether: ethyl acetate=2:1) to obtain the brown solid I-17-3 (1.6 g, 37%).
LC-MS m/z (ESI): 285.0 $[M+H]^+$.

**I-17-4: 2-(3, 4-difluorophenyl)-2-(2,5-dioxoimidazolidin-1-yl)acetic acid**

methyl 2-(3, 4-difluorophenyl)-2-(2, 5-dioxoimidazolidin-1-yl)acetate (0.85 g, 3.0 mmol) is added into a mixed solution of concentrated hydrochloric acid (26 mL) and ethyl acetate (85 mL), and the mixed solution is heated to 120° C and stirred for 1 h. A mixture is concentrated in vacuum to obtain the yellow oily crude product **I-17-4** (0.80 g, 94%), and the crude product is directly used for a next step without further purification.
LC-MS m/z (ESI): 271.0 $[M+H]^+$.

**I-17: (Z)-2-(4-((6-chloro-7-methyl-1H-indol-3-yl)methylene)-2,5-dioxoimidazolidin-1-yl)-2-(3,4-difluorophenyl) acetic acid**

2-(3, 4-difluorophenyl)-2-(2, 5-dioxoimidazolidin-1-yl)acetic acid (0.60 g, 2.2 mmol) is added into ethanol (20 mL), and piperidine (1.9 g, 22 mmol) is added into a mixed system. A mixture is heated and reflowed for 2 h, and then 6-chloro-7-methyl-1H-indole-3-formaldehyde (0.43 g, 2.2 mmol) is added. The mixture is continuously reflowed and stirred for 24 h. The mixture is filtered and extracted with ethyl acetate (2 mL). A solid is dried in vacuum to obtain the yellow solid **I-17** (0.7 g, 71%), and the solid is directly used for a next step without further purification.
LC-MS m/z (ESI): 446.0 $[M+H]^+$. $^1$H NMR (400 MHz, DMSO-$d_6$) δ 11.95 (brs, 1H), 8.75 (brs, 1H), 8.16 (brs, 1H), 7.62-7.58 (m, 2H), 7.33-7.19 (m, 2H), 7.14 (d, J = 8.4 Hz, 1H), 6.74 (s, 1H), 6.67 (s, 1H), 5.31 (s, 1H), 2.49 (s, 3H).

**Intermediate I-18: Synthesis of [(4Z)-4-[(6-chloro-1H-indol-3-yl)methylene]-2,5-dioxoimidazolidin-1-yl](4-cyanophenyl)acetic acid**

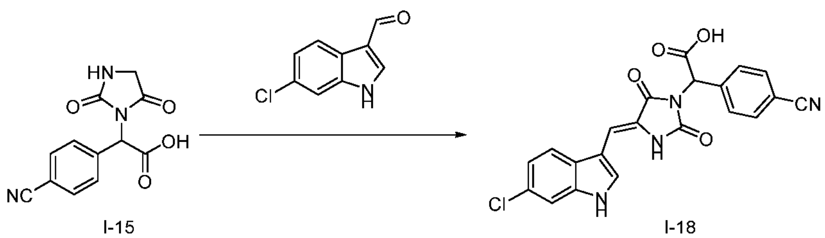

I-15 I-18

At room temperature, (4-cyanophenyl)(2,5-dioxoimidazolidin-1-yl)acetic acid (200 mg, 0.772 mmol) and piperazine (279.82 mg, 3.088 mmol) are added into an ethanol (3 mL) solution. The obtained mixture is heated to 65° C and stirred for 20 min. 6-chloro-1H-indole-3-formaldehyde (102 mg, 0.540 mmol) is added in batches into the mixture. The mixture is then stirred at 65° C for 12 h. Through vacuum concentration, a residue is purified by reversed-phase chromatography to obtain the yellow solid **I-18** (102 mg, 31%).
LC-MS m/z (ESI): 419.0 $[M-H]^-$.

**Intermediate I-19: Synthesis of [(4Z)-4-[(6-chloro-7-methyl-1H-indol-3-yl)methylene]-2,5-dioxoimidazolidin-1-yl](4-cyanophenyl)acetic acid**

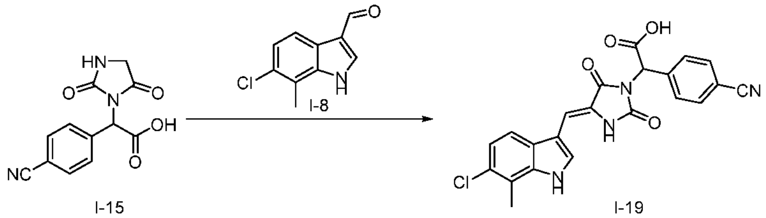


I-15 I-19

At room temperature, (4-cyanophenyl)(2,5-dioxoimidazolidin-1-yl)acetic acid (300 mg, 1.157 mmol) and piperazine (420 mg, 4.628 mmol) are added into an ethanol (3 mL) solution, and the mixture is heated to 65° C and stirred for 20 min. After the mixture is cooled to room temperature, 6-chloro-7-methyl-1H-indole-3-formaldehyde (157 mg, 0.810 mmol) is added in batches, and the obtained mixture is heated to 65° C and then stirred for 12 h. Through vacuum concentration, a residue is purified by reversed-phase chromatography to obtain the yellow solid **I-19** (270 mg, 59%).

LC-MS m/z (ESI): 433.0 [M-H]$^-$.

**I-20: Synthesis of (Z)-2-(4-((6-chloro-1H-indol-3-yl)methylene)-2,5-dioxoimidazolidin-1-yl)-2-(3,4-difluorophenyl)acetic acid**

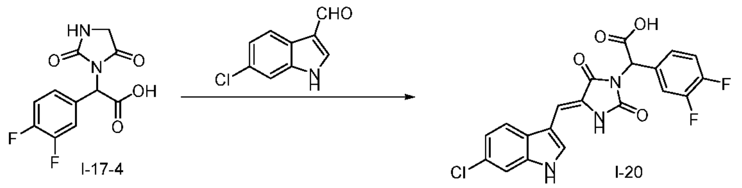

I-17-4 I-20

By using the method for the intermediate **I-19,** the red solid **I-20** (1.5 g, 84.77%) is prepared by using the **I-17-4** and corresponding aldehyde.

LC-MS m/z (ESI): 432.1 [M+H]$^+$.

**I-21: Synthesis of 2-(4-cyano-3-fluorophenyl)-2-(2,5-dioxoimidazolidin-1-yl)acetic acid**

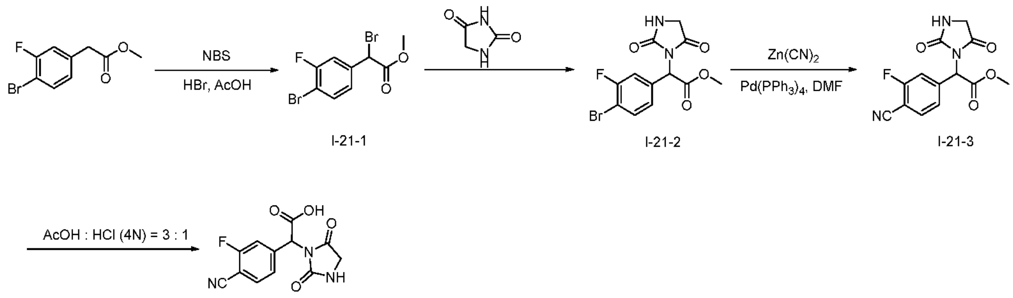


I-21-1 I-21-2 I-21-3

I-21

**I-21-1: Methyl 2-bromo-2-(4-bromo-3-fluorophenyl)acetate**

At room temperature, a solution of HBr in AcOH (2.3 mL, 16.261 mmol) is added into a solution of methyl 2-(4-bromo-3-fluorophenyl)acetate (3 g, 11.536 mmol) and NBS (2.81 g, 14.997 mmol) in DCE (30 mL), and the obtained mixture is stirred at 85° C for 12 h. The mixture is concentrated in vacuum, and a residue is diluted with water (100 mL). The obtained mixture is extracted with EtOAc (100 mL×3), and combined organic layers are washed with saline and dried with anhydrous $Na_2SO_4$. After the organic layers are filtered, a filtrate is concentrated in vacuum. A residue is purified by silica gel column chromatography and eluted with petroleum PE/EA (8:1) to obtain the yellow oily **I-21-1** (3.2 g, 77%).

**I-21-2: Methyl 2-(4-bromo-fluorophenyl)-2-(2,5-dioxoimidazolidin-1-yl)acetate**

At room temperature, hydantoin (4.36 g, 41.388 mmol) is added in batches into a stirred solution of methyl 2-bromo-2-(4-bromo-3-fluorophenyl)acetate (10 g, 27.611 mmol) and potassium carbonate (8.03 g, 55.222 mmol) in DMF (100 mL), and the obtained mixture is stirred under nitrogen protection at room temperature for 12 h. The obtained mixture is diluted with water (100 mL) and extracted with EtOAc (100 mL×3), and combined organic layers are washed with saline and dried with anhydrous $Na_2SO_4$. After the organic layers are filtered, a filtrate is concentrated in vacuum. A residue is purified by fast reversed-phase chromatography to obtain the white solid I-21-2 (3.9 g, 36.38%).
LC-MS m/z (ESI): 344.9 $[M+H]^+$.

**I-21-3: Methyl 2-(4-cyano-3-fluorophenyl)-2-(2, 5-dioxoimidazolidin-1-yl)acetate**

At room temperature, zinc cyanide (318 mg, 2.576 mmol) is added in batches into a stirred solution of methyl 2-(4-bromo-3-fluorophenyl)-2-(2, 5-dioxopyrrolidin-1-yl)acetate (1000 mg, 2.576 mmol) and tetrakis(triphenylphosphine) palladium (313 mg, 0.258 mmol) in DMF (10 mL), and the obtained mixture is stirred under nitrogen protection at 120° C for 16 h. The obtained mixture is concentrated in vacuum, and a residue is purified by fast reversed-phase chromatography to obtain the yellow solid I-21-3 (600 mg, 79%).
LC-MS m/z (ESI): 290.0 $[M-H]^-$.

**I-21: (4-cyano-3-fluorophenyl)(2,5-dioxoimidazolidin-1-yl)acetic acid**

At room temperature, HCl (2 mL, 4N) is added in batches into a solution of methyl 2-(4-cyano-3-fluorophenyl)-2-(2,5-dioxopyrrolidin-1-yl)acetate (800 mg, 2.747 mmol) in AcOH (6 mL), and the obtained mixture is stirred under nitrogen protection at 100° C for 1 h. A reaction solution is concentrated to obtain white solid **I-21** (700 mg, 92%). A crude product is directly used for a next step without further purification.
LC-MS m/z (ESI): 278.1 $[M+H]^+$.

**Examples**

**Example 1**

**Compound 1: (Z)-5-((6-chloro-1H-indol-3-yl)methylene)-3-(3,4-difluorobenzyl)imidazolidine-2,4-dione**

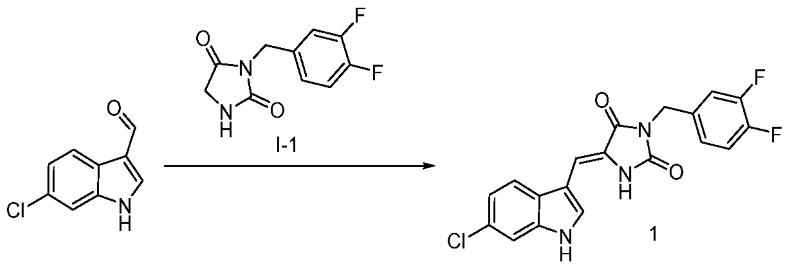


At room temperature, an intermediate **I-1** (119 mg, 0.505 mmol) and 6-chloro-1H-indole-3-formaldehyde (90 mg, 0.476 mmol) were dissolved in ethanol (5 ml). Under stirring, acetic acid (150 mg, 2.370 mmol) and piperidine (0.5 ml) were slowly added into the mixture, and a reaction solution was heated under nitrogen protection to 100° C and stirred for 2 h. After being cooled to room temperature, the reaction solution was concentrated in vacuum. A crude product was purified by a preparative liquid chromatography to obtain a yellow solid compound 1 (33.5 mg, 17.61%).
LC-MS m/z (ESI): 385.75 $[M-H]^-$. $^1H$ NMR (400 MHz, DMSO-$d_6$) δ 11.99 (brs, 1H), 10.51 (brs, 1H), 8.19 (s, 1H), 7.83 (d, $J$ = 8.4 Hz, 1H), 7.48 (d, $J$ = 2.0 Hz, 1H), 7.45-7.35 (m, 2H), 7.19-7.11 (m, 2H), 6.88 (s, 1H), 4.68 (s, 2H).

**Example 2:**

**Compound 2: (Z)-5-((6-chloro-1H-indol-3-yl)methylene)-3-(4-chlorobenzyl)imidazolidine-2,4-dione**

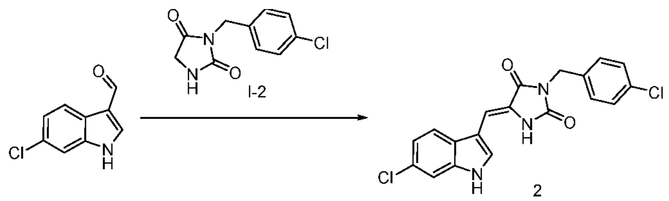


2

By using the method in Example **1**, the yellow solid compound **2** (104.6 mg, 32.42%) was prepared by using the **I-2** and corresponding aldehyde.

LC-MS m/z (ESI): 386.60 [M+H]+. $^1$H NMR (400 MHz, DMSO-*d6*) δ 11.96 (s, 1H), 10. 54-10. 30 (m, 1H), 8.16 (d, *J* = 1.9 Hz, 1H), 7.80 (d, *J* = 8.5 Hz, 1H), 7.45 (d, *J* = 1.8 Hz, 1H), 7.40-7.36 (m, 2H), 7.32-7.27 (m, 2H), 7.10 (dd, *J* = 8.5, 1.9 Hz, 1H), 6.85-6.83 (m, 1H), 4.64 (s, 2H).

**Example 3:**

**Compound 3: (Z)-5-((6-chloro-1H-indol-3-yl)methylene)-3-(4-fluorobenzyl)imidazolidine-2,4-dione**

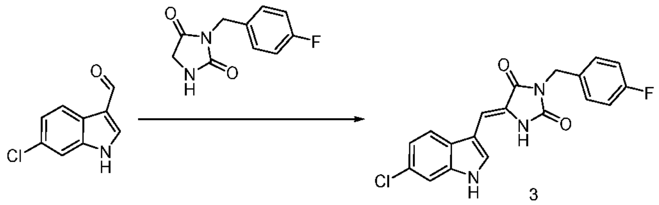

3

By using the method in Example **1**, the yellow solid compound 3 (75.8 mg, 14.24%) was prepared by using 3-(4-fluorobenzyl)imidazolidine-2,4-dione and corresponding aldehyde.

LC-MS m/z (ESI): 369.70 [M+H]+. $^1$H NMR (400 MHz, DMSO-d6) δ 11.99 (s, 1H), 10.50 (s, 1H), 8.19 (s, 1H), 7.83 (d, *J* = 8.5 Hz, 1H), 7.48 (d, *J* = 1.7 Hz, 1H), 7.36 (dd, *J* = 8.6, 5.6 Hz, 2H), 7.24-7.10 (m, 3H), 6.87 (s, 1H), 4.67 (s, 2H).

**Example 4**

**Compound 4: (Z)-4-((4-((6-chloro-1H-indol-3-yl)methylene)-2,5-dioxoimidazolidin-1-yl)methyl)benzonitrile**

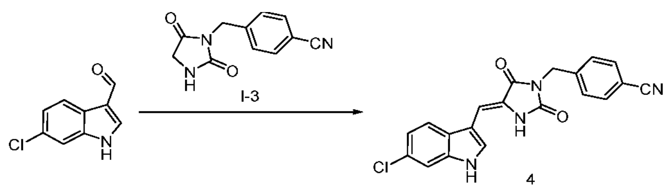


4

By using the method in Example **1**, the white solid compound 4 (12.3 mg, 2.34%) was prepared by using the **I-3** and corresponding aldehyde.

LC-MS m/z (ESI): 376.70 [M+H]+. $^1$H NMR (400 MHz, DMSO-$d_6$) δ 12.01 (s, 1H), 10.56 (s, 1H), 8.21 (d, *J* = 2.5 Hz, 1H), 7.84 (dd, *J* = 8.3, 1.6 Hz, 3H), 7.50 (d, *J* = 8.2 Hz, 3H), 7.14 (dd, *J* = 8.5, 1.8 Hz, 1H), 6.90 (s, 1H), 4.78 (s, 2H).

Example 5

**Compound 5: (Z)-5-((6-bromo-1H-indol-3-yl)methylene)-3-(4-chlorobenzyl)imidazolidine-2,4-dione**

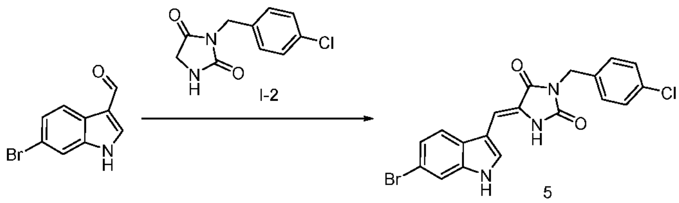


5

By using the method in Example **1**, the yellow solid compound **5** (15.6 mg, 6.73%) was prepared by using the **I-2** and corresponding aldehyde.

LC-MS m/z (ESI): 430.50 $[M+H]^+$. $^1H$ NMR (400 MHz, DMSO-*d6*) δ 12.00 (s, 1H), 10.45 (s, 1H), 8.18 (s, 1H), 7.78 (d, *J* = 8.5 Hz, 1H), 7.62 (d, *J* = 1.3 Hz, 1H), 7.42 (d, *J* = 8.4 Hz, 2H), 7.33 (d, *J* = 8.4 Hz, 2H), 7.28-7.23 (m, 1H), 6.87 (s, 1H), 4.68 (s, 2H).

## Example 6

### Compound 6: (Z)-5-((6-chloro-7-(fluoromethoxyl)-1H-indol-3-yl)methylene)-3-(3,4-difluorobenzyl)imidazolidine-2,4-dione

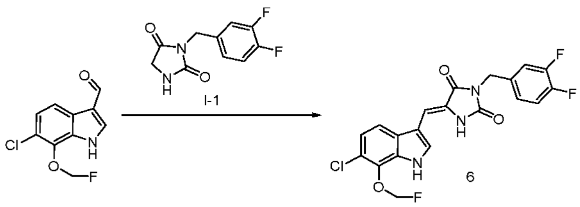


6

By using the method in Example **1**, the yellow solid compound **6** (32.5 mg, 34. 43%) was prepared by using the **I-1** and corresponding aldehyde.

$^1H$ NMR (400 MHz, DMSO-*d6*) δ 12.20 (s, 1H), 10. 57 (s, 1H), 8.22 (s, 1H), 7. 70 (m, 1H), 7.45-7.37 (m, 2H), 7.21 (d, *J* = 8.5 Hz, 1H), 7.16 (s, 1H), 6.86 (s, 1H), 5. 90 (s, 1H), 5.77 (s, 1H), 4.68 (s, 2H).

## Example 7

### Compound 7: (Z)-5-((6, 7-dichloro-1H-indol-3-yl)methylene)-3-(3,4-difluorobenzyl)imidazolidine-2,4-dione

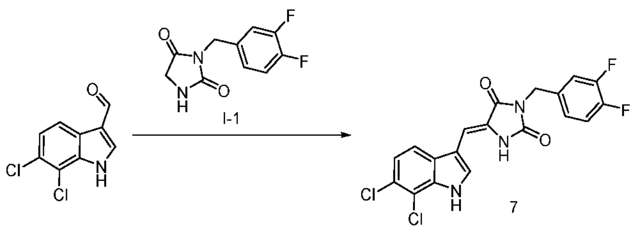


7

By using the method in Example **1**, the yellow solid compound **7** (21.6 mg, 4.95%) was prepared by using the compound **I-1** and corresponding aldehyde.

LC-MS m/z (ESI): 420.05 $[M-H]^-$. $^1H$ NMR (400 MHz, DMSO-d6) δ 12.36 (s, 1H), 10.62 (s, 1H), 8.27 (s, 1H), 7.85 (d, *J* = 8.5 Hz, 1H), 7.41 (ddd, *J* = 13.5, 10.0, 6.9 Hz, 2H), 7.31 (d, *J* = 8.5 Hz, 1H), 7.16 (s, 1H), 6.86 (s, 1H), 4.68 (s, 2H).

## Example 8

### Compound 8: (Z)-5-((6-chloro-7-methoxyl-1H-indol-3-yl)methylene)-3-(3,4-difluorobenzyl)imidazolidine-2,4-dione

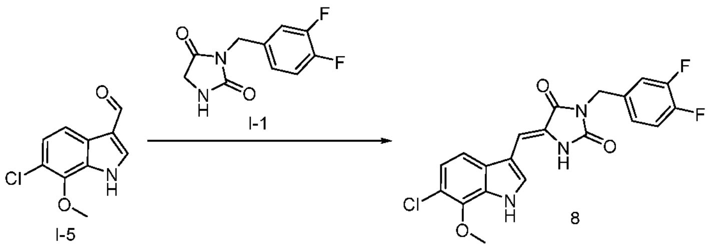

By using the method in Example **1**, the yellow solid compound **8** (40 mg, 16.56%) was prepared by using the intermediates **I-5** and **I-1.**

LC-MS m/z (ESI): 417.9 $[M+H]^+$. $^1$H NMR (400 MHz, DMSO-$d_6$) δ 12.19 (d, *J* = 2.4 Hz, 1H), 10.53 (s, 1H), 8.17 (d, *J* = 2.6 Hz, 1H), 7.54 (d, *J* = 8.5 Hz, 1H), 7.37 (ddt, *J* = 10.0, 8.1, 5.3 Hz, 2H), 7.14-7.08 (m, 2H), 6.81 (s, 1H), 4.64 (s, 2H), 3.89 (s, 3H).

**Example 9**

**Compound 9: (Z)-5-((6-chloro-7-(methylsulfonyl)-1H-indol-3-yl)methylene)-3-(3,4-difluorobenzyl)imidazolidine-2,4-dione**

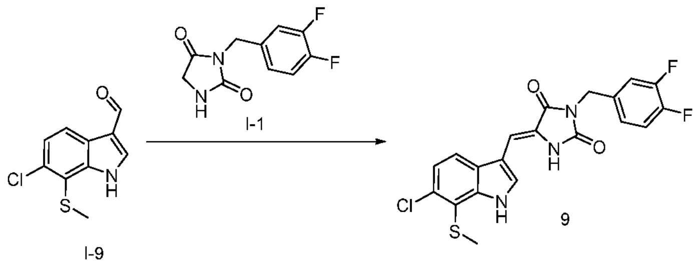

By using the method in Example **1**, the yellow solid compound **9** (12.7 mg, 33.28%) was prepared by using the intermediates **I-9** and **I-1**.

LC-MS m/z (ESI): 433.9 $[M+H]^+$. $^1$H NMR (300 MHz, DMSO-$d_6$) 12.06 (s, 1H), 10.63 (s, 1H), 8.25 (s, 1H), 7.86-7.83 (m, 1H), 7.44-7.29 (m, 3H), 7.26-7.16 (m, 1H), 6.86 (s, 1H), 4.68 (s, 2H), 2.43 (s, 3H).

**Example 10**

**Compound 10: (Z)-5-((6-chloro-7-fluoro-1H-indol-3-yl)methylene)-3-(3,4-difluorobenzyl)imidazolidine-2,4-dione**

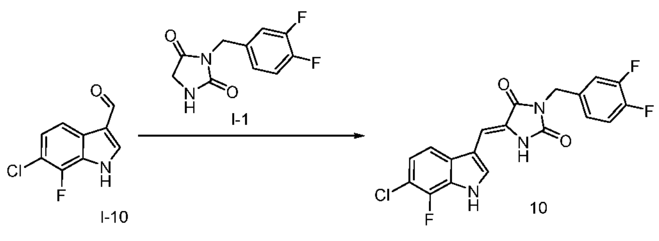

By using the method in Example **1**, the yellow solid compound **10** (19.5 mg, 13.35%) was prepared by using the intermediates **I-10** and **I-1.**

LC-MS m/z (ESI): 403.8 $[M-H]^-$. $^1$H NMR (300 MHz, DMSO-$d_6$) δ 12.54 (s, 1H), 10.57 (s, 1H), 8.25 (s, 1H), 7.68 (d, *J* = 8.5 Hz, 1H), 7. 46-7. 36 (m, 2H), 7.23-7.18 (m, 2H), 6.86 (s, 1H), 4.68 (s, 2H).

**Example 11**

**Compound 11: (Z)-4-((4-((6-chloro-7-(fluoromethoxyl)-1H-indol-3-yl)methylene)-2,5-dioxoimidazolidin-1-yl) methyl)-2-fluorobenzonitrile**

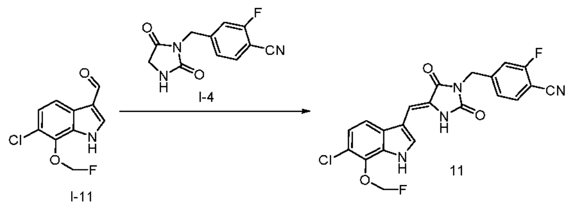


By using the method in Example **1,** the yellow solid compound **11** (9.8 mg, 2.2%) was prepared by using the intermediates **I-11** and **I-4.**

LC-MS m/z (ESI): 443.1 $[M+H]^+$. 1H NMR (300 MHz, DMSO-$d_6$) δ 12.21 (s, 1H), 10.63 (s, 1H), 8.24 (s, 1H), 7.94-7.89 (m, 1H), 7.73-7.70 (m, 1H), 7.52-7.48 (m, 1H), 7.35-7.33 (m, 1H), 7.23-7.20 (m, 1H), 6.88 (s, 1H), 5.93 (s, 1H), 5.75 (s, 1H), 4.80 (s, 2H).

Example 12

**Compound 12: (Z)-5-((6-chloro-7-(fluoromethoxyl)-1H-indol-3-yl)methylene)-3-(4-chlorobenzyl)imidazolidine-2,4-dione**

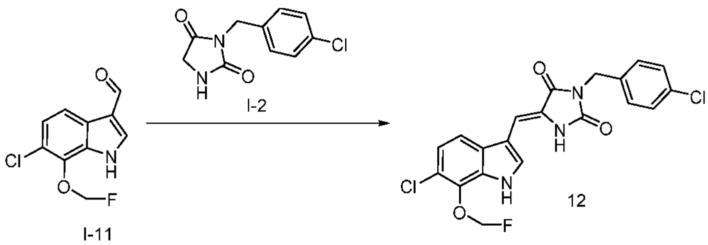


By using the method in Example **1,** the yellow solid compound **12** (8.9 mg, 5.18%) was prepared by using the intermediates **I-11** and **I-2.**

LC-MS m/z (ESI): 431.8 $[M-H]^-$. $^1$H NMR (300 MHz, DMSO-$d_6$) δ 12.20 (s, 1H), 10.58 (s, 1H), 8.22 (s, 1H), 7.72 (d, *J* = 8.6 Hz, 1H), 7.44-7.40 (m, 2H), 7.35-7.22 (m, 2H), 7.17-7.27 (m, 1H), 6.86 (s, 1H), 5.93 (s, 1H), 5.75 (s, 1H), 4.68 (s, 2H).

**Example 13**

**Compound 13: (Z)-4-((4-((6-chloro-7-(fluoromethoxyl)-1H-indol-3-yl)methylene)-2,5-dioxoimidazolidin-1-yl) methyl)benzonitrile**

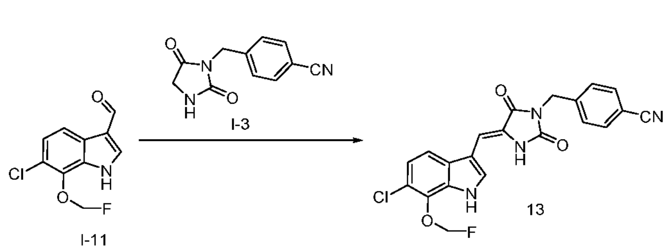


By using the method in Example **1**, the yellow solid compound **13** (8.1 mg, 4.68%) was prepared by using the intermediates **I-11** and **I-3**.

LC-MS m/z (ESI): 422.8 $[M-H]^-$. $^1$H NMR (300 MHz, DMSO- $d_6$) δ 12.21 (s, 1H), 10.62 (s, 1H), 8.23 (m, 1H), 7.85-7.82 (m, 2H), 7.72-7.69 (m, 1H), 7.51-7.48 (m, 2H), 7.22-7.20 (m, 1H), 6.87 (s, 1H), 5.93 (s, 1H), 5.75 (s, 1H), 4.78 (s,

2H).

**Example 14**

**Compound 14: -(Z)-4-((4-((6-chloro-7-fluoro-1H-indol-3-yl)methylene) 2,5-dioxoimidazolidin-1-yl)methyl)-2-fluorobenzonitrile**

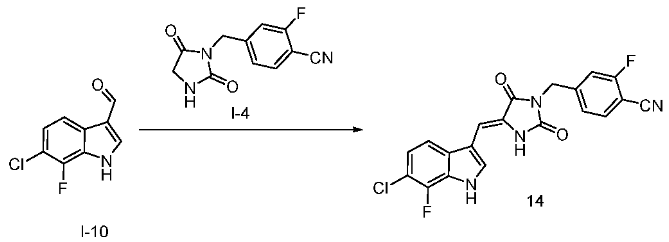

By using the method in Example 1, the yellow solid compound **14** (6.6 mg, 4.89%) was prepared by using the intermediates **I-10** and **I-4.**

LC-MS m/z (ESI): 410.9 [M-H]$^-$. $^1$H NMR (400 MHz, DMSO-$d_6$) δ 12.57 (s, 1H), 10.63 (s, 1H), 8.28 (s, 1H), 7.95-7.90 (m, 1H), 7.71-7.68 (m, 1H), 7.52-7.48 (m, 1H), 7.35-7.32 (m, 1H), 7.24-7.19 (m, 1H), 6.87 (s, 1H), 4.80 (s, 2H).

**Example 15**

**Compound 15: (Z)-5-((6-chloro-7-fluoro-1H-indol-3-yl)methylene)-3-(4-chlorobenzyl)imidazolidine-2,4-dione**

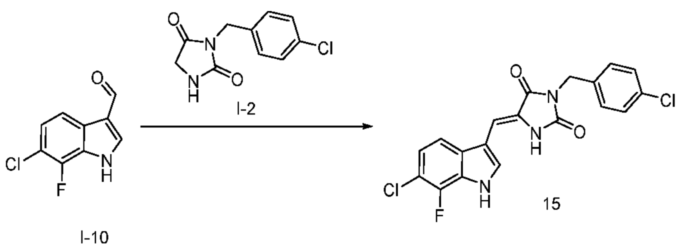

By using the method in Example 1, the yellow solid compound **15** (9.4 mg, 7.51%) was prepared by using the intermediates **I-10** and I-2.

LC-MS m/z (ESI): 401.70 [M-H]$^-$. $^1$H NMR (400 MHz, DMSO- $d_6$) 12.53 (s, 1H), 10.56 (s, 1H), 8.25 (s, 1H), 7.67 (d, $J$ = 8.4 Hz, 1H), 7.43-7.33 (m, 4H), 7.22-7.19 (m, 1H), 6.85 (s, 1H), 4.68 (s, 2H).

**Example 16**

**Compound 16: (Z)-4-((4-((6-chloro-7-fluoro-1H-indol-3-yl)methylene)-2,5-dioxoimidazolidin-1-yl)methyl)benzonitrile**

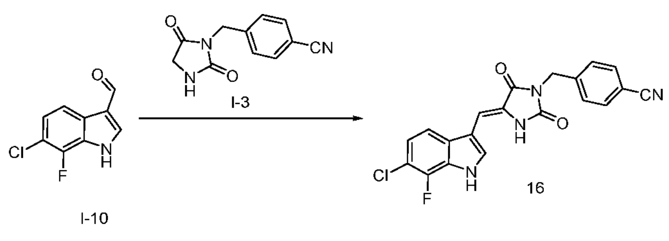

By using the method in Example 1, the yellow solid compound **16** (25.9 mg, 12.16%) was prepared by using the intermediates **I-10** and **I-3.**

LC-MS m/z (ESI): 392.8 [M-H]⁻. $^1H$ NMR (400 MHz, DMSO-$d_6$) δ 12.54 (s, 1H), 10.60 (s, 1H), 8.26 (s, 1H), 7.83 (d, *J* = 8.0 Hz 2H), 7.68 (d, *J* = 8.4 Hz, 1H), 7.50 (d, *J* = 8 4 Hz, 2H), 7.21 (t, *J* = 6.8 Hz, 1H), 6.87 (s, 1H), 4.78 (s, 2H).

**Example 17**

**Compound 17: (Z)-5-((6-chloro-7-fluoro-1H-indol-3-yl)methylene)-3-(4-acetenylbenzyl)imidazolidine-2,4-dione**

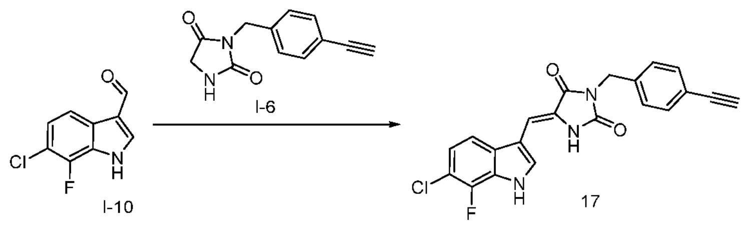

By using the method in Example 1, the yellow solid compound **17** (23.1 mg, 22.88%) was prepared by using the intermediates **I-10** and **I-6.**

LC-MS m/z (ESI): 391.9 [M-H]⁻. $^1H$ NMR (300 MHz, DMSO-$d_6$) δ 12.56 (s, 1H), 10. 59 (s, 1H), 8.26 (d, *J* = 2.8 Hz, 1H), 7.67 (d, *J* = 8.6 Hz, 1H), 7. 50-7.43 (m, 2H), 7.31 (d, *J* = 8.3 Hz, 2H), 7.20 (dd, *J* = 8.5, 6.5 Hz, 1H), 6.85 (s, 1H), 4.70 (s, 2H), 4.18 (s, 1H).

**Example 18**

**Compound 18: -(Z)-3-((4-((6-chloro-7-fluoro-1H-indol-3-yl)methylene)-2,5-dioxoimidazolidin-1-yl)methyl)benzonitrile**

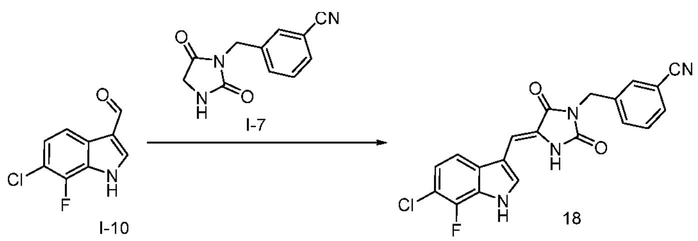

By using the method in Example 1, the yellow solid compound **18** (12.8 mg, 14.52%) was prepared by using the intermediates **I-10** and **I-7.**

LC-MS m/z (ESI): 392.8 [M-H]⁻. $^1H$ NMR (300 MHz, DMSO-$d_6$) δ 12.60-12.39 (m, 1H), 10.58 (s, 1H), 8.82-8.21 (m, 1H), 7.82-7.73 (m, 2H), 7. 72-7. 57 (m, 3H), 7.33-7.10 (m, 1H), 6.80 (s, 1H), 4.75 (s, 2H).

**Example 19**

**Compound 19: (Z)-4-((4-((6-chloro-7-methyl-1H-indol-3-yl)methylene)-2,5-dioxoimidazolidin-1-yl)methyl)benzonitrile**

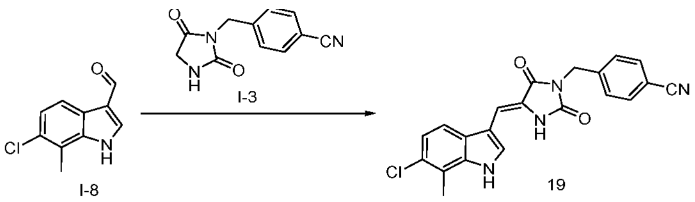


By using the method in Example **1,** the yellow solid compound **19** (47 mg, 23%) was prepared by using the intermediates **I-3** and **I-8.**

LC-MS m/z (ESI): 388.8 [M-H]$^-$. $^1$H NMR (300 MHz, DMSO-$d_6$) δ 12.00 (s, 1H), 10.55 (s, 1H), 8.21 (d, $J$ = 2.5 Hz, 1H), 7.84-7.78 (m, 2H), 7.64 (d, $J$ = 8.5 Hz, 1H), 7.54-7.45 (m, 2H), 7.14 (d, $J$ = 8.5 Hz, 1H), 6.87 (d, $J$ = 0.6 Hz, 1H), 4.78 (s, 2H), 2.52 (s, 3H).

**Example 20**

**Compound 20: (Z)-4-((4-((6-chloro-1H-indol-3-yl)methylene)-2,5-dioxoimidazolidin-1-yl)methyl)-2-fluorobenzonitrile**

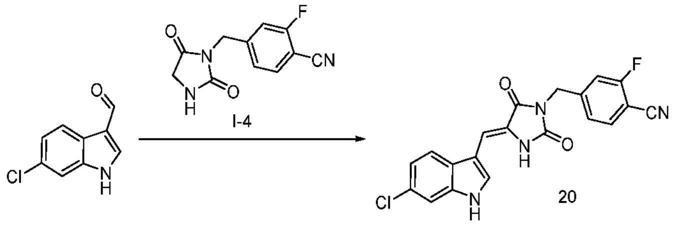


By using the method in Example **1**, the yellow solid compound **20** (27 mg, 12%) was prepared by using the intermediate **I-4** and corresponding aldehyde.

LC-MS m/z (ESI): 393.0 [M+H]$^-$. $^1$H NMR (400 MHz, DMSO-$d$6) δ 12.01-11.75 (m, 1H), 10.53 (m, 1H), 8.48 (m, 1H), 7.91 (t, $J$ = 7.5 Hz, 1H), 7.83 (d, $J$ = 8.5 Hz, 1H), 7.67-7.45 (m, 2H), 7.33 (d, $J$ = 8.1 Hz, 1H), 7.15 (m, 1H), 6.84 (m, 1H), 4.79 (s, 2H).

**Example 21**

**Compound 21: (Z)-4-((4-((6-chloro-7-methyl-1H-indol-3-yl)methylene)-2, 5-dioxoimidazolidin-1-yl)methyl)-2-fluorobenzonitrile**

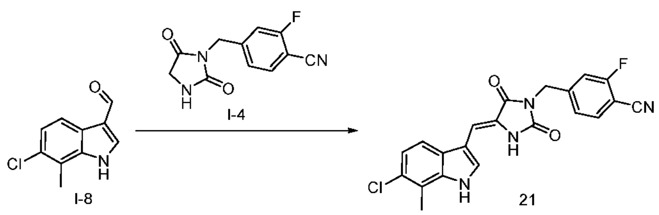


By using the method in Example 1, the yellow solid compound **21** (42 mg, 18%) was prepared by using the intermediates **I-4** and **I-8.**

LC-MS m/z (ESI): 407.1 [M-H]$^-$. $^1$H NMR (300 MHz, DMSO-$d_6$) δ 12.02 (s, 1H), 10.57 (s, 1H), 8.22 (d, $J$ = 2.9 Hz, 1H), 7.91 (t, $J$ = 7.4 Hz, 1H), 7.65 (d, $J$ = 8.5 Hz, 1H), 7.54-7.44 (m, 1H), 7.33 (dd, $J$ = 8.1, 1.6 Hz, 1H), 7.15 (d, $J$ = 8.5 Hz, 1H), 6.88 (s, 1H), 4.80 (s, 2H), 2.52 (s, 3H).

**Example 22**

**Compound 22: (Z)-2-(4-((6-chloro-1H-indol-3-yl)methylene)-2,5-dioxoimidazolidin-1-yl)-2- (4-chlorophenyl) -N-(1, 3-dihydroxypropan-2-yl)acetamide**

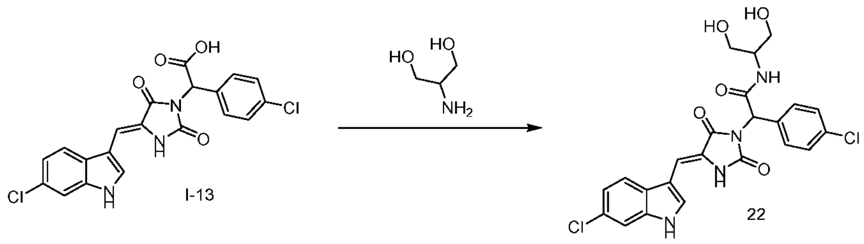

The intermediate **I-13** (150 mg, 0.35 mmol), HATU (199 mg, 0.52 mmol), and N,N-diisopropylethylamine (90 mg, 0.7 mmol) were added into a dimethylformamide (5 mL) solution. The mixture was stirred at 25° C for 10 min, and then 2-aminopropane-1,3-diol (47 mg, 0.52 mmol) was added into the mixture. The mixture was continuously stirred at 25° C for 2 h, concentrated in vacuum, and purified by preparative thin-layer chromatography to obtain a yellow solid compound **22** (160 mg, 90%).

LC-MS m/z (ESI): 502.9 $[M+H]^+$. $^1H$ NMR (400 MHz, DMSO-$d_6$) δ 11.98 (d, *J* = 2.0 Hz, 1H), 10.53 (s, 1H), 8.19 (d, *J* = 2.5 Hz, 1H), 7.82 (d, *J* = 8.5 Hz, 1H), 7.75 (d, *J* = 8.2 Hz, 1H), 7.48 (d, *J* = 1.8 Hz, 1H), 7. 46-7. 39 (m, 4H), 7. 13 (dd, *J* = 8.5, 1.9 Hz, 1H), 6.83 (s, 1H), 5.77 (s, 1H), 4.63 (t, *J* = 5.5 Hz, 1H), 4.49 (t, *J* = 5.7 Hz, 1H), 3.85-3.77 (m, 1H), 3. 47-3. 35 (m, 4H).

**Example 23**

**Compound 23: (Z)-2-(4-((6-chloro-1H-indol-3-yl)methylene)-2,5-dioxoimidazolidin-1-yl)-2- (4-chlorophenyl)-N-(2-(diethylamino)ethyl) acetamide**

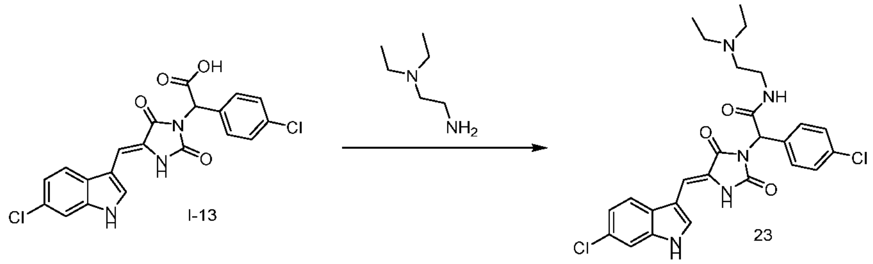

By using the method in Example **22,** the yellow solid compound **23** (180 mg, 58.61%) was prepared by using the intermediate **I-13** and N,N-diethenylethane-1, 2-diamine.

LC-MS m/z (ESI): 527.9 $[M+H]^+$. $^1H$NMR (400 MHz, DMSO-$d_6$) δ 12.08 (s, 1H), 10.68 (s, 1H), 8.26 (d, *J* = 2.7 Hz, 2H), 7.87 (d, *J* = 8.6 Hz, 1H), 7.54 (d, *J* = 1.8 Hz, 1H), 7.51 (s, 4H), 7.20 (dd, *J* = 8.5, 1.9 Hz, 1H), 6.93 (s, 1H), 5.86 (s, 1H), 3.51 (m, 2H), 3.27-3.15 (m, 6H), 1.24 (t, *J* = 7.1 Hz, 6H).

**Example 24**

**Compound 24: (Z)-2-(4-((6-chloro-1H-indol-3-yl)methylene)-2,5-dioxoimidazolidin-1-yl)-2-(3,4-difluorophenyl)-N-(1,3-dihydroxypropan-2-yl) acetamide**

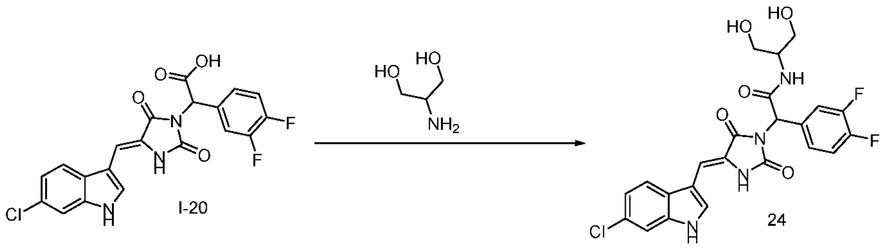


By using the method in Example **22,** the yellow solid compound **24** (30 mg, 24.98%) was prepared by using the intermediate **I-20** and 2-aminopropane-1,3-diol.

LC-MS m/z (ESI): 502.9 [M-H]$^-$. $^1$HNMR (400 MHz, DMSO-$d_6$) δ 11.98 (s, 1H), 10.53 (s, 1H), 8.19 (s, 1H), 7.87-7.77 (m, 2H), 7.55-7.36 (m, 3H), 7.30-7.28 (m, 1H), 7.18-7.10 (m, 1H), 6.84 (s, 1H), 5.79 (s, 1H), 4.64-4.61 (m, 1H), 4.50-4.47 (m, 1H), 3. 85-3. 80 (m, 1H), 3. 52-3. 43 (m, 4H).

### Example 25

**Compound 25: (Z)-5-((6-chloro-7-methoxyl-1H-indol-3-yl)methylene)-3-(1-(3,4-difluorophenyl)ethyl)imidazolidine-2,4-dione**

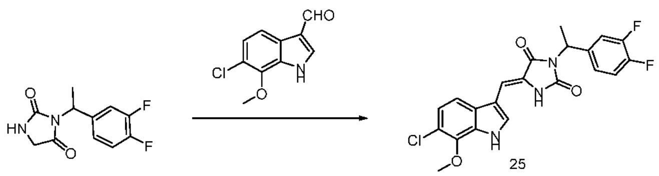


By using the method in Example **1,** the yellow solid compound **25** (7.3 mg, 0.76%) was prepared.

LC-MS m/z (ESI): 432.0 [M+H]$^+$. $^1$HNMR (400 MHz, DMSO-$d_6$) δ 12.19 (s, 1H), 10.51 (s, 1H), 8. 18 (d, *J* = 2.5 Hz, 1H), 7.55 (d, *J* = 8.6 Hz, 1H), 7. 52-7. 36 (m, 2H), 7. 23 (s, 1H), 7. 14 (d, *J* = 8.6 Hz, 1H), 6. 79 (s, 1H), 5. 41-5. 29 (m, 1H), 3.93 (s, 3H), 1. 78 (d, *J* = 7.2 Hz, 3H).

### Example 26

**Compound 26: 6-chloro-3-{[(4Z)-1-[1-(3,4-difluorophenyl)-2-hydroxyethyl]-2,5-dioxoimidazolidin-4-methylene] methyl}indol-1-carboxylate**

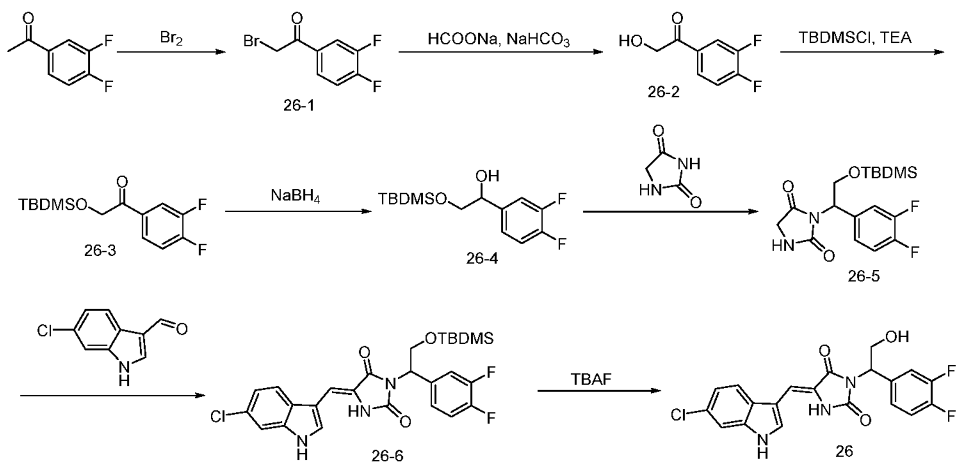

**26-1: 2-bromo-1-(3,4-difluorophenyl)ethanone**

At 0° C, bromine (2 g, 11.889 mmol) was slowly added into a solution of 1-(3,4-difluorophenyl)ethanone (2 g, 12.169 mmol) in dichloromethane (20 mL). After the reaction was finished, the mixture was poured into ice water. The obtained mixture was extracted with dichloromethane (40 mL) three times. Organic phases were combined, washed with saturated saline (20 mL), and dried with anhydrous sodium sulfate. After the organic phases were filtered, a filtrate was concentrated at a reduced pressure. A residue was purified by silica gel column chromatography and eluted with DCM/PE(1:20) to obtain the white solid **26-1** (1.68 g, 58%).

$^1$H NMR (300 MHz, DMSO-$d_6$) δ 8.15-8.05 (m, 1H), 7.98-7.86 (m, 1H), 7.70-7.60 (m, 1H), 4.95 (s, 2H).

**26-2: 1-(3,4-difluorophenyl)-2-hydroxyethanone**

At room temperature, 2-bromo-1-(3,4-difluorophenyl)ethanone (2 g, 8.399 mmol), sodium formate (6.97 g, 97.365 mmol), and sodium hydrogen carbonate (800 mg, 9.047 mmol) were added into a mixed solution of acetonitrile (8 mL) and water (4 mL). The obtained mixture was heated to 65° C under nitrogen protection and stirred for 18 h. After the reaction was completed, the mixture was cooled to room temperature. The obtained mixture was poured into water (5 mL). The mixture was extracted with ethyl acetate (20 mL) three times. Organic phases were combined, washed with saturated saline (10 mL) three times, and dried with anhydrous sodium sulfate. After the organic phases were filtered, a filtrate was concentrated in vacuum. A residue was purified by silica gel column chromatography and eluted with petroleum ether/ethyl acetate (2:1) to obtain the yellow solid **26-2** (610 mg, 39%).

$^1$HNMR (300 MHz, DMSO-d6) δ 8.05-7.95 (m, 1H), 7.90-7.80 (m, 1H), 7.75-7. 62 (m, 1H), 5. 26-5. 18 (m, 1H), 4. 82-4. 74 (m, 2H).

**26-3: 2-[(tert-butyldimethylsilyl)oxy]-1-(3,4-difluorophenyl)ethanone**

At room temperature, 1-(3, 4-difluorophenyl)-2-hydroxyethanone (2.1 g, 11.346 mmol) and triethylamine (1.48 g, 13.894 mmol) were added into a dichloromethane (20 mL) solution, and tert-butyl dimethyl chlorosilane (2.76 g, 17.396 mmol) was added into the solution when the solution was stirred. The obtained mixture was stirred at room temperature under nitrogen protection for 16 h. The mixture was poured into ice water, extracted with ethyl acetate (80 mL) three times, and combined organic phases were washed with saturated saline (50 mL) three times and dried with anhydrous sodium sulfate. After the organic layers were filtered, a filtrate was concentrated in vacuum. A residue was purified by silica gel column chromatography and eluted with DCM/PE (1:2) to obtain the yellow oily **26-3** (2.48 g, 69%).

LC-MS m/z (ESI): 285.0 $[M-H]^-$.

**26-4: 2-[(tert-butyldimethylsilyl)oxy]-1-(3,4-difluorophenyl)ethanol**

Under nitrogen protection, 2-[(tert-butyldimethylsilyl)oxy]-1-(3,4-difluorophenyl)ethanone (2.0 g, 6.299 mmol) was added into an ethanol (25 mL) solution, and at 0° C, sodium borohydride (800 mg, 20.090 mmol) was added in batches. After being stirred at room temperature for 0.5 h, the mixture was poured into ice water and extracted with ethyl acetate (10 mL) three times. Organic phases were combined, washed with saturated saline (5 mL) three times, and dried with anhydrous sodium sulfate. After the organic phases were filtered, a filtrate was concentrated in vacuum. A residue was purified by silica gel column chromatography and eluted with petroleum ether/ethyl acetate (1:20) to obtain the brown oily compound **26-4** (1.56 g, 86%).

$^1$HNMR (400 MHz, DMSO-d6) δ 7. 45-7. 35 (m, 2H), 7. 30-7. 20 (m, 1H), 5. 56-5. 50 (m, 1H), 4. 70-4. 60 (m, 1H), 3. 80-3. 70 (m, 1H), 3. 65-3. 55 (m, 1H) 0. 86 (s, 9H), 0.07 (s, 6H).

**26-5: 3-{2-[(tert-butyldimethylsilyl)oxy]-1-(3,4-difluorophenyl)ethyl}imidazolidine-2,4-dione**

At 0° C, 2-[(tert-butyldimethylsilyl)oxy]-1-(3,4-difluorophenyl)ethanol (1.56 g, 5. 404 mmol) and triphenylphosphine (2.84 g, 10.286 mmol) were added into a THF (20 mL) solution, and under nitrogen protection, diethyl azodicarbonate (1.88 g, 10.255 mmol) was added into the mixture. The obtained mixture was heated to 40° C and stirred for 16 h. After the system was cooled to room temperature, the obtained mixture was concentrated in vacuum. A residue was purified by reversed-phase chromatography to obtain the brown oily compound **26-5** (640 mg, 25%).

LC-MS m/z (ESI): 371.2 $[M+H]^+$.

**26-6: (5Z) 3-{2-[(tert-butyldimethylsilyl)oxy]-1-(3,4-difluorophenyl)ethyl}-5-[(6-chloro-1H-indol-3-yl)methylene] imidazolidine-2,4-dione**

At room temperature, 3-{2-[(tert-butyldimethylsilyl)oxy]-1-(3, 4-difluorophenyl)ethyl}imidazolidine-2,4-dione (440 mg, 0.927 mmol) and 6-chloro-1H-indole-3-formaldehyde (236 mg, 1.25 mmol) were added into an ethanol (5 mL) solution, and piperidine (0. 5 mL) was dropwise added into the system at room temperature. The obtained mixture was heated to 100° C and stirred for 4 h. After being cooled to room temperature, the mixture was concentrated in vacuum. A residue was purified by reversed-phase chromatography to obtain the yellow solid compound solid 26-6 (325 mg, 37%).
LC-MS m/z (ESI): 532.3 $[M+H]^+$.

**Compound 26: 6-chloro-3-{[(4Z)-1-[1-(3,4-difluorophenyl)-2-hydroxyethyl]-2,5-dioxoimidazolidin-4-methylene] methyl}indol-1-carboxylate**

At room temperature, (Z)-3-(2-(tert-butyldimethylsilyloxy)-1-(3,4-difluorophenyl)ethyl)-5-((6-chloro-1H-indol-3-yl)methylene)imidazolidine-2,4-dione (263 mg, 0.457 mmol) was added into a tetrahydrofuran (6 mL) solution, and tetramethylammonium fluoride (3.66 mL, 3.66 mmol, 1N) was dropwise added into the solution. The obtained mixture was stirred at room temperature for 1 h. The obtained mixture was concentrated in vacuum. A residue was purified by preparative high performance liquid chromatography to obtain the yellow solid compound solid **26** (2.4 mg, 29.66%).
LC-MS m/z (ESI): 416.1 $[M-H]^-$. $^1$HNMR (300 MHz, DMSO-d6) δ 12.03-11.73 (m, 1H), 10.50 (s, 1H), 8.79-8.14 (m, 1H), 7.86-7.61 (m, 1H), 7.56-7.36 (m, 3H), 7.27 (dd, *J* = 5.2, 3.0 Hz, 1H), 7. 18-7. 10 (m, 1H), 6.84 (s, 1H), 5.20 (dt, *J* = 10.7, 5.7 Hz, 2H), 4. 31 (td, *J* = 10.3, 5.4 Hz, 1H), 3.94 (dt, *J* = 11.2, 5.8 Hz, 1H).

**Example 27**

**Compound 27: (Z)-2-(4-((6-chloro-7-fluoro-1H-indol-3-yl)methylene)-2,5-dioxoimidazolidin-1-yl)-2-(4-cyano-3-fluorophenyl)-N-(1,3-dihydroxypropan-2-yl)acetamide**

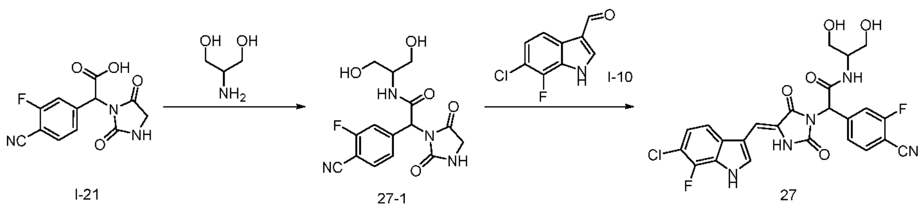


I-21 27-1 27

**27-1: 2-(4-cyano-3-fluorophenyl)-N-(1,3-dihydroxypropan-2-yl)-2-(2,5-dioxoimidazolidin-1-yl)acetamide**

At room temperature, 2-aminopropane-1, 3-diol (62 mg, 0.686 mmol) and HATU (261 mg, 0.686 mmol) were added into dimethylformamide (1 mL), and (4-cyano-3-fluorophenyl)(2,5-dioxoimidazolidin-1-yl)acetic acid (100 mg, 0.343 mmol) and diisopropylethylamine (74 mg, 0.686 mmol) were added in batches into the solution. The obtained mixture was heated to 40° C under nitrogen protection and stirred for 12 h. The mixture was concentrated in vacuum, and a residue was purified by reversed-phase chromatography to obtain the yellow solid compound solid **27-1** (60 mg, 50%).
LC-MS m/z (ESI): 349.0 $[M-H]^-$.

**Compound 27: (Z)-2-(4-((6-chloro-7-fluoro-1H-indol-3-yl)methylene)-2,5-dioxoimidazolidin-1-yl)-2-(4-cyano-3-fluorophenyl)-N-(1,3-dihydroxypropan-2-yl)acetamide**

At room temperature, 2-(4-cyano-3-fluorophenyl)-N-(1,3-dihydroxypropan-2-yl)-2-(2, 5-dioxoimidazolidin-1-yl) acetamide (20 mg, 0.051 mmol) was added into ethyl acetate (1 mL), piperidine (18.42 mg, 0.204 mmol) was added in batches into the mixed solution, and the obtained mixture was reflowed for 20 min. After the mixture was cooled to room temperature, 6-chloro-7-fluoro-1H-indole-3-formaldehyde (8 mg, 0.036 mmol) was added in batches. The obtained mixture was then reflowed for 12 h, and after being cooled to room temperature, the mixture was concentrated in vacuum. A residue was purified by preparative high performance liquid chromatography to obtain the yellow solid compound solid **27** (4.3 mg, 15.35%).
LC-MS m/z (ESI): 528.1 $[M-H]^-$. $^1$H NMR (400 MHz, DMSO-$d_6$) δ 12. 55 (d, *J* = 3.0 Hz, 1H), 10.63 (s, 1H), 8.71-8.20 (m, 1H), 8.04 (d, *J* = 8.2 Hz, 1H), 7.96-7.80 (m, 1H), 7. 76-7. 55 (m, 2H), 7.43 (d, *J* = 8.1 Hz, 1H), 7.25-7.16 (m, 1H),

6.83 (s, 1H), 5.91 (s, 1H), 4.58 (dt, *J* = 57. 7, 5.7 Hz, 2H), 4.10-3.77 (m, 1H), 3. 56-3. 35 (m, 4H).

**Example 28**

**Compound 28: (Z)-2-(4-((6-chloro-7-fluoro-1H-indol-3-yl)methylene)-2,5-dioxoimidazolidin-1-yl)-2-(4-chlorophenyl)-N-(1,3-dihydroxypropan-2-yl)acetamide**

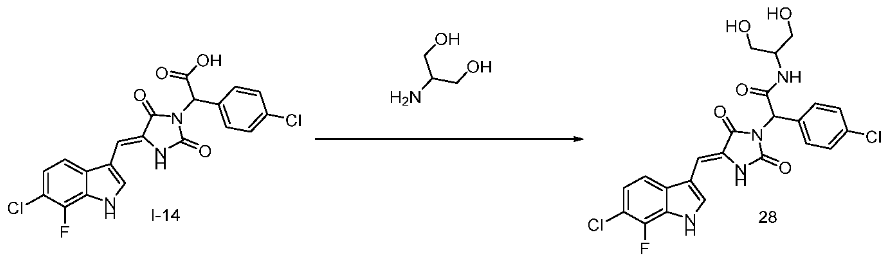

I-14

28

By using the method in Example **22,** the yellow solid compound **28** (54.1 mg, 29%) was prepared by using the intermediate **I-14** and 2-aminopropane-1,3-diol.

LC-MS m/z (ESI): 521.1 $[M+H]^+$. $^1$HNMR (300 MHz, DMSO-$d_6$) δ 12.52 (s, 1H), 10.57 (s, 1H), 8.24 (d, *J* = 2.8 Hz, 1H), 7.69 (dd, *J* = 17.6, 8.4 Hz, 2H), 7.42 (d, *J* = 1.2 Hz, 5H), 7.20 (dd, *J* = 8.6, 6.5 Hz, 1H), 6.81 (s, 1H), 5.77 (s, 1H), 4. 60-4. 30 (m, 1H), 3. 86-3. 75 (m, 1H), 3. 48-3. 33 (m, 5H).

**Example 29**

**Compound 29: (Z)-2-(4-((6-chloro-7-fluoro-1H-indol-3-yl)methylene)-2,5-dioxoimidazolidin-1-yl)-2-(4-cyanophenyl)-N-(1, 3-dihydroxypropan-2-yl)acetamide**

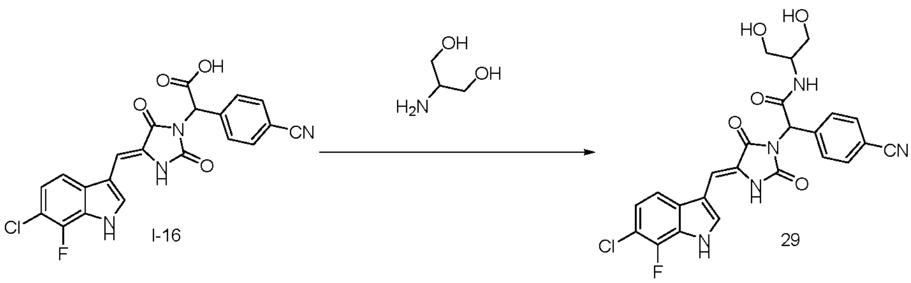

I-16

29

By using the method in Example **22,** the yellow solid compound **29** (34 mg, 21%) was prepared by using the intermediate **I-16** and 2-aminopropane-1, 3-diol.

LC-MS m/z (ESI): 510. 1 $[M-H]^-$. $^1$HNMR (400 MHz, DMSO-$d_6$) δ 12. 59-12. 37 (m, 1H), 10.62 (s, 1H), 8.25 (d, *J* = 2.8 Hz, 1H), 7.94 (d, *J* = 8.2 Hz, 1H), 7.82 (d, *J* = 8.1 Hz, 2H), 7.67 (d, *J* = 8.6 Hz, 1H), 7. 59 (d, *J* = 8.1 Hz, 2H), 7. 20 (dd, *J* = 8.5, 6. 4 Hz, 1H), 6.82 (s, 1H), 5. 87 (s, 1H), 3. 82 (q, *J* = 6. 5 Hz, 2H), 3. 53-3. 31 (m, 5H).

**Example 30**

**Compound 30: (Z)-2-(4-((6-chloro-7-methyl-1H-indol-3-yl)methylene)-2,5-dioxoimidazolidin-1-yl)-2-(3,4-difluorophenyl)-N-(1,3-dyhydroxl-2-(hydroxymethyl)-2-propyl) acetamide**

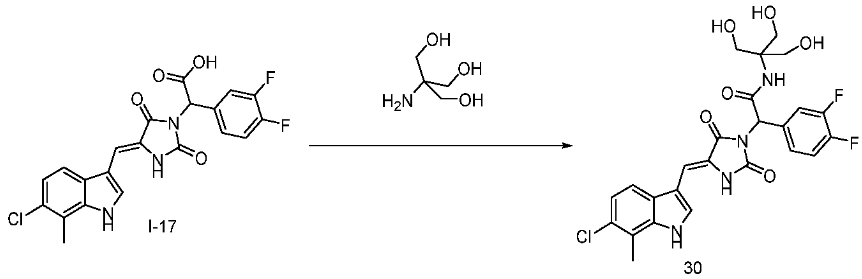

I-17

30

By using the method in Example **22,** the yellow solid compound **30** (4. 4 mg, 4.57%) was prepared by using the intermediate **I-17** and 2-amino-2-(hydroxymethyl)propane-1, 3-diol.

LC-MS m/z (ESI): 549. 1 $[M+H]^+$. $^1$HNMR (300 MHz, DMSO-$d_6$) δ 12.07 (s, 1H), 10.64 (s, 1H), 8.24 (s, 1H), 7.69-7.31 (m, 4H), 7.19-7.14 (m, 2H), 6.86 (s, 1H), 5.86 (s, 1H), 4. 54 (br, 2H), 3. 70-3. 40 (m, 7H).

**Example 31**

**Compound 31: (Z)-2-(4-((6-chloro-1H-indol-3-yl)methylene)-2,5-dioxoimidazolidin-1-yl)-2-(4-cyanophenyl)-N-(1, 3-dihydroxypropan-2-yl)acetamide**

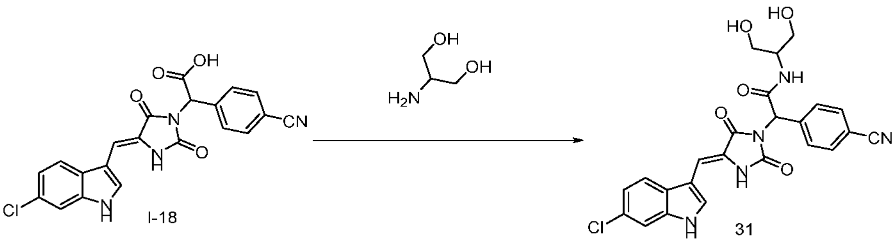

I-18

31

By using the method in Example **22,** the yellow solid compound **31** (6.4 mg, 4.28%) was prepared by using the intermediate **I-18** and 2-aminopropane-1, 3-diol.

LC-MS m/z (ESI): 494.1 $[M+H]^+$. $^1$HNMR (400 MHz, DMSO-$d_6$) δ 12.07 (d, *J* = 2.8 Hz, 1H), 10.76 (s, 1H), 8.24 (d, *J* = 2.5 Hz, 1H), 8.00 (s, 3H), 7.86 (dd, *J* = 21.8, 8.3 Hz, 3H), 7.64 (d, *J* = 8.0 Hz, 2H), 7.50 (d, *J* = 1.9 Hz, 1H), 7.15 (dd, *J* = 8.5, 1.9 Hz, 1H), 6.95 (s, 1H), 6.24 (s, 1H), 5.40 (s, 1H), 4.31 (q, *J* = 6.9, 6.0 Hz, 2H), 3. 62-3. 53 (m, 1H), 3. 51-3. 45 (m, 1H).

**Example 32**

**Compound 32: (Z)-2-(4-((6-chloro-7-methyl-1H-indol-3-yl)methylene)-2,5-dioxoimidazolidin-1-yl)-2-(4-cyanophenyl)-N-(1, 3-dihydroxypropan-2-yl)acetamide**

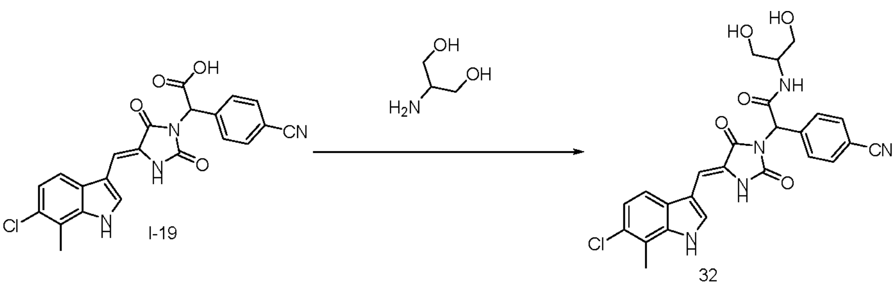

I-19

32

By using the method in Example **22,** the yellow solid compound **32** (58 mg, 21%) was prepared by using the intermediate **I-19** and 2-aminopropane-1,3-diol.

LC-MS m/z (ESI): 508.0 $[M+H]^+$. $^1$HNMR (400 MHz, DMSO-$d_6$) δ 12.01 (d, *J* = 3.0 Hz, 1H), 10.57 (s, 1H), 8.21 (d, *J* = 2.9 Hz, 1H), 7.94 (d, *J* = 8.2 Hz, 1H), 7.82 (d, *J* = 8.1 Hz, 2H), 7.64 (d, *J* = 8.5 Hz, 1H), 7.59 (d, *J* = 8.1 Hz, 2H), 7.14 (d, *J* = 8.5

Hz, 1H), 6.83 (s, 1H), 5.87 (s, 1H), 4. 18-3. 65 (m, 3H), 3. 53-3. 31 (m, 4H), 2.51 (s, 3H).

**Example 33**

**Compound 33: (Z)-4-(1-(4-((6-chloro-7-fluoro-1H-indol-3-yl)methylene)-2, 5-dioxoimidazolidin-1-yl)-2-oxy-ethyl)-2-fluorobenzonitrile**

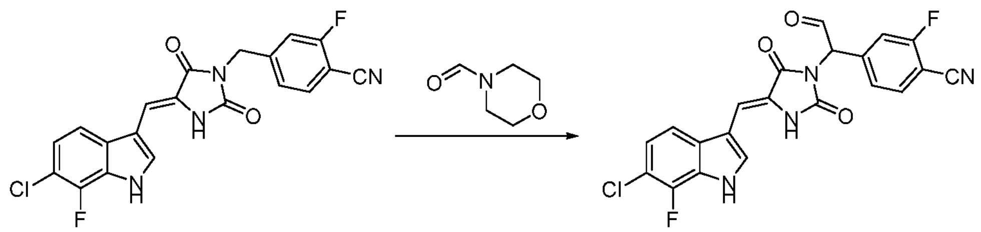

14 33

Under nitrogen protection at -78° C, Li MDS (4.8 mL, 4.8 mmol, 5 equiv, 1 N) was added into a solution of the compound **14** (400 mg, 0.958 mmol, 1 equiv, 98.9%) in tetrahydrofuran (24.5 mL), and the mixture was stirred at -78° C for 1 h. Then formyl morpholine (1.4 g, 11.552 mmol, 12.05 equiv, 95%) was added, and the mixture was stirred at -80° C for 4 h. A solution of HOAc (1 mL) in THF (3 mL) was added at -80° C to terminate the reaction, the mixture was heated to -20° C, and water (100 mL) was added in the reaction. The obtained mixture was extracted with EtOAc (3×50 mL), and combined organic layers were washed with saline (100 mL) and dried with anhydrous sodium sulfate. After the organic layers were filtered, a filtrate was concentrated at a reduced pressure. A residue was purified by reverse HPLC to obtain the yellow solid compound **33** (14.2 mg, 3.3%).

LC-MS m/z (ESI): 439.0 $[M-H]^-$. $^1H$ NMR (300 MHz, DMSO-$d_6$) δ 12.55 (s, 1H), 11.08 (s, 1H), 10.62 (s, 1H), 8. 30 (s, 1H), 7.93 (s, 1H), 7.87-7.62 (m, 2H), 7.58-7.48 (m, 1H), 7.44-7.16 (m, 2H), 6. 87 (s, 1H).

**Example 34**

**Compound 34: (Z)-4-((4-((6-chloro-7-fluoro-1H-indol-3-yl)methylene)-2, 5-dioxoimidazolidin-1-yl)-2, 3-difluoro-benzonitrile**

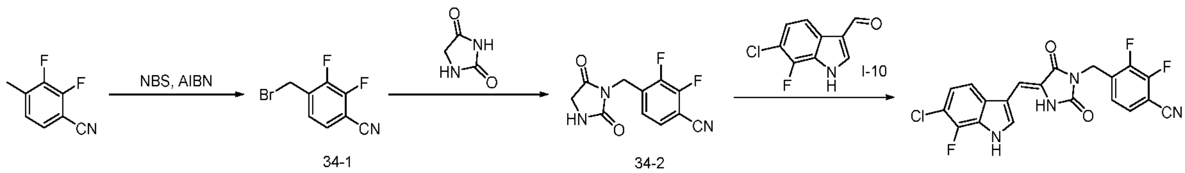


34-1 34-2 34

**34-1: 4-(bromomethyl)-2,3-difluorobenzonitrile**

At room temperature, AIBN (180 mg, 1.061 mmol, 1.0 equiv, 95%) was added in batches into a solution of 2,3-difluoro-4-methyl benzonitrile (2 g, 11.787 mmol, 1.0 equiv, 95%) and NBS (3 g, 16.738 mmol, 1.4 equiv, 95%) in $CHCl_3$ (95 mL), and the obtained mixture was stirred under nitrogen protection at 80° C for 8 h. The mixture was cooled to room temperature, and water was added to terminate the reaction. The obtained mixture was extracted with EtOAc (3×200 mL), and combined organic layers were washed with saline (3×200 mL) and dried with anhydrous sodium sulfate. After the organic layers were filtered, a filtrate was concentrated at a reduced pressure. A residue was purified by silica gel column chromatography and eluted with PE/EA (1:1) to obtain the yellow oily **34-1** (1.7 g, 67.5%).

LC-MS m/z (ESI): 299.8 $[M-H]^-$.

**34-2: 4-[(2, 5-dioxoimidazolidin-1-yl)methyl]-2, 3-difluorobenzonitrile**

At room temperature, hydantoin (1.0 g, 9.398 mmol, 1.3 equiv, 95%) was added into a solution of **34-1** (1.7 g,

7.241 mmol, 1.0 equiv, 99%) and potassium carbonate (2.0 g, 13.748 mmol, 1.9 equiv, 95%) in DMF (15 mL), and the obtained mixture was stirred under nitrogen protection at room temperature for 12 h. Water was added to terminate the reaction, the obtained mixture was extracted with EtOAc (3×100 mL), and combined organic layers were washed with saline (3×60 mL) and dried with anhydrous sodium sulfate. After the organic layers were filtered, a filtrate was concentrated at a reduced pressure to obtain the white solid **34-2** (1.2 g, 67%).

LC-MS $(M-H)^-$=250.0

**Compound 34: 4-{[(4Z)-4-[(6-chloro-7-fluoro-1H-indol-3-yl)methylene]-2, 5-dioxoimidazolidin-1-yl]methyl}-2,3-difluorobenzonitrile**

By using the method in Example 1, the yellow solid compound **34** (31.7 mg, 14.6%) was prepared by using **34-2** and **I-10.**

LC-MS $(M-H)^-$ = 428.8. $^1H$ NMR (400 MHz, DMSO-$d_6$) δ 12.56 (s, 1H), 10.64 (s, 1H), 8.26 (s, 1H), 7.79-7.62 (m, 2H), 7.41-7.31 (m, 1H), 7.27-7.16 (m, 1H), 6.86 (s, 1H), 4.84 (s, 2H).

**Example 35**

**Compound 35: (Z)-4-((4-((6-chloro-7-fluoro-1H-indol-3-yl)methylene)-2,5-dioxoimidazolidin-1-yl)-2,6-difluoro-benzonitrile**

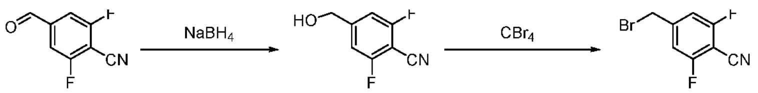


35-1 35-2

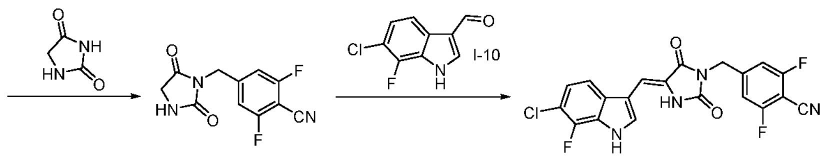


35-3 35

**35-1: 2,6-difluoro-4-(hydroxymethyl)benzonitrile**

At 0° C and under nitrogen protection, sodium borohydride (257 mg, 6.458 mmol, 1.00 equiv) was added in batches into a solution of 2,6-difluoro-4-formyl benzonitrile (11.40 g, 6.458 mmol, 1.00 equiv) in methanol (25 mL), and the obtained mixture was stirred at 0° C for 1 h. Water was added to terminate the reaction, the obtained mixture was extracted with EtOAc (3×100 mL), and combined organic layers were washed with saline (3×50 mL) and dried with anhydrous sodium sulfate. After the organic layers were filtered, a filtrate was concentrated at a reduced pressure. A residue was purified by silica gel column chromatography and eluted with $CH_2Cl_2$/EA (5:1) to obtain the yellow oily **35-1** (936 mg, 3.682 mmol, 84.7%).

LC-MS m/z (ESI):168.0 $[M-H]^-$

**35-2: 4-(bromomethyl)-2,6-difluorobenzonitrile**

At 0° C and under nitrogen protection, carbon tetrabromide (2.30 g, 6.917 mmol, 1.30 equiv) was added in batches into a solution of **35-1** (936 mg, 5.534 mmol, 1.00 equiv) and PPI (1.33 g, 6.032 mmol, 1.10 equiv) in THF (33.0 mL), and the obtained mixture was stirred at room temperature for 20 h. The mixture was filtered, a reaction solution was extracted with EtOAc (3×50 mL), and combined organic layers were washed with saline (3×30 mL) and dried with anhydrous sodium sulfate. After the organic layers were filtered, a filtrate was concentrated at a reduced pressure. A residue was purified by silica gel column chromatography and eluted with PE/EA (5:1) to obtain the yellow oily **35-2** (218 mg, 0.930 mmol, 16.8%).

LC-MS m/z (ESI): 247.9 $[M-H+18]^-$

**35-3: Synthesis of 4-[(2, 5-dioxoimidazolidin-1-yl)methyl]-2,6-difluorobenzonitrile**

By using the method for **34-2,** the white solid compound **35-3** (164 mg, 0.648 mmol, 75.9%) was prepared by using **35-2.**

LC-MS m/z (ESI):250.0 [M-H]$^-$

**Compound 35: 4-{[(4Z)-4-[(6-chloro-7-fluoro-1H-indol-3-yl)methylene]-2, 5-dioxoimidazolidin-1-yl]methyl}-2,6-difluorobenzonitrile**

By using the method in Example **1,** the yellow solid compound **35** (5.1 mg, 0.011 mmol, 2.95%) was prepared by using **35-3** and **I-10.**

LC-MS m/z (ESI): 428.8[M-H]$^-$. $^1$H NMR: (400 MHz, DMSO-$d_6$) δ 12.55 (s, 1H), 10.62 (s, 1H), 8.27 (s, 1H), 7.73-7.63 (m, 1H), 7. 55-7. 35 (m, 2H), 7.27-7.17 (m, 1H), 6.87 (s, 1H), 4.80 (s, 2H).

**Example 36**

**Compound 36: (Z)-4-((4-((6-chloro-7-fluoro-1H-indol-3-yl)methylene)-2, 5-dioxoimidazolidin-1-yl)-2,5-difluorobenzonitrile**

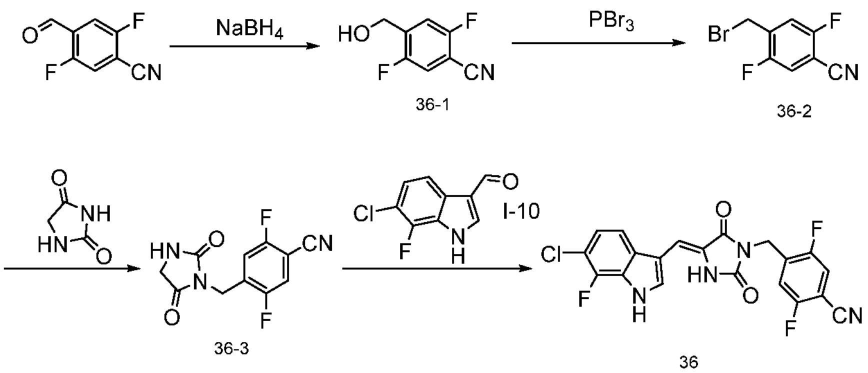

36-1
36-2
36-3
36

**36-1: Synthesis of 2,5-difluoro-4-(hydroxymethyl)benzonitrile**

By using the method for **35-1,** the white solid compound **36-1** (950 mg, 5.611 mmol, 96.6%) was prepared by using 2,5-difluoro-4-formyl benzonitrile.

LC-MS m/z (ESI):168.0 [M-H]$^-$

**36-2: 4-(bromomethyl)-2,5-difluorobenzonitrile**

Under nitrogen protection, phosphorus tribromide (1.55 g, 5.804 mmol, 1.10 equiv) was dropwise added into a solution of 2,5-difluoro-4-(hydroxymethyl)benzonitrile (920 mg, 5.276 mmol, 1.00 equiv) in DCM (5 mL) at 0° C, and the obtained mixture was stirred at room temperature for 20 h. The reaction solution was diluted with DCM (50 mL) and extracted with sodium hydrogen carbonate (3×70 mL), and organic phases were dried with anhydrous sodium sulfate. After the organic phases were filtered, a filtrate was concentrated at a reduced pressure to obtain the white solid **36-2** (545 mg, 1.966 mmol, 40.3%).

LC-MS m/z (ESI): 232.00 [M+H]$^+$

**36-3: 4-[(2,5-dioxoimidazol-1-yl)methyl]-2,5-difluorobenzonitrile**

By using the method for **34-2,** the yellow solid compound **36-3** (183 mg, 0.626 mmol, 29.1%) was prepared by using **36-2.**

LC-MS m/z (ESI) :250.0 [M-H]$^-$

**Compound 36: 4-{[(4Z)-4-[(6-chloro-7-fluoro-1H-indol-3-yl)methylene]-2, 5-dioxoimidazolidin-1-yl]methyl}-2,5-difluorobenzonitrile**

By using the method in Example **1,** the yellow solid compound **36** (18.6 mg, 0.041 mmol, 13.6%) was prepared by using the **36-3** and corresponding aldehyde.
LC-MS m/z (ESI): 428.8 [M-H]$^-$. $^1$H NMR: (400 MHz, $CDCl_3$) δ12.55 (s, 1H), 10.61 (s, 1H), 8.27 (s, 1H), 8.07-7.97(m, 1H), 7.78-7.68 (m, 1H), 7.65-7.45 (m, 1H), 7. 28-7.18 (m, 1H), 6.86 (s, 1H), 4.79 (s, 2H).

**Example 37**

**Compound 37: (Z)-4-(1-(4-((6-chloro-7-fluoro-1H-indol-3-yl)methylene)-2, 5-dioxoimidazolidin-1-yl)-2-hydroxyethyl)-2-fluorobenzonitrile**

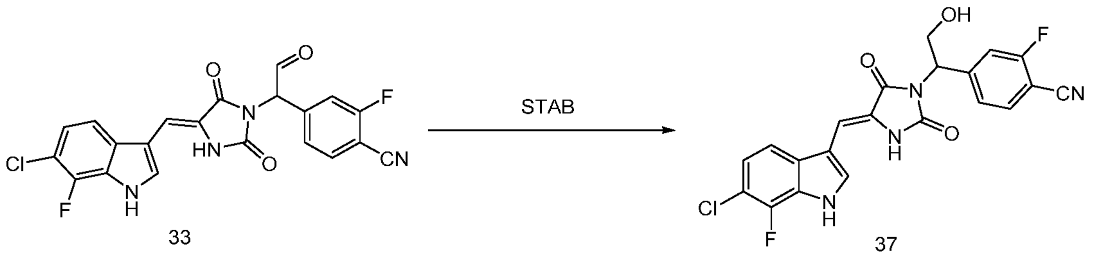


33 37

STAB (38 mg, 0.174 mmol, 2.63 equiv) was added in batches into a stirred solution of 4-{1-[(4Z)-4-[(6-chloro-7-fluoro-1H-indol-3-yl)methylene]-2, 5-dioxoimidazolidin -1-yl]-2-oxyethyl}-2-fluorobenzonitrile (30 mg, 0.066 mmol, 1 equiv) and AcOH (10 mg, 0.153 mmol, 2.32 equiv) in DCE (1 mL) at 0° C, and the obtained mixture was stirred at 40° C for 1 h. The mixture was cooled to room temperature and concentrated in vacuum. A crude product was purified by preparative HPLC to obtain the yellow solid compound **37** (6.9 mg, 0.012 mmol, 18.8%).
LC-MS m/z (ESI): 440.9 [M-H]$^-$. $^1$H NMR (400 MHz, DMSO-$d_6$) δ 12. 54 (s, 1H), 10.61 (s, 1H), 8. 31-8. 21 (m, 1H), 7. 98-7. 88 (m, 1H), 7. 73-7. 63 (m, 1H), 7. 65-7. 55 (m, 1H), 7. 50-7. 40 (m, 1H), 7. 27-7. 17 (m, 1H), 6.84 (s, 1H), 5. 35-5. 25 (m, 2H), 4. 33-4. 23 (m, 1H), 4. 09-3. 99 (m, 1H).

**Example 38**

**Compound 38: (Z)-4-(1-(4-((6-chloro-1H-indol-3-yl)methylene)-2,5-dioxoimidazolidin -1-yl)-2-hydroxyethyl)benzonitrile**

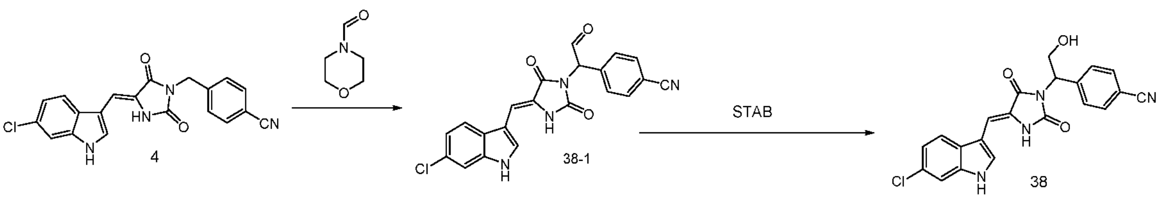


4 38-1 38

**38-1: 4-{1-[(4Z)-4-[(6-chloro-1H-indol-3-yl)methylene]-2,5-dioxoimidazolidin-1-yl]-2-hydroxyethyl}benzonitrile**

By using the method in Example **33,** the yellow solid **38-1** (220 mg, 0.327 mmol, 9.7%) was prepared by using the compound 4.
LC-MS m/z (ESI): 403.1 [M-H]$^-$

**Compound 38: (Z)-4-(1-(4-((6-chloro-1H-indol-3-yl)methylene)-2,5-dioxoimidazolidin-1-yl]-2-hydroxyethyl}benzonitrile**

By using the method in Example **37,** the yellow solid **38** (13.6 mg, 0.032 mmol, 40%) was prepared by using **38-1.**
LC-MS m/z (ESI): 407.1 [M+H]$^+$. $^1$H NMR (300 MHz, DMSO-$d_6$) δ 11.97 (s, 1H), 10.51 (brs, 1H), 8.19 (s, 1H), 7.88-7.68 (m, 3H), 7.66-7.56 (m, 2H), 7.55-7.45 (m, 1H), 7.21-7.09 (m, 1H), 6.85 (s, 1H), 5.33-5.17 (m, 2H), 4.37-4.27 (m,

1H), 4.07-3.97 (m, 1H).

**Example 39**

**Compound 39: (Z)-5-((6-chloro-7-methyl-1H-indol-3-yl)methylene)-3-(1-(3, 4-difluorophenyl)-2-hydroxyethyl) imidazolidine-2,4-dione**

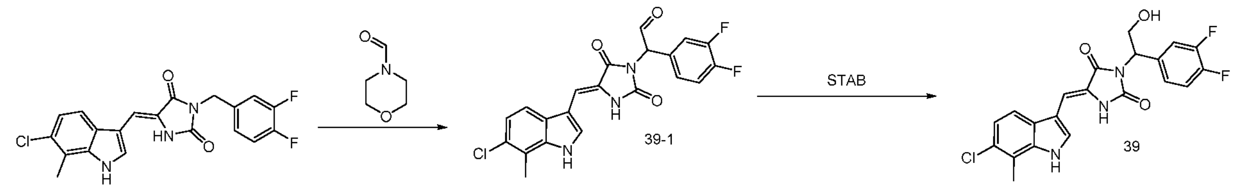


39-1

39

**39-1: 2-[(4Z)-4-[(6-chloro-7-methyl-1H-indol-3-yl)methylene]-2,5-dioxoimidazolidin-1-yl]-2-(3,4-difluorophenyl) acetaldehyde**

By using the method in Example **33,** the yellow solid **39-1** (80 mg, 0.219 mmol, 12.7%) was prepared by using (Z)-5-((6-chloro-7-methyl-1H-indol-3-yl)methylene)-3-(3,4-difluorobenzyl)imidazolidine-2,4-dione.

LC-MS m/z (ESI): 430.0 [M+H]$^+$

**Compound 39: (Z)-5-((6-chloro-7-methyl-1H-indol-3-yl)methylene)-3-(1-**(3, **4-difluorophenyl)-2-hydroxyethyl) imidazolidine-2,4-dione**

By using the method in Example **37,** the yellow solid **39** (8.5 mg, 0.019 mmol, 9.5%) was prepared by using **39-1.**

LC-MS m/z (ESI): 430.0 [M-H]$^-$. $^1$H NMR (300 MHz, DMSO-$d_6$) δ 11.99 (s, 1H), 10.51 (s, 1H), 8.21 (s, 1H), 7.69-7.59 (m, 1H), 7.58-7.35 (m, 2H), 7.33-7.10 (m, 2H), 6.83 (s, 1H), 5.26-5.15 (m, 2H), 4. 37-4. 27 (m, 1H), 4. 00-3. 90 (m, 1H). 2.45 (s, 3H).

**Example 40**

**Compound 40: (Z)-2-(4-((6-chloro-1H-indol-3-yl)methylene)-2,5-dioxoimidazolidin-1-yl)-2-(4-cyanophenyl)ethyl dihydrophosphate**

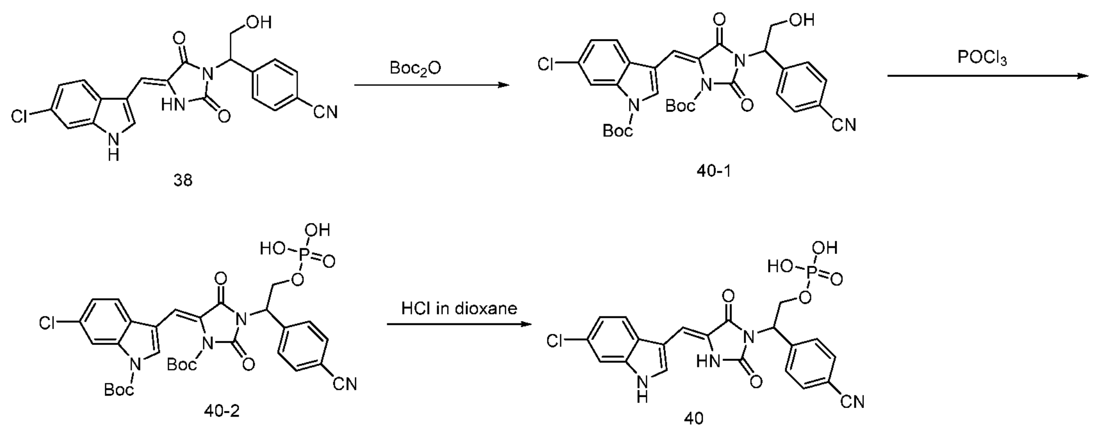


38

40-1

40-2

40

**40-1: tert-butyl 3-{[(4Z)-3-(tert-butoxycarbonyl)-1-[1-(4-cyanophenyl)-2-hydroxyethyl]-2,5-dioxoimidazolidin-4-ylene]methyl}-6-chloroindole-1-carboxylate**

DIEA (104 mg, 0.763 mmol, 2.12 equiv) was added into a stirred solution of 4-{1-[(4Z)-4-[(6-chloro-1H-indol-3-yl) methylene]-2,5-dioxoimidazolidin-1-yl]-2-hydroxyethyl}benzonitrile (155 mg, 0.360 mmol, 1 equiv), DMAP (9 mg, 0.068

mmol, 0.19 equiv), and $Boc_2O$ (243 mg, 1.058 mmol, 2.94 equiv) in DMF (3 mL), and the obtained mixture was stirred in a nitrogen atmosphere at 40° C for 2 h. The mixture was cooled to room temperature and concentrated in vacuum. A residue was purified by fast reversed-phase chromatography to obtain the yellow solid **40-1** (20 mg, 0.033 mmol, 9.13%).

LC-MS m/z (ESI): 505.1 $[M-H-100]^-$

**40-2: 2-[(4Z)-3-(tert-butoxycarbonyl)-4-{[1-(tert-butoxycarbonyl)-6-chloroindol-3-yl]methylene}-2,5-dioxoimidazolidin-1-yl]-2-(4-cyanophenyl)ethoxyl phosphonic acid**

Phosphorus oxychloride (125 mg, 0.772 mmol, 0.16 mL, 18.83 equiv) was added into a stirred solution of tert-butyl 3-{[(4Z)-3-(tert-butoxycarbonyl)-1-[1-(4-cyanophenyl)-2-hydroxyethyl]-2, 5-dioxoimidazolidin-4-ylene]methyl}-6-chloroindol-1-carboxylate (25 mg, 0.041 mmol, 1 equiv) and TEA (150 mg, 1.403 mmol, 0.20 M1, 34.23 equiv) in THF (1 mL) at 0° C, and the obtained mixture was stirred at room temperature for 18 h. Then, water (0.5 mL) was added into a reaction solution at 0° C, and the obtained mixture was stirred at room temperature for 2 h. The obtained mixture was concentrated in vacuum to obtain the yellow solid **40-2** (40 mg, crude product). The crude product was directly used for a next step without further purification.

LC-MS m/z (ESI): 685.1 $[M-H]^-$

**Compound 40: 2-[(4Z)-4-[(6-chloro-1H-indol-3-yl)methylene]-2,5-dioxoimidazolidin-1-yl]-2-(4-cyanophenyl) ethoxyl phosphonic acid**

A 1,4-dioxane (1 mL, 4.000 mmol, 4 N) in HCl was added into 2-[(4Z)-3-(tert-butoxycarbonyl)-4-{[1-(tert-butoxycarbonyl)-6-chloroindol-3-yl]methylene}-2, 5-dioxoimidazol-1-yl]-2-(4-cyanophenyl)ethoxyl phosphonic acid (40 mg, crude product), and the obtained mixture was stirred at room temperature for 12 h. The obtained mixture was concentrated in vacuum. The crude product was purified by reversed-phase HPLC to obtain the white solid compound **40** (2.2 mg, 0.004 mmol, 29.11%).

LC-MS m/z (ESI): 484.9 $[M-H]^-$.

$^1H$ NMR (300 MHz, DMSO-$d_6$) δ 8.20 (s, 1H), 7.85-7.75 (m, 3H), 7.71-7.57 (m, 2H), 7.54-7.44 (m, 1H), 7.13 (d, *J* = 8.5 Hz, 1H), 6.82 (s, 1H), 5.47-5.37 (m, 1H), 4.46-4.36 (m, 2H).

**Example 41**

**Compound 41: (Z)-2-(4-((6-chloro-7-fluoro-1H-indol-3-yl)methylene)-2,5-dioxoimidazolidin-1-yl)-2-(4-cyano-3-fluorophenyl)-N-(2-hydroxyethyl)acetamide**

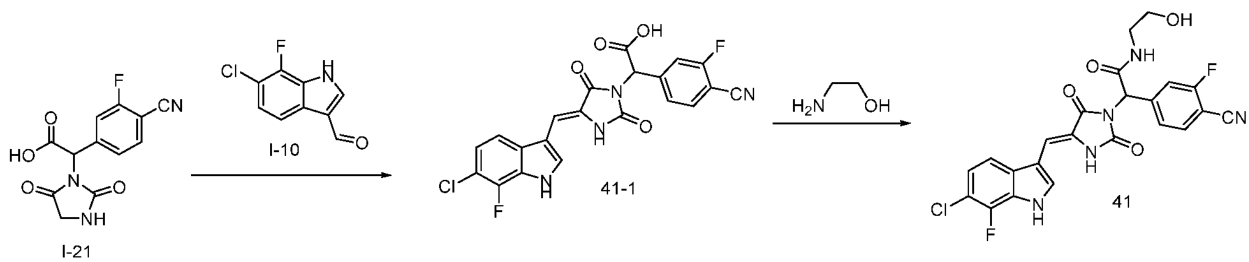

**41-1: (Z)-2-(4-((6-chloro-7-fluoro-1H-indol-3-yl)methylene)-2,5-dioxoimidazolidin-1-yl)-2-(4-cyano-3-fluorophenyl)acetic acid**

Pyridine (341 mg, 4.100 mmol, 4.0 equiv, 95%) was added in batches into a solution of methyl 2-(4-cyano-3-fluorophenyl)-2-(2,5-dioxoimidazolidin-1-yl)acetate (300 mg, 1.020 mmol, 1.0 equiv, 99%) and 6-chloro-7-fluoro-1H-indole-3-formaldehyde (409 mg, 2.050 mmol, 2.0 equiv, 99%) in ethanol (5 mL) at room temperature, and the obtained mixture was stirred under nitrogen protection at 60° C for 4 h. A residue was purified by fast reversed-phase chromatography to obtain the yellow solid compound **41-1** (120 mg, 23%).

LC-MS $(M-H)^-$ =455. 0.

**Compound 41: (Z)-2-(4-((6-chloro-7-fluoro-1H-indol-3-yl)methylene)-2,5-dioxoimidazol-1-yl)-2-(4-cyano-3-fluorophenyl)-N-(2-hydroxyethyl) acetamide**

Cholamine (27 mg, 0.420 mmol, 9.80 equiv) was dropwise added into a stirred solution of [(4Z)-4-[(6-chloro-7-fluoro-1H-indol-3-yl)methylene]-2,5-dioxoimidazolidin-1-yl] (4-cyano-3-fluorophenyl)acetic acid (20 mg, 0.043 mmol, 1 equiv), HATU (30 mg, 0.075 mmol, 1. 75 equiv), and DIEA (27 mg, 0.198 mmol, 4.63 equiv) in DMF (0.5 mL), and the obtained mixture was stirred under nitrogen protection at 40° C for 12 h. The mixture was cooled to room temperature and purified by Prep-HPLC to obtain the yellow solid compound **41** (1.6 mg, 0.003 mmol, 7.55%).

LC-MS m/z (ESI): 500.1 $[M+H]^+$.

$^1H$ NMR (400 MHz, DMSO-$d_6$) δ 12.56 (s, 1H), 10.64 (s, 1H), 8.29-8.18 (m, 2H), 7.97-7.89 (m, 1H), 7.69 (d, *J* = 8.6 Hz, 1H), 7.59 (d, *J* = 10.6 Hz, 1H), 7.47 (d, *J* = 8.6 Hz, 1H), 7.28-7.17 (m, 1H), 6.84 (s, 1H), 5.89 (s, 1H), 4.67-4.60 (m, 1H), 3.46-3.39 (m, 2H), 3.26-3.19 (m, 1H), 3.18-3.09 (m, 1H).

**Example 42**

**Compound 42: (Z)-2-(4-((6-chloro-1H-indol-3-yl)methylene)-2,5-dioxoimidazolidin-1-yl)-2-(3,4-difluorophenyl) ethyl dihydrophosphate**

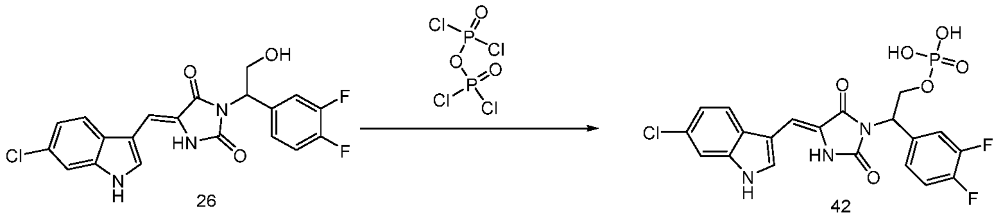

26 42

Dichlorophosphonic anhydride (35 mg, 0.14 mmol, 3.91 eq) was dropwise added into a stirred solution of (5Z)-5-[(6-chloro-1H-indol-3-yl)methylene]-3-[1-(3, 4-difluorophenyl)-2-hydroxyethyl]imidazolidine-2, 4- dione (15 mg, 0.036 mmol, 1 eq) in ACN (1 mL) at 0° C, and the obtained mixture was then stirred at 0° C for 3 h. The mixture was alkalized with sodium hydrogen carbonate (1 M) at 0° C till the pH=8-9, and the mixture was stirred at 0° C for 30 min. Then, the mixture was acidized with HCl (12 M) till pH=1, and the mixture was stirred at 0° C for 1.5 h. The obtained mixture was extracted with EtOAc (3×25 mL), and combined organic layers were washed with saline (1x25 mL) and dried with anhydrous sodium sulfate. After the organic layers were filtered, a filtrate was concentrated at a reduced pressure. The product was purified by Prep-HPLC to obtain the yellow solid compound **42** (4.4 mg, 23.0%).

LC-MS m/z (ESI): 496.0 $[M-H]^-$. $^1H$ NMR (300 MHz, DMSO-$d_6$) δ 12.09 (s, 1H), 8.20 (s,1H), 7.79 (d, *J* = 8.5 Hz, 1H), 7.60-7.20 (m, 4H), 7.19-7.05 (m, 1H), 6.84 (s, 1H), 5. 42-5. 28 (m, 1H), 4. 60-4. 30 (m, 2H).

**Example 43**

**Compound 43: (Z)-2-(4-((6-chloro-7-methyl-1H-indol-3-yl)methylene)-2,5-dioxoimidazolidin-1-yl)-2-(3,4-difluorophenyl)ethyl dihydrophosphate**

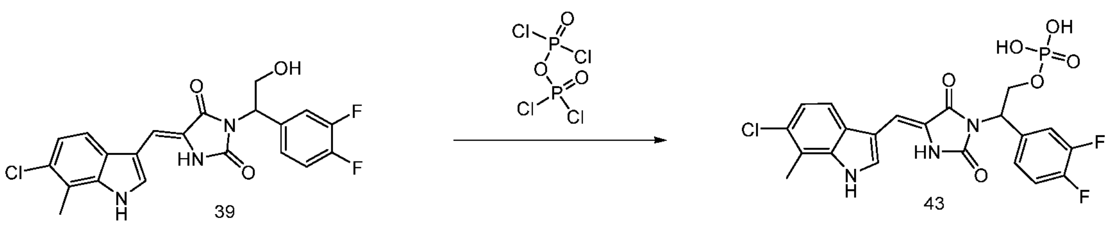

39 43

By using the method in Example **42,** the yellow solid compound **43** (10.9 mg, 17.4%) was prepared by using the compound **39.**

LC-MS m/z (ESI) : 510. 1 $[M-H]^-$. $^1H$ NMR (300 MHz, DMSO-$d_6$) δ 12. 09 (s, 1H), 8.21 (s, 1H), 7.62 (d, J = 8.5 Hz, 1H), 7.57- 7.32 (m, 2H), 7. 30-7. 21 (m, 1H), 7. 14 (d, J = 8.5 Hz, 1H), 6.82 (s, 1H), 5.40-5.29 (m, 1H), 4.56-4.25 (m, 2H), 2.54

(s, 3H).

**Example 44**

**Compound 44: (Z)-2-(2-(4-((6-chloro-7-methyl-1H-indol-3-yl)methylene)-2,5-dioxoimidazolidin-1-yl)-2-(3,4-difluorophenyl)acetamido)dihydroethyl phosphate**

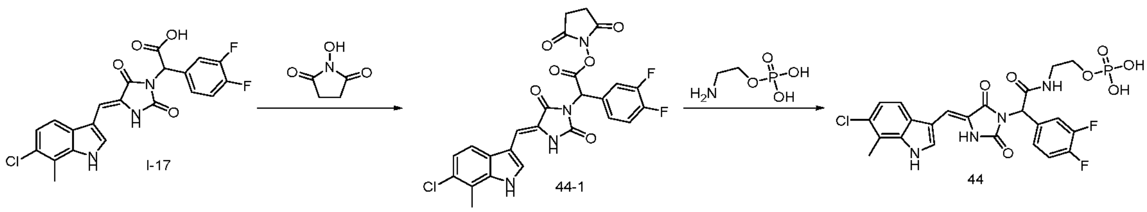

**44-1: 2,5-dioxopyrrolidin-1-yl(Z)-2-(4-((6-chloro-7-methyl-1H-indol-3-yl)methylene)-2,5-dioxoimidazolidin-1-yl)-2-(3,4-difluorophenyl)acetate**

Under nitrogen protection at 0° C, DCC (30 mg, 0.138 mmol) was added into a stirred solution of [(4Z)-4-[(6-chloro-7-methyl-1H-indol-3-yl)methylene]-2,5-dioxoimidazolidin 1-yl] (3, 4-difluorophenyl)acetic acid (60 mg, 0.128 mmol, 1 equiv) and N-hydroxysuccinimide (16 mg, 0.132 mmol, 1.03 equiv, 95%) in DCM (2 mL), and the obtained mixture was stirred at room temperature for 12 h. The reaction was terminated with ice water. The obtained mixture was extracted with DCM (3×10 mL). Combined organic layers were washed with saline (3×5 mL) and dried with anhydrous sodium sulfate. After the organic layers were filtered, a filtrate was concentrated at a reduced pressure to obtain the yellow solid **44-1** (100 mg, 99.12%). The crude product was directly used for a next step without further purification.
LC-MS m/z (ESI): 541.0 $[M-H]^-$ .

**Compound 44: (Z)-2-(2-(4-((6-chloro-7-methyl-1H-indol-3-yl)methylene)-2,5-dioxoimidazolidin-1-yl)-2-(3,4-difluorophenyl)acetamido)dihydroethyl phosphate**

Under nitrogen protection at 0° C, TEA (33 mg, 0.308 mmol, 2.37 equiv) was added into a stirred solution of 2,5-pyrrolidin-1-yl-2-[(4Z)-4-[(6-chloro-1H-indol-3-yl)methylene]-2, 5-dioxoimidazolidin-1-yl]-2-(3,4-difluorophenyl)acetate (100 mg, 0.130 mmol, 1 equiv) and 0-phosphorylethanolamine (33 mg, 0.221 mmol, 1.70 equiv) in DMF (3 mL) and water (2 mL), and the obtained mixture was stirred at room temperature for 16 h. The obtained mixture was concentrated in vacuum. The crude product was purified by Prep-HPLC to obtain the yellow solid compound **44** (17.3 mg, 19.85%).
LC-MS m/z (ESI): 569.1 $[M+H]^+$. $^1H$ NMR (300 MHz, DMSO-$d_6$) δ 12.02 (s, 1H), 8.52-8.46 (m, 1H), 8.21 (s, 1H), 7.63 (d, *J* = 8.5 Hz, 1H), 7.55-7.21 (m, 3H), 7.14 (d, *J* = 8.5 Hz, 1H), 6.82 (s, 1H), 5.75 (s, 1H), 3.75-3.69 (m, 2H), 3.27-3.08 (m, 2H), 2.51 (s, 3H).

**Example 45**

**Compound 45: (Z)-5-((6-chloro-1H-indol-3-yl)methylene)-3-(1-(4-chlorophenyl)-2-hydroxyethyl)imidazolidine-2,4-dione**

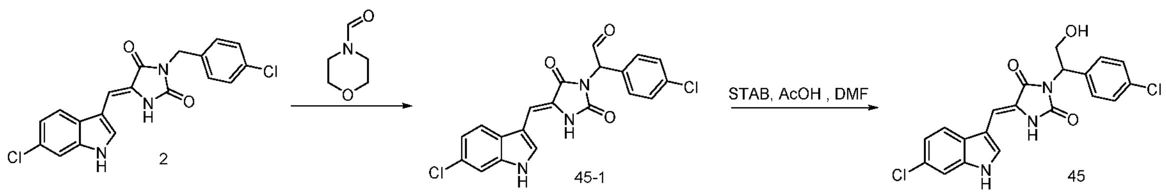


**45-1: (Z)-2-(4-((6-chloro-1H-indol-3-yl)methylene)-2,5-dioxoimidazolidin-1-yl)-2-(4-chlorophenyl)acetaldehyde**

By using the method for the compound **33,** the yellow solid compound **45-1** (205 mg, 38.2%) was prepared by

using the compound **2.**
LC-MS m/z (ESI): 411.9 [M-H]-.

**Compound 45: (Z)-5-((6-chloro-1H-indol-3-yl)methylene)-3-(1-(4-chlorophenyl)-2-hydroxyethyl)imidazolidine-2,4-dione**

By using the method for the compound **37,** the yellow solid compound **45** (8.9 mg, 40.3%) was prepared by using the compound **45-1.**
LC-MS m/z (ESI): 413.9 [M-H]- . $^1$H NMR (300 MHz, DMSO-$d_6$) δ 11. 96 (s, 1H), 10.47 (s, 1H), 8.18 (s, 1H), 7.81 (d, *J* = 8.5 Hz, 1H), 7.54-7.40 (m, 5H), 7.13 (d, *J* = 8. 5 Hz, 1H), 6. 83 (s, 1H), 5. 24-5. 13 (m, 2H), 4. 42-4. 28 (m, 1H), 4.01-3.87 (m, 1H).

**Example 46**

**Compound 46: (Z)-2-(4-((6-chloro-1H-indol-3-yl)methylene)-2,5-dioxoimidazolidin-1-yl)-2-(4-chlorophenyl)ethyl dihydrophosphate**

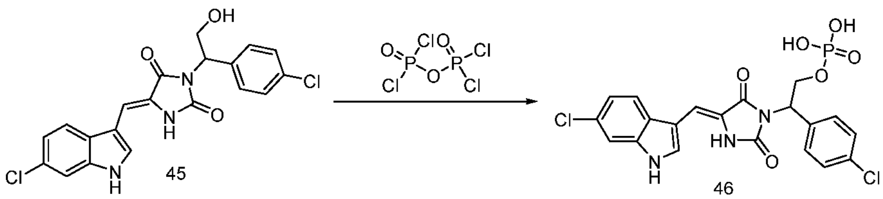

By using the method for the compound **42,** the yellow solid compound **46** (13.3 mg, 31.6%) was prepared by using the compound **45.**
LC-MS m/z (ESI): 496.0 [M+H]+. $^1$H NMR (400 MHz, DMSO-$d_6$) δ 12.13 (s, 1H), 8.21 (s, 1H), 7.80 (d, *J* = 8.5 Hz, 1H), 7.51-7.37 (m, 5H), 7.13 (d, *J* = 8.5 Hz, 1H), 6.83 (s, 1H), 5.40-5.30 (m, 1H), 4.56-4.47 (m, 1H), 4.39-4.27 (m, 1H).

**Example 47**

**Compound 47: (Z)-2-(4-((6-chloro-7-fluoro-1H-indol-3-yl)methylene)-2,5-dioxoimidazolidin-1-yl)-2-(4-cyano-3-fluorophenyl)ethyl dihydrophosphate**

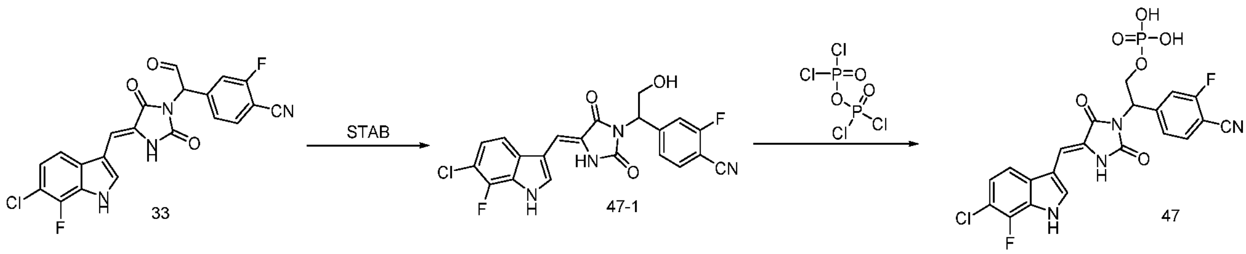


**47-1: (Z)-4-(1-(4-((6-chloro-7-fluoro-1H-indol-3-yl)methylene)-2,5-dioxoimidazolidin-1-yl)-2-hydroxyethyl)-2-fluorobenzonitrile**

By using the method for the compound **37,** the yellow solid compound **47-1** (19 mg, 39.4%) was prepared by using the compound **33.**
LC-MS m/z (ESI): 441.0[M-H]-

**Compound 47: (Z)-2-(4-((6-chloro-7-fluoro-1H-indol-3-yl)methylene)-2,5-dioxoimidazolidin-1-yl)-2-(4-cyano-3-fluorophenyl)ethyl dihydrophosphate**

By using the method for the compound **42,** the yellow solid compound **44** (2.8 mg, 17.8%) was prepared by using the compound **47-1.**

LC-MS m/z (ESI): 521.0 $[M-H]^-$ .$^1$HNMR (400 MHz, DMSO-$d_6$) δ 12.57 (s, 1H), 10.67 (s, 1H), 8.26 (s, 1H), 7.98-7.90 (m, 1H), 7. 69-7. 55 (m, 2H), 7.53-7.40 (m, 1H), 7. 25-7. 17 (m, 1H), 6.84 (s, 1H), 5. 57-5. 45 (m, 1H), 4. 65-4. 46 (m, 2H), 3. 40-3. 30 (s, 2H).

## Example 48

**Compound 48: (Z)-2-(4-((6-chloro-7-methyl-1H-indol-3-yl)methylene)-2,5-dioxoimidazolidin-1-yl)-2-(4-chlorophenyl)-N-(2-hydroxyethyl)acetamide**

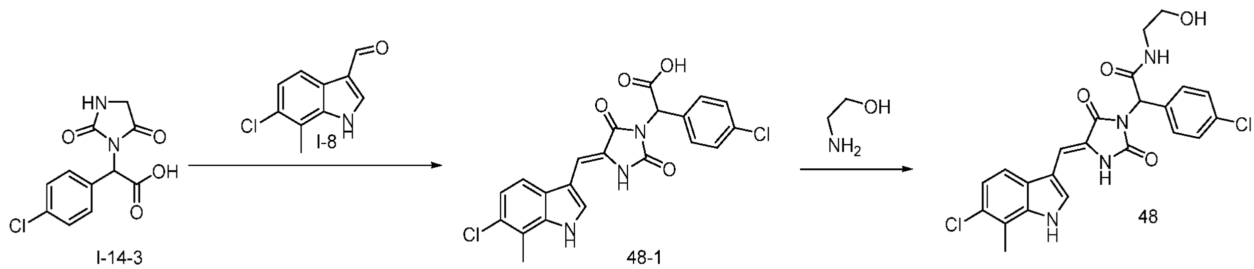

**48-1: (Z)-2-(4-((6-chloro-7-methyl-1H-indol-3-yl)methylene)-2,5-dioxoimidazolidin-1-yl)-2-(4-chlorophenyl) acetic acid**

By using the method for the **I-19,** the yellow solid compound **48-1** (16 g, 52%) was prepared by using **I-14-3** and **I-8.**

LC-MS m/z (ESI): 442.1. $0[M-H]^-$.

**Compound 48: (Z)-2-(4-((6-chloro-7-methyl-1H-indol-3-yl)methylene)-2,5-dioxoimidazolidin-1-yl)-2-(4-chlorophenyl)-N-(2-hydroxyethyl)acetamide**

By using the method for the compound **41,** the yellow solid **48** (10 g, 57%) was prepared by using **48-1.**

LC-MS m/z (ESI): 485. 1 $[M-H]^-$. $^1$H NMR (400 MHz, DMSO-*d6*) δ 12.00 (s, 1H), 10.52 (s, 1H), 8.20 (s, 1H), 8.07-8.02 (m, 1H), 7.64-7.62 (m, 1H), 7.48-7.40 (m, 4H), 7.20-7.13 (m, 1H), 6.82 (s, 1H), 5.76-5.73 (m, 1H), 4.63-4. 55 (m, 1H), 3.44-3.30 (m, 3H), 3.25-3.12 (m, 3H).

## Example 49

**Compound 49: (R,Z)-4-(1-(4-((6-chloro-1H-indol-3-yl)methylene)-2,5-dioxoimidazolidin-1-yl)-2-hydroxyethyl) benzonitrile**

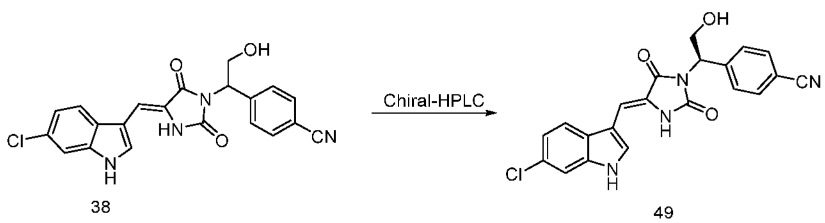


The compound **38** was separated by chiral SFC according to the following condition to obtain the compound **49:** column: CHIRALPAK ID-3 4.6×50 mm 3 um; mobile phase: Hex (0.1% DEA): EtOH=50:50, 20 min; detector, UV 254 nm.

LC-MS m/z (ESI): 404.8 [M-H]-. $^1$ H NMR (400 MHz, DMSO-$d_6$) δ 11.98 (s, 1H), 10.53 (s, 1H), 8.20 (s, 1H), 7.88-7.79 (m, 3H), 7.64-7.58 (m, 2H), 7.52-7.47 (m, 1H), 7.20-7.11 (m, 1H), 6.85 (s, 1H), 5.32-5.23 (m, 2H), 4.38-4.28 (m, 1H), 4.07-3.97 (m, 1H).

**Example 50**

**Compound 50: (S,Z)-4-(1-(4-((6-chloro-1H-indol-3-yl)methylene)-2,5-dioxoimidazolidin-1-yl)-2-hydroxyethyl) benzonitrile**

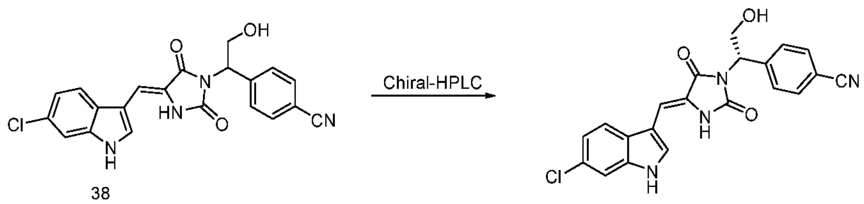


38 50

The compound **38** was separated by chiral SFC according to the following condition to obtain the compound **50:** column: CHIRALPAK ID-3 4.6×50 mm 3 um; mobile phase: Hex (0.1% DEA): EtOH=50:50, 20 min; detector, UV 254 nm.
LC-MS m/z (ESI): 404.8 [M-H]-. $^1$ H NMR (400 MHz, DMSO-$d_6$) δ 11.99 (s, 1H), 10. 53 (s, 1H), 8. 20 (s, 1H), 7. 89-7. 79 (m, 3H), 7. 63-7. 59 (m, 2H), 7. 50-7. 46 (m, 1H), 7. 22-7. 11 (m, 1H), 6. 86 (s, 1H), 5. 32-5. 24 (m, 2H), 4. 39-4. 28 (m, 1H), 4. 07-3. 98 (m, 1H) .

**Example** 51

**Compound 51: (R,Z)-2-(4-((6-chloro-1H-indol-3-yl)methylene)-2,5-dioxoimidazolidin-1-yl)-2-(4-cyanophenyl) ethyl dihydrophosphate**

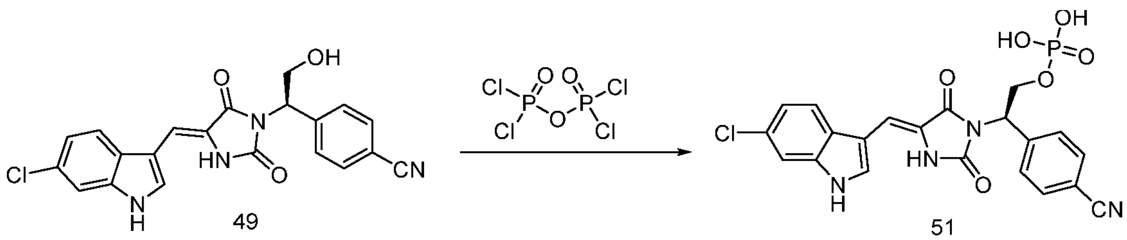

49 51

By using the method for the compound **42,** the yellow solid compound **51** (17.3 mg, 53.0%) was prepared by using the compound **49.**
LC-MS m/z (ESI): 484.9 [M-H]-. $^1$H NMR (300 MHz, DMSO-$d_6$) δ 12.20 (s, 1H), 8.21 (s, 1H), 7. 85-7. 75 (m, 3H), 7. 72-7. 57 (m, 2H), 7.54-7.46 (m, 1H), 7.17-7.08 (m, 1H), 6. 84 (s, 1H), 5.49-5.38 (m, 1H), 4. 59-4. 33 (m, 2H).

**Example 52**

**Compound 52: (S,Z)-2-(4-((6-chloro-1H-indol-3-yl)methylene)-2,5-dioxoimidazolidin-1-yl)-2-(4-cyanophenyl) ethyl dihydrophosphate**

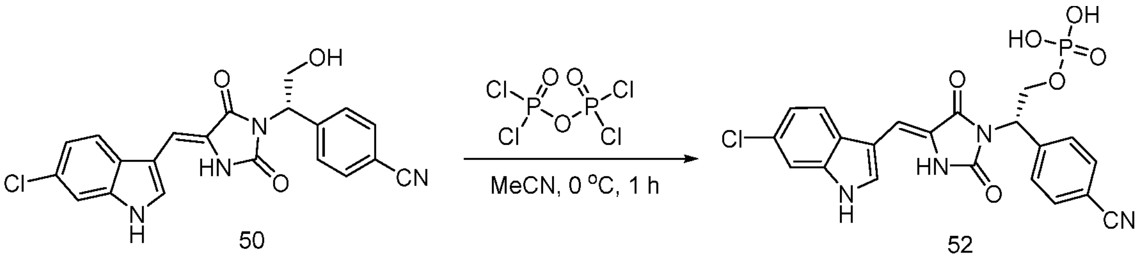


50 52

By using the method for the compound **42,** the yellow solid compound **52** (17.9 mg, 59.1%) was prepared by using the compound 50.

LC-MS m/z (ESI): 487.0 $[M+H]^+$. $^1H$ NMR (300 MHz, DMSO-$d_6$) δ 12. 19 (s, 1H), 8.21 (s, 1H), 7.85-7.75 (m, 3H), 7.72-7.57 (m, 2H), 7.54-7.46 (m, 1H), 7.17-7.07 (m, 1H), 6. 84 (s, 1H), 5. 49-5. 38 (m, 1H), 4. 52-4. 34 (m, 2H).

**Example 53**

**Compound 53: (S, Z)-5-((6-chloro-1H-indol-3-yl)methylene)-3-(1-(3, 4-difluorophenyl)-2-hydroxyethyl)imidazolidine-2,4-dione**

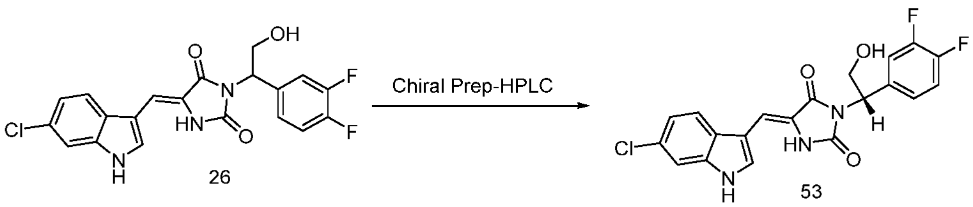


26 53

The compound **26** was separated by chiral HPLC according to the following condition to obtain the compound **53:** CHIRALPAK IG, 2× 25cm; mobile phase, Hex (0.5% 2M NH3-MeOH) in MeOH, 50% isocratic in 15 min; detector, UV 254 nm.

LC-MS m/z (ESI): 415.8 $[M-H]^-$. $^1H$ NMR (400 MHz, DMSO-$d_6$) δ 11.96 (s, 1H), 10.49 (s, 1H), 8.19 (s, 1H), 7.82 (d, J = 8.5 Hz, 1H), 7.57-7.47 (m, 2H), 7.47-7.37 (m, 1H), 7.33-7.23 (m, 1H), 7.19-7.09 (m, 1H), 6.83 (s, 1H), 5.25-5.15 (m, 2H), 4.36-4.26 (m, 1H), 3. 99-3. 89 (m, 1H).

**Example 54**

**Compound 54: (R,Z)-5-((6-chloro-1H-indol-3-yl)methylene)-3-(1-(3,4-difluorophenyl)-2-hydroxyethyl)imidazolidine-2,4-dione**

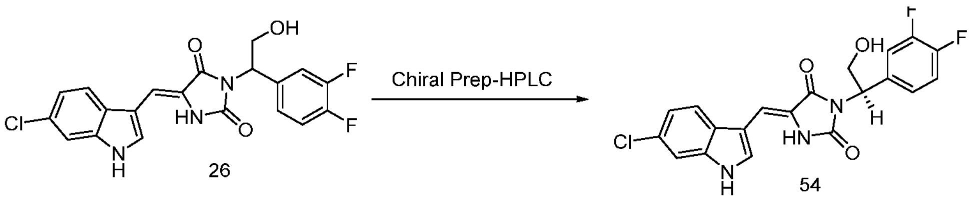


26 54

The compound **26** was separated by chiral HPLC according to the following condition to obtain the compound **54:** CHIRALPAK IG, 2×25cm; mobile phase, Hex (0.5% 2M NH3-MeOH) in MeOH, 50% isocratic in 15 min; detector, UV 254 nm.

LC-MS m/z (ESI): 415.8 $[M-H]^-$. $^1H$ NMR (400 MHz, DMSO-d6) δ 11.96 (s, 1H), 10.49 (s, 1H), 8.19 (s, 1H), 7.82 (d, J = 8.5 Hz, 1H), 7.57-7.37 (m, 3H), 7.33-7.23 (m, 1H), 7.19-7.09 (m, 1H), 6.83 (s, 1H), 5.25-5.15 (m, 2H), 4.36-4.26 (m, 1H), 3. 99-3. 89 (m, 1H).

**Example 55**

**Compound 55: (Z)-(6-chloro-3-((1-(3,4-difluorobenzyl)-2,5-dioxoimidazolidin-4-ylene)methyl)-1H-indol-1-yl) phosphonic acid**

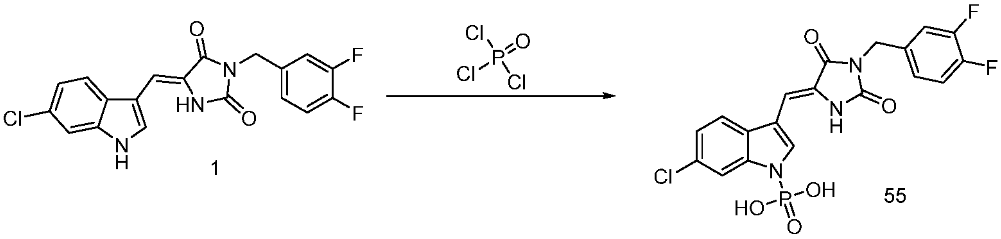

1

55

NaH (68 mg, 2.84 mmol, 2.20 eq) was added into a solution of the compound 1 (500 mg, 1.29 mmol, 1.00 eq) in THF (10 mL) and DCM (20 mL), and the mixture was stirred under argon protection at room temperature for 15 min. Phosphorus oxychloride (988 mg, 6.44 mmol, 5.00 eq) was dropwise added into the mixture at 0° C, and the mixture was stirred under argon protection at 0° C for 3 h. The obtained mixture was concentrated at a reduced pressure, and a residue was dissolved in acetonitrile (10 mL). The mixture was alkalized with cold saturated sodium hydrogen carbonate till pH=8, and the obtained mixture was stirred at 0° C for 30 min. The mixture was acidized with concentrated hydrochloric acid till pH=1, and the obtained mixture was stirred at 0° C for 30 min and extracted with EtOAc (3×30 mL). Combined organic layers were dried with anhydrous sodium sulfate. After the organic layers were filtered, a filtrate was concentrated at a reduced pressure. A crude product was purified by Prep-HPLC to obtain the yellow solid compound **55** (20.1 mg, 3.10%).
LC-MS m/z (ESI): 466.0[M-H]$^-$ .$^1$H NMR (300 MHz, DMSO-$d_6$) δ 8.36 (s, 1H), 8.01 (s, 1H), 7. 75-7. 55 (m, 1H), 7.54-7.29 (m, 2H), 7.28-7.00 (m, 2H), 6. 72 (s, 1H), 4. 70 (s, 2H).

**Example 56**

**Compound 56: (Z)-(6-chloro-3-((1-(1-(4-cyano-3-fluorophenyl)-2-((2-hydroxyethyl)amino)-2-oxyethyl)-2, 5-dioxoimidazolidin-4-ylene)methyl)-7-fluoro-1H-indol-1-yl)phosphonic acid**

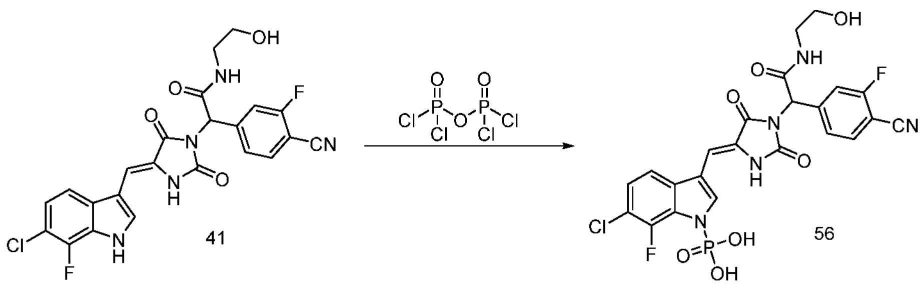

41

56

By using the method for the compound 42, the yellow solid compound 56 (6.1 mg, 15.6%) was prepared by using the compound 41.
LC-MS m/z (ESI): 578.2 [M-H]$^-$. $^1$H NMR (300 MHz, DMSO-$d_6$) δ 8.19 (s, 1H), 7. 85-7. 75 (m, 1H), 7.59 (d, J = 8.6 Hz, 1H), 7.49 (d, J = 10.4 Hz, 1H), 7.39 (d, J = 8.1 Hz, 1H), 7.20-7.09 (m, 1H), 6.76 (s, 1H), 4.50 (s, 1H), 3.70-3.61 (m, 2H), 3.36-3.17 (m, 2H).

**Example 57**

**Compound 57: (Z)-2-(4-((6-chloro-7-methyl-1H-indol-3-yl)methylene)-2,5-dioxoimidazolidin-1-yl)-2-(3,4-difluorophenyl)-N-(3-hydroxylbicyclo[1.1.1]pentan-1-yl)acetamide**

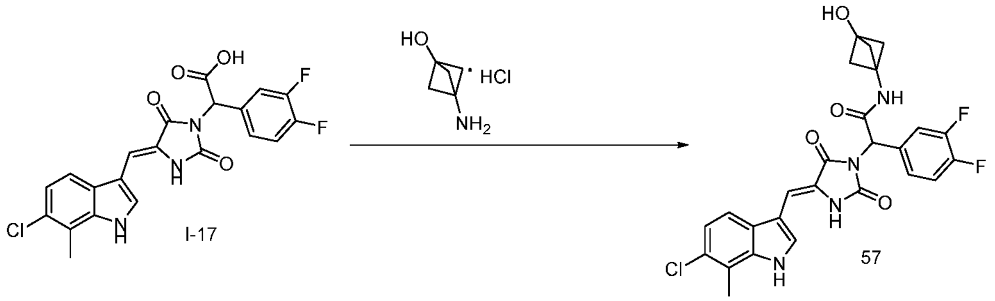


By using the method for the compound **41,** the yellow solid compound **57** (12.4 mg, 34.5%) was prepared by using **I-17.**

LC-MS m/z (ESI): 527.2 $[M+H]^+$. $^1H$ NMR (400 MHz, DMSO-*d6*) δ 11.99 (s, 1H), 10.51 (s, 1H), 8.68 (s, 1H), 8.20-8.19 (m, 1H), 7.67-7.64 (d, J = 8.5 Hz, 1H), 7. 50-7. 35 (m, 2H), 7. 22-7. 13 (m, 2H), 6.82 (s, 1H), 6.18 (s, 1H), 5.68 (s, 1H), 2. 54 (s, 3H), 2. 02 (s, 6H).

**Example 58**

**Compound 58: (Z)-2-(4-((6-chloro-7-fluoro-1H-indol-3-yl)methylene)-2,5-dioxoimidazolidin-1-yl)-2-(4-cyanophenyl)-N-(2-hydroxyethyl) acetamide**

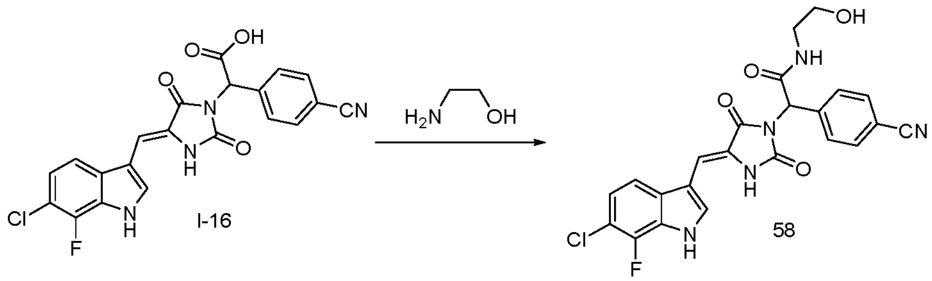

By using the method for the compound **41,** the yellow solid compound **58** (11.2 mg, 29.9%) was prepared by using the **I-16.**

LC-MS m/z (ESI): 480.0 [M-H] . $^1H$ NMR (300 MHz, DMSO-$d_6$) δ 12.54 (s, 1H), 10.61 (s, 1H), 8.30-8.14 (m, 2H), 7.83 (d, *J* = 7.9 Hz, 2H), 7. 72-7. 52 (m, 3H), 7. 32-7. 15 (m, 1H), 6.82 (s, 1H), 5.84 (s, 1H), 4. 66-4. 56 (m, 1H), 3. 53-3. 15 (m, 4H).

**Example 59**

**Compound 59: (Z)-2-(4-((6-chloro-7-fluoro-1H-indol-3-yl)methylene)-2,5-dioxoimidazolidin-1-yl)-2-(4-chlorophenyl)-N-(2-hydroxyethyl)acetamide**

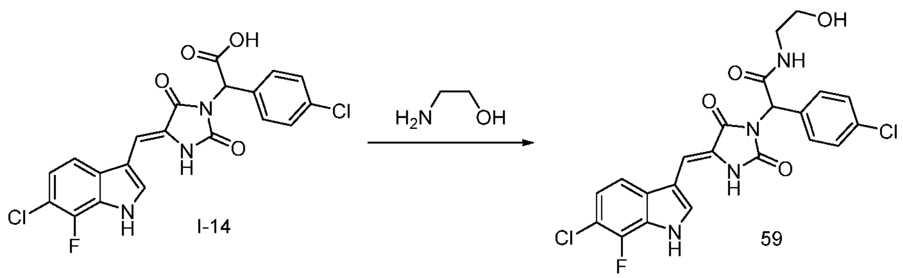

By using the method for the compound **41,** the yellow solid compound 59 (6.2 mg, 17.0%) was prepared by using the **I-14.**

LC-MS m/z (ESI): 491.0 [M+H]$^+$. $^1$H NMR (400 MHz, DMSO-$d_6$) δ 12.54 (s, 1H), 10.59 (s, 1H), 8.25 (s, 1H), 8.09-8.00 (m, 1H), 7.67 (d, *J* = 8.5 Hz, 1H), 7. 52-7. 38 (m, 4H), 7. 25-7. 17 (m, 1H), 6.82 (s, 1H), 5.74 (s, 1H), 4. 65-4. 58 (m, 1H), 3.46-3.39 (m, 2H), 3. 28-3. 10 (m, 2H).

**Example 60**

**Compound 60: (Z)-2-(4-((6-chloro-7-methyl-1H-indol-3-yl)methylene)-2,5-dioxoimidazolidin-1-yl)-2-(4-cyanophenyl)-N-(2-hydroxyethyl)acetamide**

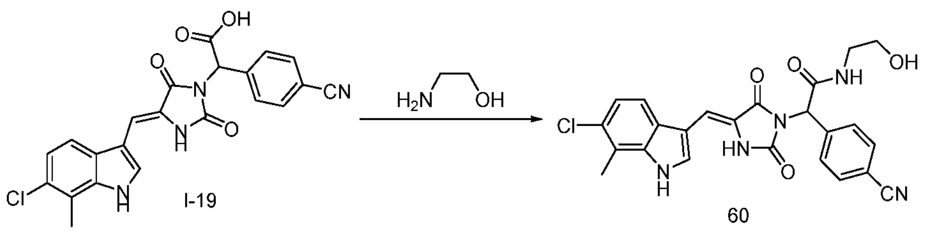

I-19 60

By using the method for the compound **41,** the yellow solid compound **60** (12 mg, 42. 1%) was prepared by using **I-19.**

LC-MS m/z (ESI): 476.1 [M-H]$^-$. $^1$H NMR (300 MHz, DMSO-$d_6$) δ 11. 99 (s, 1H), 10.54 (s, 1H), 8.24-8.13 (m, 2H), 7. 87-7. 78 (m, 2H), 7. 68-7. 55 (m, 3H), 7. 15 (d, J = 8.5 Hz, 1H), 6. 83 (s, 1H), 5. 84 (s, 1H), 4. 65-4. 55 (m, 1H), 3. 49-3. 36 (m, 2H), 3. 27-3.08 (m, 2H), 2. 50 (s, 3H).

**Example 61**

**Compound 61: (Z)-2-(4-((6-chloro-1H-indol-3-yl)methylene)-2,5-dioxoimidazolidin-1-yl)-2-(4-cyanophenyl)-N-(2-hydroxyethyl)acetamide**

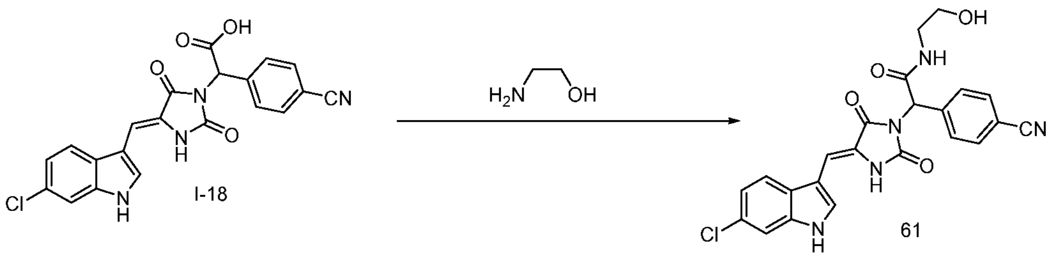

I-18 61

By using the method for the compound **41,** the yellow solid compound **61** (14.1 mg, 29.9%) was prepared by using **I-18.**

LC-MS m/z (ESI) : 464.1 [M+H]$^+$. $^1$H NMR (300 MHz, DMSO-$d_6$) δ 11.97 (s, 1H), 10. 54 (s, 1H), 8.23-8.13 (m, 2H), 7.87-7.77 (m, 3H), 7.65-7.55 (m, 2H), 7.53-7.43 (m, 1H), 7.19-7.09 (m, 1H), 6.85 (s, 1H), 5.84 (s, 1H), 4. 70-4. 56 (m, 1H), 3.48-3.37 (m, 2H), 3.29-3.08 (m, 2H).

**Example 62**

**Compound 62: (Z)-2-(4-((6-chloro-1H-indol-3-yl)methylene)-2,5-dioxoimidazolidin-1-yl)-2-(4-chlorophenyl)-N-(2-hydroxyethyl)acetamide**

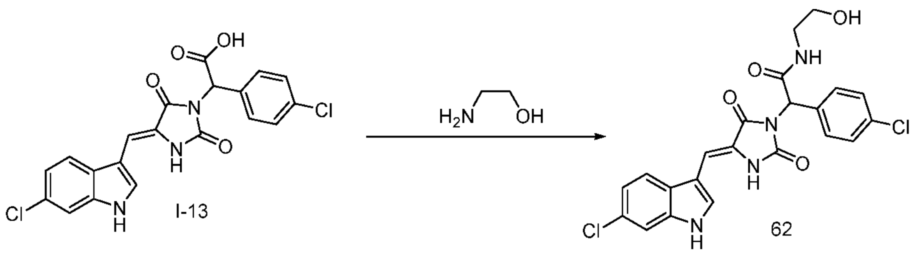


By using the method for the compound **41,** the yellow solid compound **62** (12.1 mg, 38.5%) was prepared by using **I-13.**

LC-MS m/z (ESI): 473.0 $[M+H]^+$. $^1H$ NMR (400 MHz, DMSO-*d6*) δ 11.95 (s, 1H), 10. 50 (s, 1H), 8.18 (s, 1H), 8.05-8.03 (m, 1H), 7.82 (d, J = 8.5Hz, 1H), 7. 48-7. 39 (m, 5H), 7. 13 (d, J = 8.5Hz, 1H), 6.83 (s, 1H), 5. 73 (s, 1H), 4. 61-4. 57 (m, 1H), 3.44-3.39 (m, 2H), 3.25-3.13 (m, 2H).

**Example 63**

**Compound 63: (Z)-2-(4-((6-chloro-1H-indol-3-yl)methylene)-2,5-dioxoimidazolidin-1-yl)-2-(3,4-difluorophenyl)-N-(2-hydroxyethyl)acetamide**

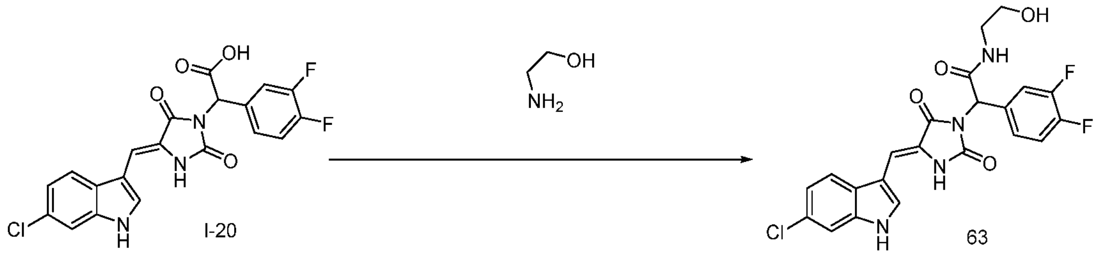


By using the method for the compound **41,** the yellow solid compound 63 (11 mg, 14. 5%) was prepared by using the **I-20.**

LC-MS m/z (ESI): 475.0 $[M+H]^+$. $^1H$ NMR (300 MHz, DMSO-$d_6$) δ 11.97 (s, 1H), 10. 52 (s, 1H), 8.19 (s, 1H), 8.11-8.00 (m, 1H), 7.82 (d, *J* = 8.6 Hz, 1H), 7. 58-7.34 (m, 3H), 7.32-7.21 (m, 1H), 7.14 (d, *J* = 8.6, 1H), 6.85 (s, 1H), 5.75 (s, 1H), 3.48-3.35 (m, 2H), 3.31-3.05 (m, 2H).

**Example 64**

**Compound 64: (Z)-2-(4-((6-chloro-7-methyl-1H-indol-3-yl)methylene)-2,5-dioxoimidazolidin-1-yl)-2-(3,4-difluorophenyl)-N-((4-hydroxylbicyclo[2.2.2]octan-1-yl)methyl)acetamide**

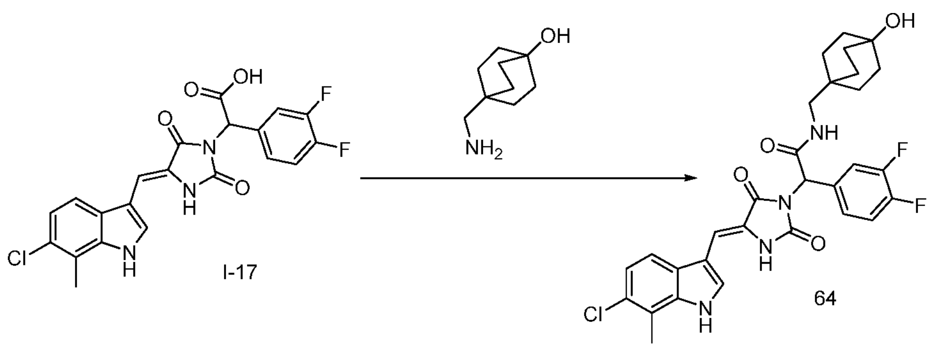


By using the method for the compound **41,** the yellow solid compound **64** (19.1 mg, 47.0%) was prepared by using **I-17.**

LC-MS m/z (ESI): 581.0 $[M-H]^-$. $^1H$ NMR (400 MHz, DMSO-*d6*) δ 12.00 (s, 1H), 10. 53 (s, 1H), 8.28 (s, 1H), 7.97-7.94 (m, 1H), 7.63 (d, J = 8.4 Hz, 1H), 7.51-7.26 (m, 2H), 7.30-7.22 (m, 1H), 7.15 (d, J = 8.4 Hz, 1H), 6.82 (s, 1H), 5.74

(s, 1H), 4.22-4.12 (m, 1H), 2.89-2.78 (m, 2H), 2.51 (s, 3H), 1. 43-1. 42 (m, 12H).

**Example 65**

**Compound 65: (Z)-2-(2-(4-((6-chloro-7-methyl-1H-indol-3-yl)methylene)-2,5-dioxoimidazolidin-1-yl)-2-(3,4-difluorophenyl)acetamido)-3-hydroxypropyl dihydrophosphate**

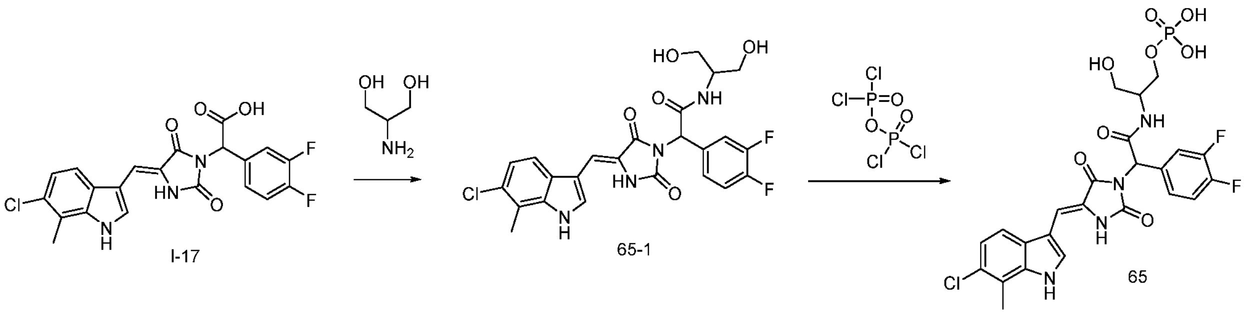

I-17

65-1

65

**65-1: (Z)-2-(4-((6-chloro-7-methyl-1H-indol-3-yl)methylene)-2,5-dioxoimidazolidin-1-yl)-2-(3,4-difluorophenyl)-N-(1,3-dihydroxypropan-2-yl)acetamide**

By using the method for the compound **41,** the yellow solid compound **65-1** (23.4 mg, 41%) was prepared by using the **I-17.**

LC-MS m/z (ESI): 519.05 [M+H]$^+$.

**Compound 65: (Z)-2-(2-(4-((6-chloro-7-methyl-1H-indol-3-yl)methylene)-2,5-dioxoimidazolidin-1-yl)-2-(3,4-difluorophenyl)acetamido)-3-hydroxypropyl dihydrophosphate**

Under argon protection at 0° C, dichlorophosphate anhydride (243 mg, 0.96 mmol, 5.00 eq) was added into a stirred solution of the compound **65-1** (100 mg, 0.19 mmol, 1.00 eq) in ACN (5 mL) and THF (2.5 mL), and the obtained mixture was stirred at 0° C for 3 h. Ice water (3 mL) was added to terminate the reaction, and the mixture was neutralized with saturated sodium hydrogen carbonate till pH=8. The obtained mixture was stirred under argon protection at 0° C for 30 min. The mixture was acidized with concentrated hydrochloric acid till pH=1. The obtained mixture was stirred under argon protection at 0° C for 1.5 h. The obtained mixture was extracted with EtOAc (3×30 mL), and combined organic layers were washed with saline (30 mL) and dried with anhydrous sodium sulfate. After the organic layers were filtered, a filtrate was concentrated at a reduced pressure. The crude product was purified by Prep-HPLC to obtain the yellow solid compound **65** (4.9 mg, 4.16%).

LC-MS m/z (ESI): 597.1[M-H]$^-$. $^1$HNMR (300 MHz, DMSO-$d_6$) δ 8.20 (s, 1H), 7.63 (d, J = 8.5 Hz, 1H), 7. 57-7. 32 (m, 2H), 7.31-7.11 (m, 2H), 6.82 (s, 1H), 5.78 (s, 1H), 3. 88-3. 83 (m, 1H), 3. 82-3. 65 (m, 2H), 3. 46-3. 30 (m, 2H), 2.57 (s, 3H).

**Example 66**

**Compound 66: (Z)-2-(4-((6-chloro-7-methyl-1H-indol-3-yl)methylene)-2,5-dioxoimidazolidin-1-yl)-2-(3,4-difluorophenyl)-N-(4-hydroxyl bicyclo[2.2.2]octan-1-yl)acetamide**

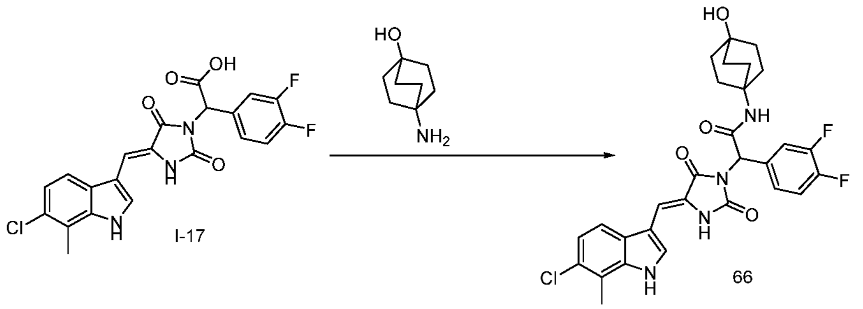


By using the method for the compound **41,** the yellow solid compound **66** (2.2 mg, 49.5%) was prepared by using the **I-17.**

LC-MS m/z (ESI): 566.9 [M-H]$^-$. $^1$H NMR (400 MHz, DMSO-*d6*) δ 11. 97 (s, 1H), 10.04 (s, 1H), 8.18 (s, 1H), 7.64-7.61 (m, 2H), 7.48-7.35 (m, 2H), 7. 18-7. 12 (m, 2H), 6. 77 (s, 1H), 5. 64 (s, 1H), 4. 27 (s, 1H), 2. 45 (s, 3H), 1. 92-1. 87 (m, 6H), 1. 59-1. 53 (m, 6H).

**Example 67**

**Compound 67: (Z)-2-(4-((6-chloro-7-methyl-1H-indol-3-yl)methylene)-2,5-dioxoimidazolidin-1-yl)-2-(4-cyano-3-fluorophenyl)-N-(2-hydroxyethyl)acetamide**

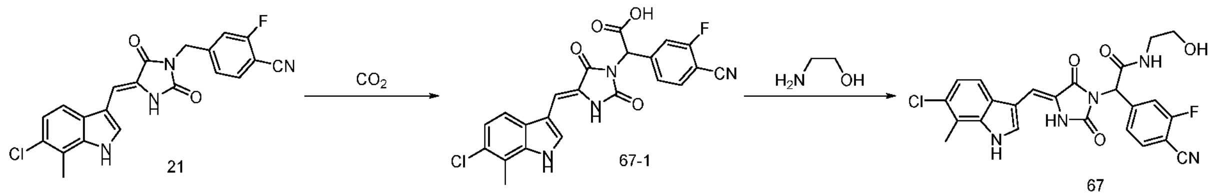


**67-1: (Z)-2-(4-((6-chloro-7-methyl-1H-indol-3-yl)methylene)-2,5-dioxoimidazolidin-1-yl)-2-(4-cyano-3-fluorophenyl)acetic acid**

Under nitrogen protection at -78° C, LiHMDS (1 mL, 1.000 mmol, 5.4 equiv) was added into a stirred solution of the compound **21** (80 mg, 0.186 mmol, 1.0 equiv) in THF (3 mL), and the obtained mixture was stirred at -78° C for 1 h. Then, the mixture was stirred in a $CO_2$ atmosphere to react for 15 min. Then, the obtained mixture was stirred under nitrogen protection at -65° C for 30 min. The reaction was terminated at -65° C with a solution of H0Ac (2 mL) in THF (1 mL). Then, the reaction mixture was poured into 50 mL of cold water. The obtained mixture was extracted with EtOAc (3×30 mL). Combined organic layers were washed with saline (2×15 mL) and dried with anhydrous sodium sulfate. After the organic layers were filtered, a filtrate was concentrated at a reduced pressure. A residue was purified by fast reversed-phase chromatography to obtain the yellow solid compound **67-1** (23 mg, 27.3%).

**Compound 67: (Z)-2-(4-((6-chloro-7-methyl-1H-indol-3-yl)methylene)-2,5-dioxoimidazolidin-1-yl)-2-(4-cyano-3-fluorophenyl)-N-(2-hydroxyethyl)acetamide**

By using the method for the compound **41,** the yellow solid compound **67** (3.7 mg, 13.0%) was prepared by using **67-1.**

LC-MS m/z (ESI): 496. 1 [M+H]$^+$. $^1$H NMR (300 MHz, DMSO-$d_6$) δ 12.01 (s, 1H), 10. 59 (s, 1H), 8.29-8.19 (m, 2H), 7. 98-7. 87 (m, 1H), 7. 67-7. 57 (m, 2H), 7. 53-7. 40 (m, 1H), 7.20-7.05 (m, 1H), 6.85 (s, 1H), 5.88 (s, 1H), 3.48-3.40 (m, 2H), 3.30-3.06 (m, 2H), 2.50 (s, 3H).

**Example 68**

**Compound 68: (Z)-2-(4-((6-chloro-7-methyl-1H-indol-3-yl)methylene)-2,5-dioxoimidazolidin-1-yl)-2-(3,4-difluorophenyl)-N-((3-hydroxyl** bicyclo[1. 1. 1]pentan-1-yl)**methyl)acetamide**

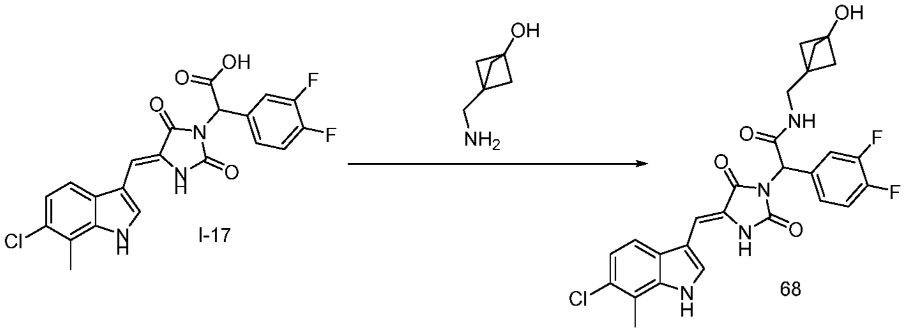

I-17

68

By using the method for the compound **41,** the yellow solid compound **68** (5.1 mg, 24.44%) was prepared by using the **I-17.**

LC-MS m/z (ESI) : 541. 1 $[M+H]^+$. $^1H$ NMR (400 MHz, DMSO-*d6*) δ 11. 99 (s, 1H), 10. 53 (s, 1H), 8.20 (s, 1H), 8. 15-8. 05 (m, 1H), 7. 65 (d, J = 8.5 Hz, 1H), 7. 55-7.37 (m, 2H), 7.40-7.30 (m, 1H), 7.26 (s, 1H), 7.15 (d, J = 8.5 Hz, 1H), 6.82 (s, 1H), 5.73 (s, 1H), 3.35-3.30 (m, 2H), 2.51 (s, 3H), 1.65 (s, 6H).

**Example 69**

**Compound 69: (Z)-5-((6-chloro-7-fluoro-1H-indol-3-yl)methylene)-3-(1-(4-chlorophenyl)-2-hydroxyethyl)imidazolidine-2,4-dione**

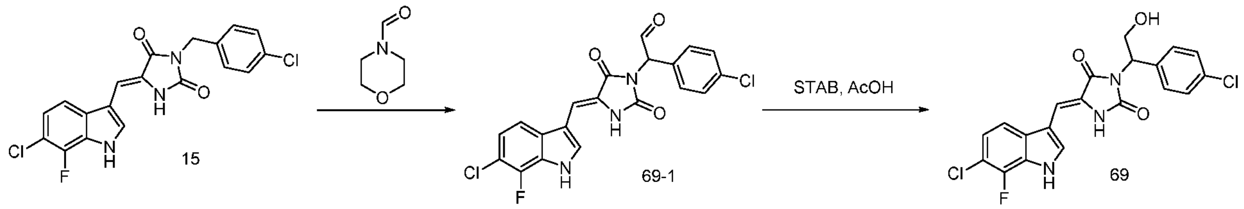


15

69-1

69

**69-1: (Z)-2-(4-((6-chloro-7-fluoro-1H-indol-3-yl)methylene)-2,5-dioxoimidazolidin-1-yl)-2-(4-chlorophenyl)acetaldehyde**

By using the method for the compound **33,** the white solid compound **69-1** (80 mg, 53. 3%) was prepared by using the compound **15.**

LC-MS m/z (ESI): 430.0 $[M-H]^-$

**Compound 69: (Z)-5-((6-chloro-7-fluoro-1H-indol-3-yl)methylene)-3-(1-(4-chlorophenyl)-2-hydroxyethyl)imidazolidine-2,4-dione**

By using the method for the compound **37,** the yellow solid compound **69** (12.3 mg, 20.4%) was prepared by using **69-1.**

LC-MS m/z (ESI): 434.0 $[M+H]^+$. $^1H$ NMR (400 MHz, DMSO-$d_6$) δ 12.53 (s, 1H), 10.55 (s, 1H), 8.25 (s, 1H), 7.66 (d, *J* = 8.5 Hz, 1H), 7.49-7.39 (m, 4H), 7.26-7.16 (m, 1H), 6.81 (s, 1H), 5. 25-5. 15 (m, 2H), 4. 40-4. 31 (m, 1H), 3. 98-3. 92 (m, 1H).

**Example 70**

**Compound 70: (Z)-5-((6-chloro-7-methyl-1H-indol-3-yl)methylene)-3-(1-(4-chlorophenyl)-2-hydroxyethyl)imidazolidine-2,4-dione**

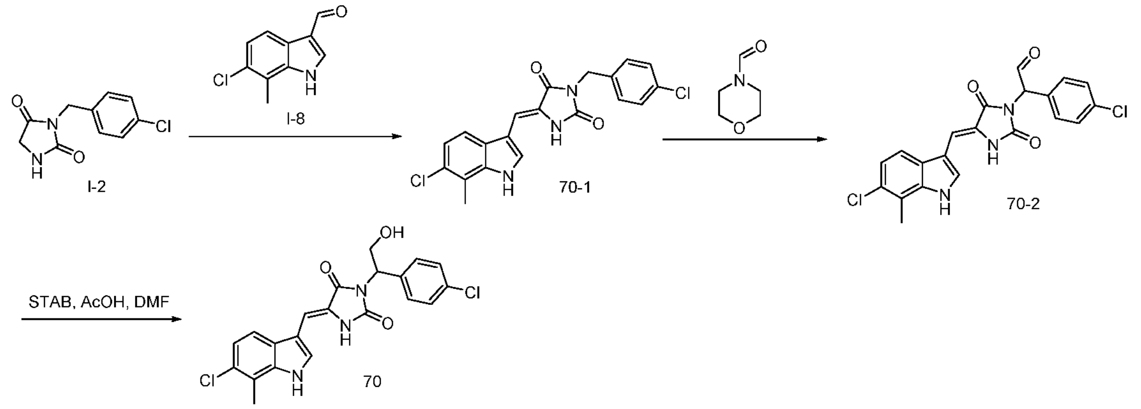

I-2 70-1 70-2 70

**70-1: (Z)-5-((6-chloro-7-methyl-1H-indol-3-yl)methylene)-3-(4-chlorobenzyl)imidazolidine-2,4-dione**

By using the method in Example **1,** the yellow solid compound **70-1** (800 mg, 62%) was prepared by using the intermediates **I-2** and **I-8.**

**70-2: (Z)-2-(4-((6-chloro-7-methyl-1H-indol-3-yl)methylene)-2,5-dioxoimidazolidin-1-yl)-2-(4-chlorophenyl)acetaldehyde**

By using the method for the compound **33,** the white solid compound **70-2** (140 mg, 21.2%) was prepared by using the compound **70-1.**

**Compound 70: (Z)-5-((6-chloro-7-methyl-1H-indol-3-yl)methylene)-3-(1-(4-chlorophenyl)-2-hydroxyethyl)imidazolidine-2,4-dione**

By using the method for the compound **37,** the yellow solid compound **70** (20 mg, 30.5%) was prepared by using the compound **70-2.**

LC-MS m/z (ESI): 428.0 $[M-H]^-$. $^1H$ NMR (400 MHz, DMSO-$d_6$) δ 11.99 (s, 1H), 10.50 (s, 1H), 8.20 (s, 1H), 7.64 (d, $J$ = 8.5 Hz, 1H), 7.46-7.41 (m, 4H), 7.15 (d, $J$ = 8.5 Hz, 1H), 6.82 (s, 1H), 5.26-5.14 (m, 2H), 4.41-4.30 (m, 1H), 4.00-3.90 (m, 1H), 2.52 (s, 3H).

**Example 71**

**Compound 71: (Z)-(6-chloro-3-((1-(4-cyano-3-fluorobenzyl)-2,5-dioxoimidazolidin-4-ylene)methyl)-7-fluoro-1H-indol-1-yl)phosphonic acid**

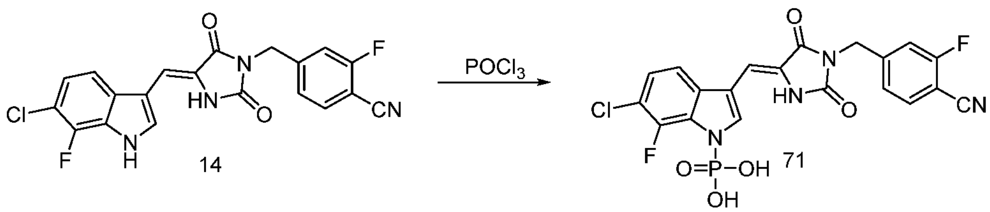

14 71

Under argon protection at 0° C, a solution of phosphorus oxychloride (186 mg, 1.21 mmol, 5.00 eq) in THF (1 mL) was added into a stirred solution of the compound **14** (100 mg, 0.24 mmol, 1.00 eq) in THF (1 mL). A THF solution (1 mL) of TEA (278 mg, 2.74 mmol, 11.32 eq) was dropwise added into the mixture at 0° C, and the obtained mixture was then stirred at 0° C for 2 h. The mixture was alkalized with saturated sodium hydrogen carbonate till pH=8, and the obtained mixture was stirred at 0° C for 30 min. The mixture was acidized with concentrated hydrochloric acid till pH=1. The obtained mixture was diluted with water (20 mL) and extracted with EtOAc (3×50 mL). Combined organic layers were washed with saline (50 mL) and dried with anhydrous sodium sulfate. After the organic layers were filtered, a filtrate was concentrated at a reduced pressure. The crude product was purified by Prep-HPLC to obtain the yellow solid compound **71** (13. 1 mg, 0.018 mmol 7.4%).

LC-MS m/z (ESI): 491.0[M-H]$^-$ .$^1$H NMR (300 MHz, DMSO-$d_6$) δ 8.15 (s, 1H), 7. 91-7. 79 (m, 1H), 7. 47-7. 36 (m, 1H), 7. 32-7. 19 (m, 2H), 7.07-6.97 (m, 1H), 6.27 (s, 1H), 4. 70 (s, 2H).

**Example 72**

**Compound 72: (Z)-2-(4-((6-chloro-7-fluoro-1H-indol-3-yl)methylene)-2,5-dioxoimidazolidin-1-yl)-2-(3,4-difluorophenyl)-N-(2-hydroxyethyl)acetamide**

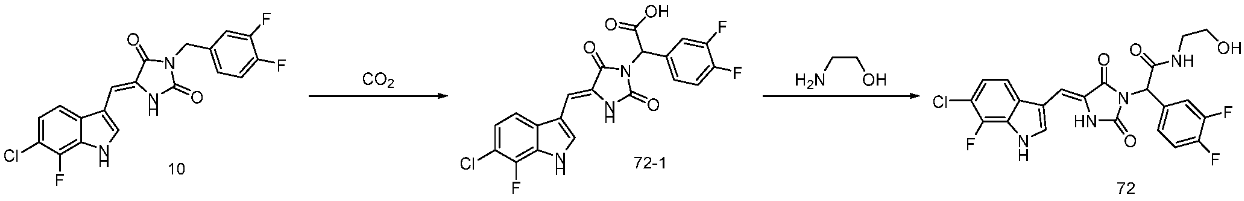


**72-1: (Z)-2-(4-((6-chloro-7-fluoro-1H-indol-3-yl)methylene)-2,5-dioxoimidazolidin-1-yl)-2-(3,4-difluorophenyl) acetic acid**

By using the method for the compound **67-1,** the yellow solid compound **72-1** (23 mg, 47%) was prepared by using the compound **10.**

LC-MS m/z (ESI): 448.01 [M-H]$^-$. $^1$H NMR (400 MHz, DMSO-$d_6$) δ

**Compound 72: (Z)-2-(4-((6-chloro-7-fluoro-1H-indol-3-yl)methylene)-2,5-dioxoimidazolidin-1-yl)-2-(3,4-difluorophenyl)-N-(2-hydroxyethyl)acetamide**

By using the method for the compound **41,** the yellow solid compound **72** (10.6 mg, 45.6%) was prepared by using the compound **72-1.**

LC-MS m/z (ESI): 493. 15 [M+H]$^+$. $^1$H NMR (400 MHz, DMSO-$d_6$) δ 12.54 (d, $J$ = 2.8 Hz, 1H), 10.60 (s, 1H), 8.25 (d, $J$ = 2.8 Hz, 1H), 8.08 (t, $J$ = 5.7 Hz, 1H), 7.68 (d, $J$ = 8.6 Hz, 1H), 7.54-7.48 (m, 1H), 7.46-7.38 (m, 1H), 7.29-7.18 (m, 2H), 6.83 (s, 1H), 5. 76 (s, 1H), 4.61 (s, 1H), 3. 43-3. 40 (m, 2H), 3. 26-3. 12 (m, 2H).

**Example 73**

**Compound 73: (Z)-3-(2-(4-((6-chloro-7-methyl-1H-indol-3-yl)methylene)-2,5-dioxoimidazolidin-1-yl)-2-(3,4-difluorophenyl)acetamido)bicyclo[1.1.1]pentan-1-yl dihydrophosphate**

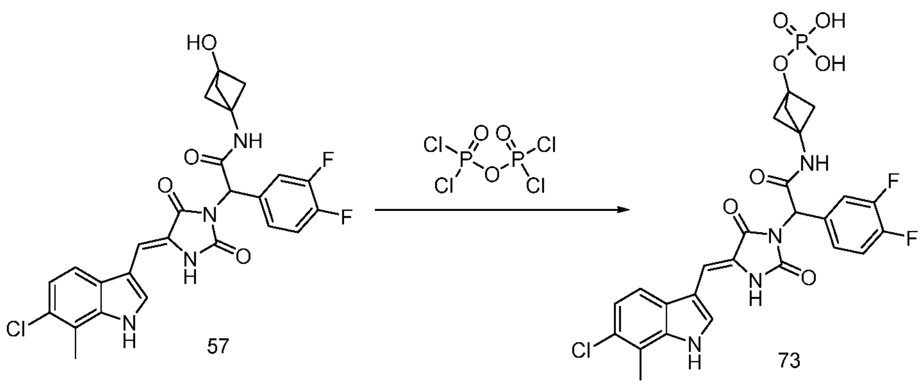

By using the method for the compound **42,** the yellow solid compound **73** (6.1 mg, 16.69%) was prepared by using the compound **57.**

LC-MS m/z (ESI): 607.01 [M+H]$^+$. $^1$H NMR (400 MHz, DMSO-$d_6$) δ 12.04 (s, 1H), 8.72 (s, 1H), 8.12 (s, 1H), 7. 65 (*d,* J = 8.5 Hz, 1H), 7. 48-7. 34 (m, 2H), 7.26-7. 18 (s, 1H), 7. 15 (*d*, J = 8.5 Hz, 1H), 6.82 (s, 1H), 5.69 (s, 1H), 3. 60-3. 50 (m, 5

H), 2.49 (s, 3H), 2.27-2.16 (m, 6H).

**Example 74**

**Compound 74: (Z)-2-(2-(4-((6-chloro-7-methyl-1H-indol-3-yl)methylene)-2, 5-dioxoimidazolidin-1-yl)-2-(3, 4-difluorophenyl)acetamido)propane-1, 3-diylbis(dihydrophosphate)**

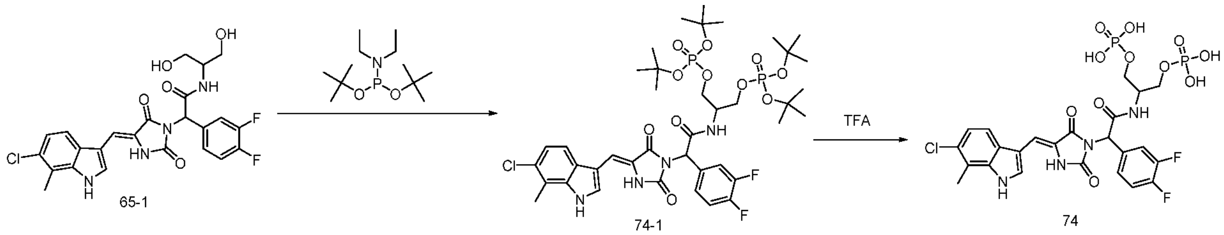


65-1 74-1 74

**74-1: (Z)-tetra-tert-butyl (2-(2-(4-((6-chloro-7-methyl-1H-indol-3-yl)methylene)-2,5-dioxoimidazolidin-1-yl)-2-(3,4-difluorophenyl) acetamido) propane-1,3-diyl) bis (phosphate)**

In an argon atmosphere, a MeCN (9.5 mL) of 1H-1, 2, 3, 4-tetrazole (259 mg, 3.70 mmol, 8.00 eq) and [bis(tert-butoxyl)phosphono]diethylamine (464 mg, 1.86 mmol, 4.02 eq) were added into a solution of **65-1** (240 mg, 0.46 mmol, 1.00 eq) in THF (24 mL), and the obtained mixture was stirred under argon protection at room temperature for 12 h. A solution of metachloroperbenzoic acid (563 mg, 2.78 mmol, 6.00 eq, 85%) in DCM (24 mL) was added into the mixture at 0° C, and the obtained mixture was then stirred at 4° C for 1.5 h. The obtained mixture was diluted with DCM (50 mL) and continuously washed 10% $Na_2S_2O_5$, sodium hydrogen carbonate, and saline respectively. Organic phases were concentrated. A residue was purified by fast reversed-phase chromatography to obtain the light brown syrupy compound **74-1** (120 mg, 26.5%).

LC-MS m/z (ESI): 901.3[M-H]$^-$.

**Compound 74: (Z)-2-(2-(4-((6-chloro-7-methyl-1H-indol-3-yl)methylene)-2,5-dioxoimidazolidin-1-yl)-2-(3,4-difluorophenyl)acetamido)propane-1,3-diylbis(dihydrophosphate)**

TFA (5 mL, 1283 eq) was dropwise added into a solution of **74-1** (50 mg, 0.05 mmol, 1.00 eq) in DCM (10 mL) at 0° C, and a reaction mixture was stirred at room temperature for 1 h. The obtained mixture was concentrated in vacuum. A crude product was purified by Prep-HPLC to obtain the yellow solid compound **74** (3.8 mg, 11.2%).

LC-MS m/z (ESI) : 677.0[M-H]$^-$ . $^1$H NMR (300 MHz, DMSO-$d_6$) δ 11.99 (s, 1H), 8.19 (s, 1H), 7.65 (d, J = 8.7 Hz, 1H), 7.48-7.34 (m, 3H), 7. 29-7. 20 (m, 1H), 7. 17-7.12 (m, 1H), 6.81 (s, 1H), 4. 80 (s, 1H), 4.02-3.94 (m, 1H), 3.93-3.85 (m, 2H), 3.84-3.66 (m, 2H), 2.52 (s, 3H).

**Example 75**

**Compound 75: (Z)-4-(2-(4-((6-chloro-7-methyl-1H-indol-3-yl)methylene)-2,5-dioxoimidazolidin-1-yl)-2-(3,4-difluorophenyl)acetamido)bicyclo[2.2.2]octan-1-yl dihydrophosphate**

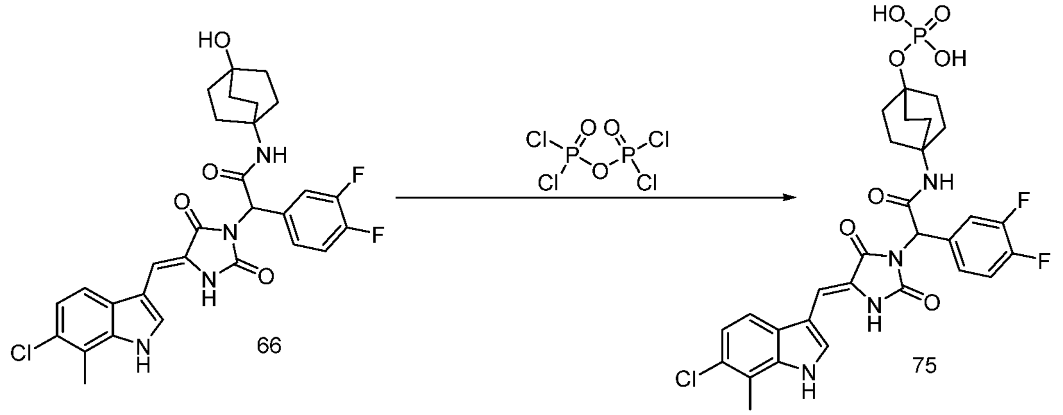

By using the method for the compound **42,** the yellow solid compound **75** (15.2 mg, 33.7%) was prepared by using the compound **66.**

LC-MS m/z (ESI): 647.05 $[M-H]^-$ . $^1H$ NMR (400 MHz, DMSO-$d_6$) δ 12.04 (s, 1H), 8.12 (s, 1H), 7.67-7.61 (m, 2H), 7.46-7.35 (m, 2H), 7.19-7.12 (m, 2H), 6.78 (s, 1H), 5.64 (s, 1H), 2.49 (s, 3H), 1.91 (s, 12H).

## Example 76

**Compound 76: (Z)-4-((2-(4-((6-chloro-7-methyl-1H-indol-3-yl)methylene)-**2, **5-dioxoimidazolidin-1-yl)-2-(3, 4-difluorophenyl)acetamido)methyl)bicyclo[2.2.2]octan-1-yl dihydrophosphate**

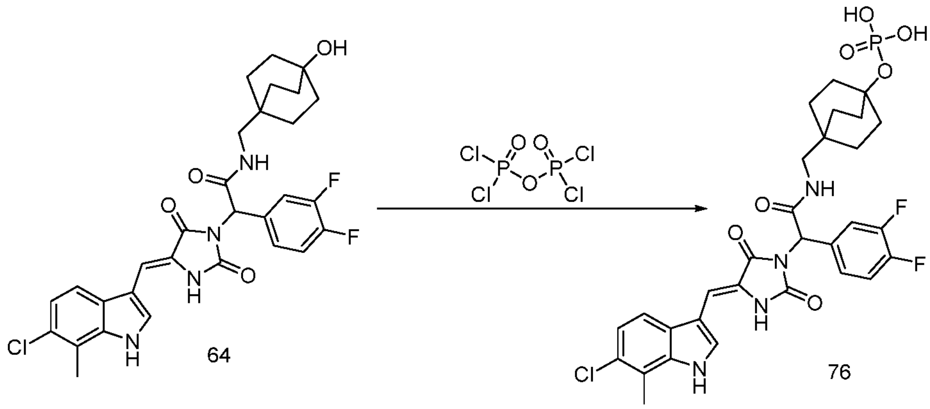

By using the method for the compound **42,** the yellow solid compound **76** (24.7 mg, 37.6%) was prepared by using the compound **64.**

LC-MS m/z (ESI): 663.01 $[M+H]^+$ . $^1H$ NMR (400 MHz, DMSO-$d_6$) δ 11. 93 (s, 1H), 8.13-8.11 (m, 1H), 7. 53-7. 45 (m, 1H), 7. 57-7. 59 (d, *J* = 8.5 Hz, 1H), 7.42-7.29 (m, 2H), 7.22-7.14 (m, 1H), 7.07-7.05 (d, *J* = 8.5 Hz, 1H), 6.75 (s, 1H), 5.67 (s, 1H), 2. 82-2. 60 (m, 2H), 2. 44 (s, 3H), 1. 79-1. 69 (m, 6H), 1. 41-1.31 (m, 6H).

## Example 77:

**Compound 77: (Z)-3-(1-(4-chloro-3-fluorophenyl)-2-hydroxyethyl)-5-((6-chloro-7-fluoro-1H-indol-3-yl)methylene)imidazolidine-2,4-dione**

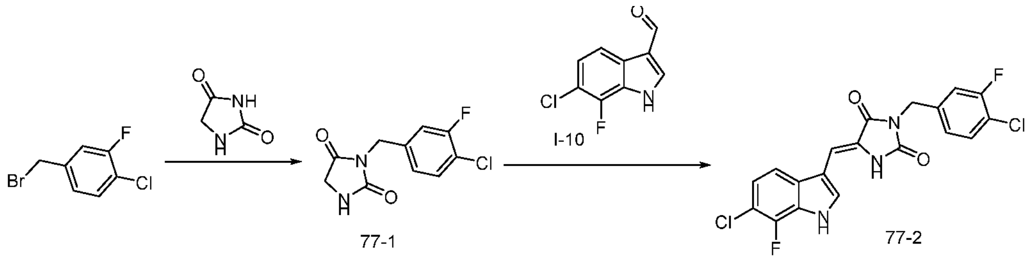

77-1 I-10 77-2

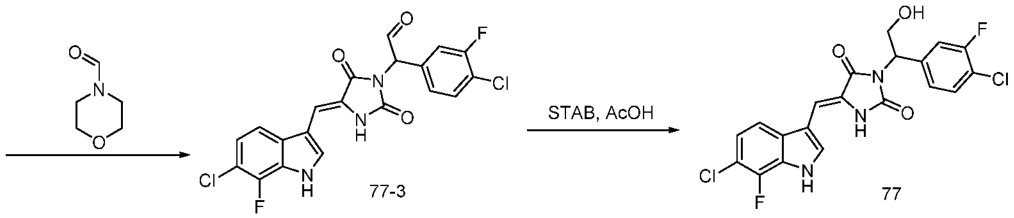


77-3 77

**77-1: 3-(4-chloro-3-fluorobenzyl)imidazolidine-2,4-dione**

By using the method for the compound **I-1,** the offwhite solid compound **77-1** (10 g, 88.4%) was prepared by using hydantoin and 4-(bromomethyl)-1-chloro-2-fluorobenzene.

LC-MS m/z (ESI): 241.0[M-H]$^-$.

**77-2: (Z)-3-(4-chloro-3-fluorobenzyl)-5-((6-chloro-7-fluoro-1H-indol-3-yl)methylene)imidazolidine-2,4-dione**

By using the method for the compound **1,** the yellow solid compound **77-2** (2.0 g, 91.5%) was prepared by using the compounds **77-1** and **I-10.**

LC-MS m/z (ESI): 420.0[M-H]$^-$.

**77-3: (Z)-2-(4-chloro-3-fluorophenyl)-2-(4-((6-chloro-7-fluoro-1H-indol-3-yl)methylene)-2,5-dioxoimidazolidin-1-yl)acetaldehyde**

By using the method for the compound **33,** the yellow solid compound **77-3** (50 mg, 8.3%) was prepared by using the compound **77-2.**

LC-MS m/z (ESI): 450.0[M+H]$^+$ .

**Compound 77: (Z)-3-(1-(4-chloro-3-fluorophenyl)-2-hydroxyethyl)-5-((6-chloro-7-fluoro-1H-indol-3-yl)methylene)imidazolidine-2,4-dione**

By using the method for the compound **37,** the yellow solid compound **77** (22.4 mg, 53.1%) was prepared by using the compound **77-3.**

LC-MS m/z (ESI): 451.9[M+H]$^+$.$^1$H NMR (300 MHz, DMSO-*d$_6$*) δ 12.53 (s, 1H), 10.56 (s, 1H), 8.25 (s, 1H), 7.72-7.64 (m, 1H), 7.63-7. 55 (m, 1H), 7.49 (d, *J* = 10.6, 1H), 7.32- 7. 16 (m, 2H), 6.82 (s, 1H), 5. 30-5. 15 (m, 2H), 4.40-4.20 (m, 1H), 4. 10-3. 90 (m, 1H).

**Example 78:**

**Compound 78: (Z)-3-(1-(4-chloro-3-fluorophenyl)-2-hydroxyethyl)-5-((6-chloro-7-methyl-1H-indol-3-yl)methylene)imidazolidine-2,4-dione**

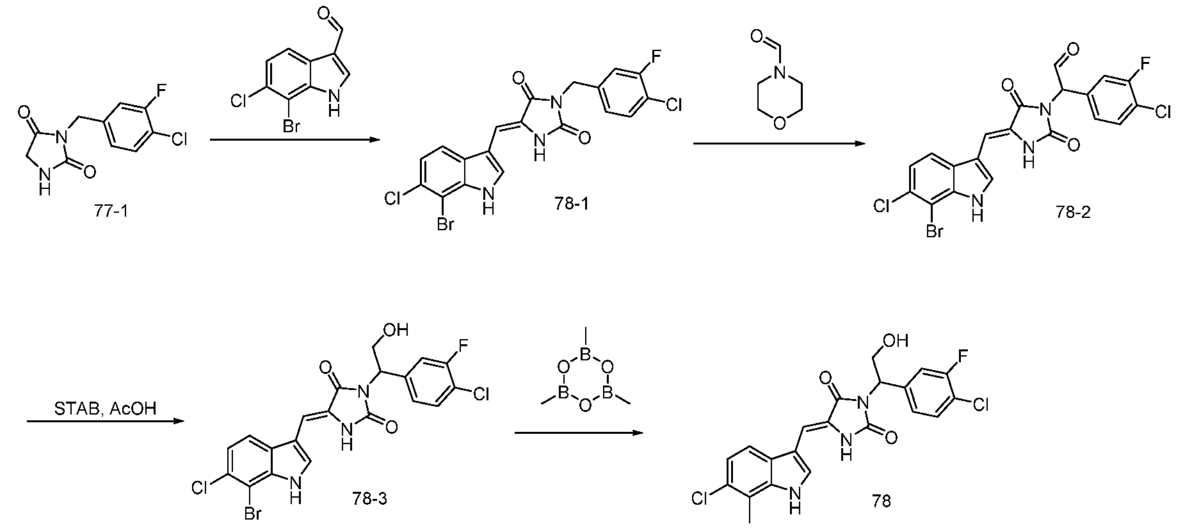


77-1 78-1 78-2 78-3 78

**78-1: (Z)-5-((7-bromo-6-chloro-1H-indol-3-yl)methylene)-3-(4-chloro-3-fluorobenzyl)imidazolidine-2,4-dione**

By using the method for the compound **1,** the yellow solid compound **78-1** (1.9 g, 97.6%) was prepared by using the compound **77-1** and 7-bromo-6-chloro-1H-indole-3-formaldehyde.

LC-MS m/z (ESI): 481.9$[M+H]^+$.

**78-2: (Z)-2-(4-((7-bromo-6-chloro-1H-indol-3-yl)methylene)-2,5-dioxoimidazolidin-1-yl)-2-(4-chloro-3-fluorophenyl)acetaldehyde**

By using the method for the compound **33,** the yellow solid compound **78-2** (110 mg, 13.5%) was prepared by using the compound **78-1.**

LC-MS m/z (ESI): 509.9$[M+H]^+$.

**78-3: (Z)-5-((7-bromo-6-chloro-1H-indol-3-yl)methylene)-3-(1-(4-chloro-3-fluorophenyl)-2-hydroxyethyl)imidazolidine-2,4-dione**

By using the method for the compound **37,** the yellow solid compound **78-3** (66 mg, 57. 5%) was prepared by using the compound **78-2.**

LC-MS m/z (ESI): 510.0$[M-H]^-$. $^1$H NMR (300 MHz, DMS0-d6) δ 12. 18 (s, 1H), 10.64 (s, 1H), 8.26 (s, 1H), 7.86 (d, J = 8.5 Hz, 1H), 7. 60-7. 58 (m, 1H), 7. 48 (d, J = 10.6, 1H), 7.34-7.20 (m, 2H), 6.81 (s, 1H), 5.30-5.10 (m, 2H), 4.40-4.20 (m, 1H), 4. 05-3. 90 (m, 1H).

**Compound 78: (Z)-3-(1-(4-chloro-3-fluorophenyl)-2-hydroxyethyl)-5-((6-chloro-7-methyl-1H-indol-3-yl)methylene)imidazolidine-2,4-dione**

Trimethyl-1, 3, 5, 2, 4, 6-trioxatripyridone (350 uL, 1.23 mmol, 19.64 eq) and potassium carbonate (51 mg, 0.5 mg) were added into a stirred mixture of the compound **78-3** (32 mg, 0.062 mmol, 1.00 eq) and dioxane (3 mL) of and H20 (0.3 mL) of Pd(dppf)$Cl_2$ (8 mg, 0.01 mmol, 0.18 eq), and the obtained mixture was stirred under argon protection at 120° C for 12 h. The mixture was cooled to room temperature and diluted with EtOAc (50 mL). The obtained mixture was washed with saline (5x30 mL), dried with anhydrous $Na_2SO_4$, and filtered. A filtrate was concentrated at a reduced pressure. A crude product was purified by Prep-HPLC to obtain the yellow solid compound **78** (3.1 mg, 9.9%).

LC-MS m/z (ESI): 448.2 $[M+H]^+$ . $^1$H NMR (300 MHz, DMSO-$d_6$) δ 12.00 (s, 1H), 10.53 (s, 1H), 8.21 (s, 1H), 7.72-7.40 (m, 3H), 7.27 (d, *J* = 8.3, 1H), 7. 16 (d, *J* = 8.4 Hz, 1H), 6.84 (s, 1H), 5.30-5.16 (m, 2H), 4.48-4.23 (m, 1H), 4.10-3.90 (m, 1H), 2.45 (s, 3H).

**Example 79:**

**Compound 79: (Z)-5-((6-chloro-1H-indol-3-yl)methylene)-3-(1-(4-chloro-3-fluorophenyl)-2-hydroxyethyl)imidazolidine-2,4-dione**

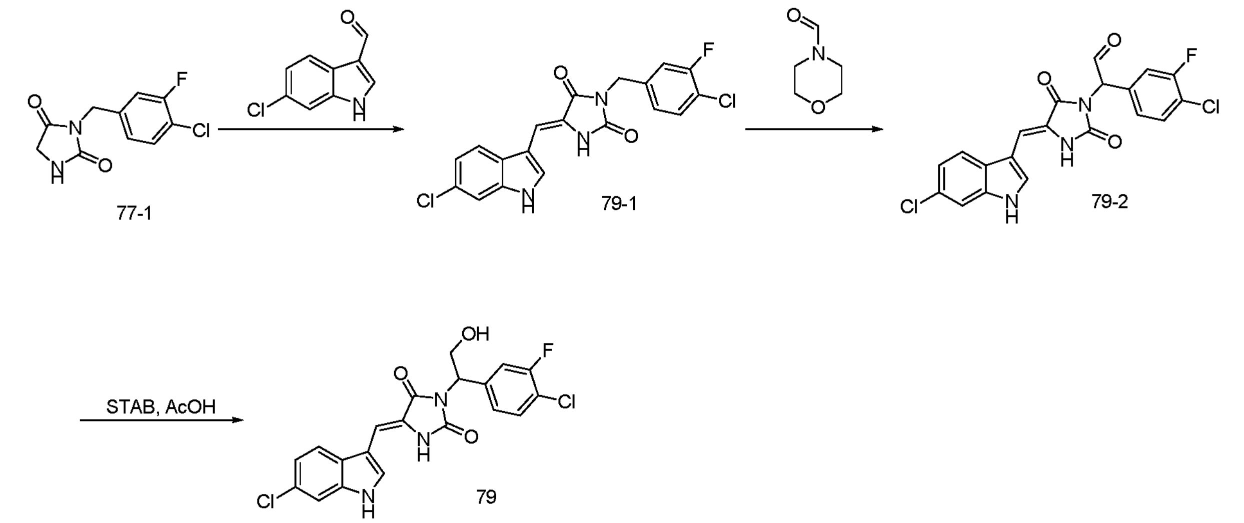


77-1 79-1 79-2 79

**79-1: (Z)-5-((6-chloro-1H-indol-3-yl)methylene)-3-(4-chloro-3-fluorobenzyl)imidazolidine-2,4-dione**

By using the method for the compound **1,** the yellow solid compound **79-1** (2.1 g, 90.3%) was prepared by using the compound **77-1** and 6-chloro-1H-indole-3-formaldehyde.
LC-MS m/z (ESI): 402.0[M-H]$^-$**.**

**79-2: (Z)-2-(4-((6-chloro-1H-indol-3-yl)methylene)-2,5-dioxoimidazolidin-1-yl)-2-(4-chloro-3-fluorophenyl)acetaldehyde**

By using the method for the compound **33,** the yellow solid compound **79-2** (210 mg, 36.1%) was prepared by using the compound **79-1.**
LC-MS m/z (ESI): 432.0[M+H]$^+$ .

**79: (Z)-5-((6-chloro-1H-indol-3-yl)methylene)-3-(1-(4-chloro-3-fluorophenyl)-2-hydroxyethyl)imidazolidine-2,4-dione**

By using the method for the compound **37,** the yellow solid compound **79** (40.2 g, 39.2%) was prepared by using the compound **79-2.**
LC-MS m/z (ESI): 431.8[M-H]$^-$ . $^1$H NMR (400 MHz, DMSO-$d_6$) δ 11.97 (s, 1H), 10.50 (s, 1H), 8.19 (s, 1H), 7.82 (d, J = 8.5 Hz, 1H), 7. 65-7. 55 (m, 1H), 7. 54-7. 45 (m, 2H), 7.27 (d, J = 8.3, 1H), 7.14 (d, J = 8.5, 1H), 6.85 (s, 1H), 5. 30-5. 15 (m, 2H), 4.40-4.18 (m, 1H), 4. 10-3. 90 (m, 1H).

**Example 84:**

**Compound 84: (Z)-5-((6-chloro-7-fluoro-1H-indol-3-yl)methylene)-3-(1-(4-fluorophenyl)-2-hydroxyethyl)imidazolidine-2,4-dione**

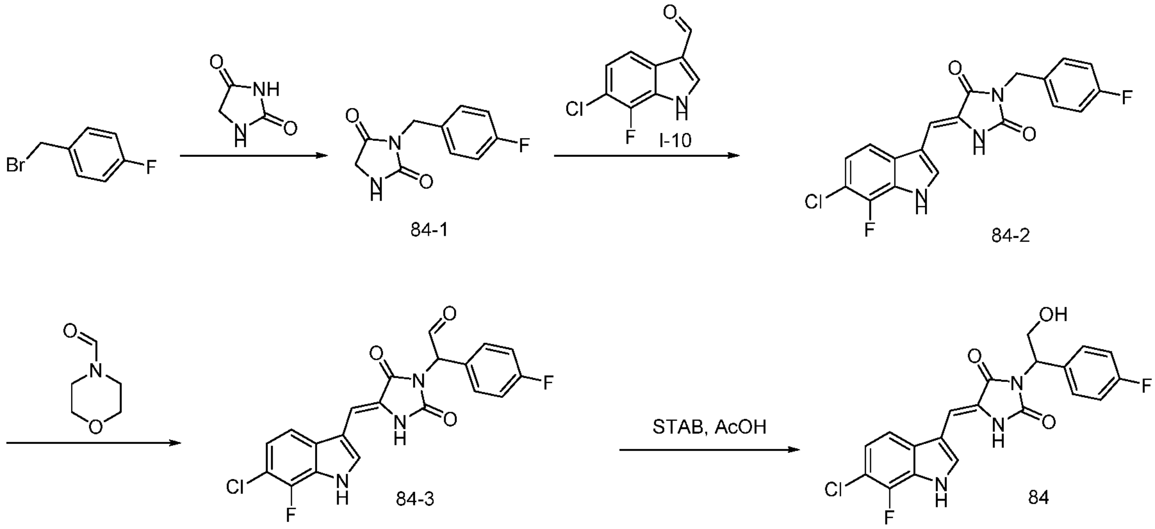

84-1 84-2 84-3 84

**84-1: 3-(4-fluorobenzyl)imidazolidine-2,4-dione**

By using the method for the compound **I-1,** the white solid compound **84-1** (4.12 g, 77.9%) was prepared by using the hydantoin and 4-(bromomethyl)-1, 2-difluorbenzene.

LC-MS m/z (ESI): 207.0 $[M-H]^-$.

**84-2: (Z)-5-((6-chloro-7-fluoro-1H-indol-3-yl)methylene)-3-(4-fluorobenzyl)imidazolidine-2,4-dione**

By using the method for the compound **1,** the yellow solid compound **84-2** (1.1 g, 79.7%) was prepared by using the compounds **84-1** and **I-10.**

LC-MS m/z (ESI): 386.0 $[M-H]^-$ .

**84-3: (Z)-2-(4-((6-chloro-7-fluoro-1H-indol-3-yl)methylene)-2,5-dioxoimidazolidin-1-yl)-2-(4-fluorophenyl)acetaldehyde**

By using the method for the compound **33,** the yellow solid compound **84-3** (340 mg, 61.45%) was prepared by using the compound **84-2.**

LC-MS m/z (ESI): 414.0$[M-H]^-$ .

**Compound 84: (Z)-5-((6-chloro-7-fluoro-1H-indol-3-yl)methylene)-3-(1-(4-fluorophenyl)-2-hydroxyethyl)imidazolidine-2,4-dione**

By using the method for the compound **37,** the yellow solid compound **84** (28.0 mg, 28.2%) was prepared by using the compound **84-3.**

LC-MS m/z (ESI): 418.1$[M+H]^+$ . $^1$H NMR (400 MHz, DMSO-$d_6$) δ 12.52 (s, 1H), 10.56 (s, 1H), 8.25 (s, 1H), 7.71-7.61 (m, 1H), 7.60-7.35 (m, 2H), 7.34-7.10 (m, 3H), 6.81 (s, 1H), 5. 25-5. 15 (m, 2H), 4. 50-4. 30 (m, 1H), 3. 97- 3.88 (m, 1H) .

**Example 85:**

**Compound 85: (Z)-4-(1-(4-((6-chloro-7-fluoro-1H-indol-3-yl)methylene)-2,5-dioxoimidazolidin-1-yl)-2-hydroxyethyl)benzonitrile**

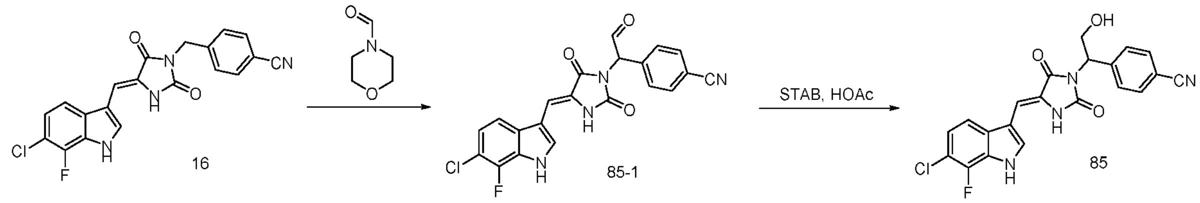


16 85-1 85

**85-1: (Z)-4-(1-(4-((6-chloro-7-fluoro-1H-indol-3-yl)methylene)-2,5-dioxoimidazolidin-1-yl)-2-oxyethyl)benzonitrile**

By using the method for the compound **33,** the brown solid compound **85-1** (120 mg, 23.0%) was prepared by using the compound **16.**

LC-MS m/z (ESI): 423.1[M+H]$^+$ .

**Compound 85: (Z)-4-(1-(4-((6-chloro-7-fluoro-1H-indol-3-yl)methylene)-2, 5-dioxoimidazolidin-1-yl)-2-hydroxyethyl)benzonitrile**

By using the method for the compound **37,** the yellow solid compound **85** (38.1 mg, 31. 1%) was prepared by using the compound **85-1.**

LC-MS m/z (ESI) : 423.1[M-H]$^-$ . $^1$H NMR (300 MHz, DMSO-*d*$_6$) δ 12. 53 (s, 1H), 10. 59 (s, 1H), 8.26 (s, 1H), 7. 89-7. 79 (m, 2H), 7.71-7.61 (m, 3H), 7. 24-7. 19 (m, 1H), 6.83 (s, 1H), 5. 31-5. 24 (m, 2H), 4. 37-4. 29 (m, 1H), 4. 06-3. 99 (m, 1H).

**Example 86:**

**Compound 86: (Z)-5-((6-chloro-7-fluoro-1H-indol-3-yl)methylene)-3-(1-(3,4-difluorophenyl)-2-hydroxyethyl)imidazolidine-2,4-dione**

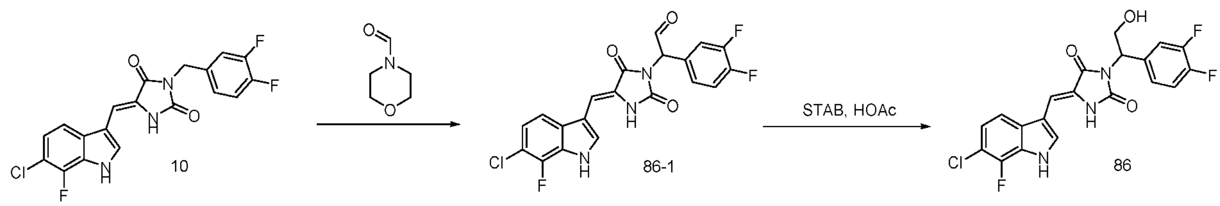


10 86-1 86

**86-1: (Z)-2-(4-((6-chloro-7-fluoro-1H-indol-3-yl)methylene)-2,5-dioxoimidazolidin-1-yl)-2-(3,4-difluorophenyl) acetaldehyde**

By using the method for the compound **33,** the brown solid compound **86-1** (240 mg, 33.5%) was prepared by using the compound **10.**

LC-MS m/z (ESI): 434.0[M+H]$^+$.

**Compound 86: (Z)-5-((6-chloro-7-fluoro-1H-indol-3-yl)methylene)-3-(1-(3,4-difluorophenyl)-2-hydroxyethyl)imidazolidine-2,4-dione**

By using the method for the compound **37,** the yellow solid compound **86** (90 mg, 46.4%) was prepared by using the compound **86-1.**

LC-MS m/z (ESI): 434.1[M-H]$^-$ . $^1$H NMR (400 MHz, DMSO-*d*$_6$) δ 12.52 (s, 1H), 10.56 (s, 1H), 8.25 (s, 1H), 7.67 (d, *J* = 8.6 Hz, 1H), 7.57-7.38 (m, 2H), 7.30-7.17 (m, 2H), 6.82 (s, 1H), 5.25- 5.16 (m, 2H), 4.40-4.25 (m, 1H), 4.00-3.90 (m, 1H) .

**Example 88:**

**Compound 88: (Z)-4-(1-(4-((6-chloro-7-methyl-1H-indol-3-yl)methylene)-2,5-dioxoimidazolidin-1-yl)-2-hydroxyethyl)benzonitrile**

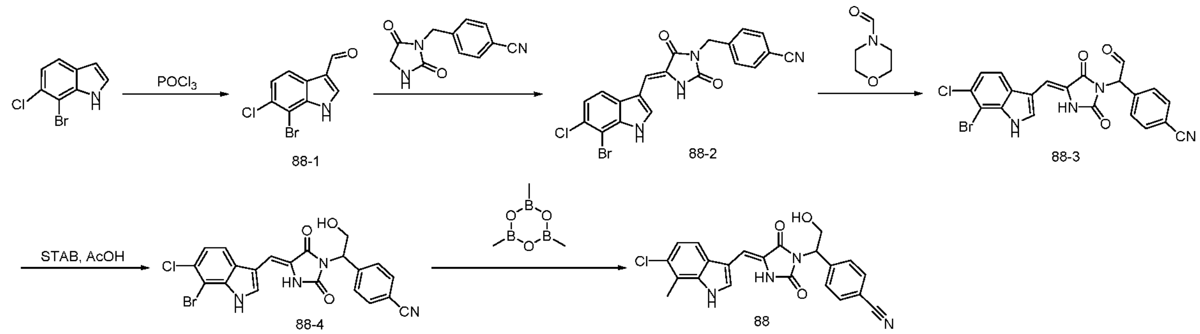


**88-1: 7-bromo-6-chloro-1H-indole-3-formaldehyde**

By using the method for the compound **I-10,** the light brown solid compound **88-1** (4.45 g, 68.9%) was prepared by using 7-bromo-6-chloro-1H-indole.
LC-MS m/z (ESI): 257.9[M+H]$^+$.

**88-2: (Z)-4-((4-((7-bromo-6-chloro-1H-indol-3-yl)methylene)-2,5-dioxoimidazolidin-1-yl)methyl)benzonitrile**

By using the method for the compound **1,** the yellow solid compound **88-2** (5.0 g, 96.9%) was prepared by using the compound **88-1.**
LC-MS m/z (ESI): 454.9[M+H]$^+$.

**88-3: (Z)-4-(1-(4-((7-bromo-6-chloro-1H-indol-3-yl)methylene)-2,5-dioxoimidazolidin-1-yl)-2-oxyethyl)benzonitrile**

By using the method for the compound **33,** the brown solid compound **88-3** (320 mg, 14.9%) was prepared by using the compound **88-2.**
LC-MS m/z (ESI): 482.9[M+H]$^+$ .

**88-4: (Z)-4-(1-(4-((7-bromo-6-chloro-1H-indol-3-yl)methylene)-2,5-dioxoimidazolidin-1-yl)-2-hydroxyethyl)benzonitrile**

By using the method for the compound **37,** the yellow solid compound **88-4** (120 mg, 46.7%) was prepared by using the compound **88-3.**
LC-MS m/z (ESI): 485.0 [M+H]$^+$ .

**Compound 88: (Z)-4-(1-(4-((6-chloro-7-methyl-1H-indol-3-yl)methylene)-2,5-dioxoimidazolidin-1-yl)-2-hydroxyethyl)benzonitrile**

Pd(dppf)$Cl_2$ (18. 1 mg, 0.025 mmol, 0.10 eq) and $K_2CO_3$ (204 mg, 1. 48 mmol, 6.00 eq) were added into a stirred mixture of the compound **88-4** (120 mg, 0.25 mmol, 1.00 eq) and trimethyl-1, 3, 5, 2, 4, 6-trioxaterphenyl (186 mg, 1.48 mmol, 6.00 eq) in 1,4-dioxane (12 mL) and water (0.3 mL), and the obtained mixture was stirred under argon protection at 120° C for 6 h. The mixture was cooled to room temperature and the reaction was terminated at room temperature with water. The obtained mixture was extracted with EtOAc (3x30 mL). Combined organic layers were washed with saline (3x30 mL), dried with anhydrous $Na_2SO_4$, and filtered. A filtrate was concentrated at a reduced pressure. A crude product was purified by Prep-HPLC to obtain the yellow solid compound **88** (19.3 mg, 18.2%).
LC-MS m/z (ESI) : 421.1[M+H]$^+$ . $^1$H NMR (400 MHz, DMSO-$d_6$) $\delta$ 12.00 (s, 1H), 10. 55 (s, 1H), 8.21 (s, 1H), 7.85-7.83 (m, 2H), 7. 65-7. 60 (m, 3H), 7.15 (d, $J$ = 8.5 Hz, 1H), 6. 83 (s, 1H), 5. 30- 5. 26 (m, 1H), 4. 35-4. 30 (m, 1H), 4. 04-3. 99 (m, 1H), 2. 50 (s, 3H) .

**Example 92:**

**Compound 92: (Z)-2-(4-((6-chloro-7-methyl-1H-indol-3-yl)methylene)-2,5-dioxoimidazolidin-1-yl)-2-(4-cyanophenyl)ethyl dihydrophosphate**

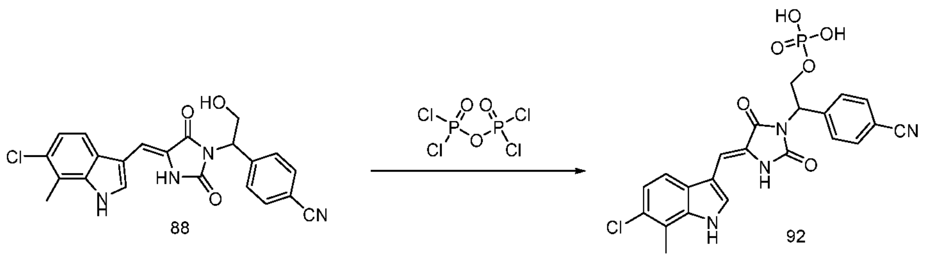

By using the method for the compound **42,** the yellow solid compound **92** (44. 7 mg, 24.6%) was prepared by using the compound **88.**

LC-MS m/z (ESI) : 499.1[M-H]$^-$ . $^1$H NMR (300 MHz, DMSO-$d_6$) δ 12. 04 (s, 1H), 8. 23 (s, 1H), 7.85-7.75 (m, 2H), 7.69-7.56 (m, 3 H), 7. 13 (d, *J* = 8.5 Hz, 1H), 6. 83 (s, 1H), 5. 55-5. 39 (m, 1H), 4. 54-4. 36 (m, 2H), 2. 52 (s, 3H).

**Example 93:**

**Compound 93: (Z)-2-(4-((6-chloro-7-fluoro-1H-indol-3-yl)methylene)-2,5-dioxoimidazolidin-1-yl)-2-(4-chlorophenyl)ethyl dihydrophosphate**

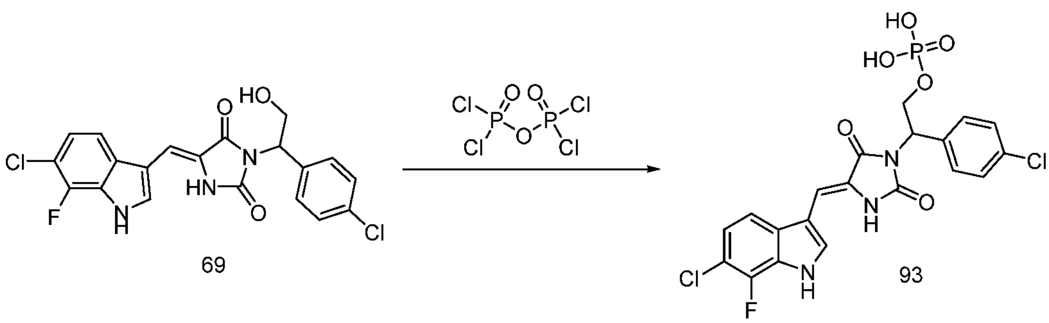

By using the method for the compound **42,** the yellow solid compound **93** (232. 5 mg, 45.0%) was prepared by using the compound **69.**

LC-MS m/z (ESI): 511.9[M-H]$^-$ . $^1$H NMR (400 MHz, DMSO-$d_6$) δ 12.55 (s, 1H), 10.63 (s, 1H), 8.26 (s, 1H), 7.67 (d, J = 8.6 Hz, 1H), 7. 52-7. 38 (m, 4H), 7.30-7.15 (m, 1H), 6. 84 (s, 1H), 5. 50- 5. 32 (m, 1H), 4. 90-4. 67 (m, 1H), 4. 55-4. 36 (m, 1H) .

**Example 94:**

**Compound 94: (S,Z)-2-(4-((6-chloro-1H-indol-3-yl)methylene)-2,5-dioxoimidazolidin-1-yl)-2-(3,4-difluorophenyl) dihydroethyl phosphate**

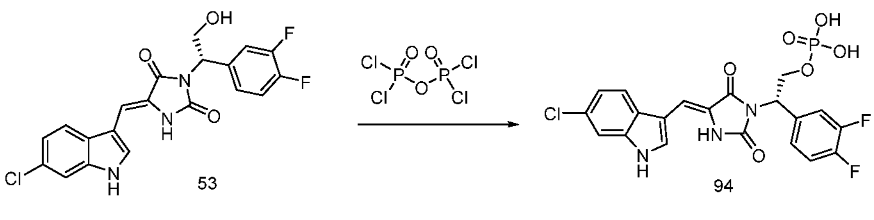

By using the method for the compound **42,** the yellow solid compound **94** (16.4 mg, 0.032 mmol) was prepared by using the compound **53.**

LC-MS m/z (ESI): 498.1 [M+H]$^+$. $^1$H NMR (400 MHz, DMSO-$d_6$) δ 12.04 (s, 1H), 8.19 (s, 1H), 7.83-7.77 (d, *J* = 8.4 Hz, 1H), 7.52-7.47 (m, 2H), 7.54 -7.45 (m, 1H), 7.29-7.25 (m, 1H), 7.13 (d, *J* = 8.4 Hz, 1H), 6.86 (s, 1H), 5.40-5.31 (m, 1H), 4.52-4.47 (m, 2H).

**Example 95:**

**Compound 95: (R,Z)-2-(4-((6-chloro-1H-indol-3-yl)methylene)-2,5-dioxoimidazolidin-1-yl)-2-(3,4-difluorophenyl)dihydroethyl phosphate**

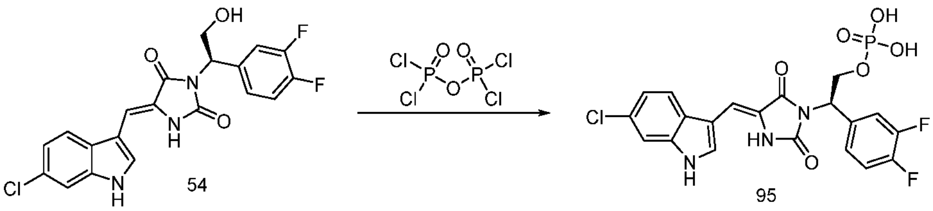

54

95

By using the method for the compound **42,** the yellow solid compound **95** (21.2 mg, 0.042 mmol) was prepared by using the compound **54.**

LC-MS m/z (ESI) : 498. 1 [M+H]$^+$. $^1$H NMR (400 MHz, DMSO- $d_6$) δ 12. 04 (s, 1H), 8.19 (s, 1H), 7. 83-7. 77 (d, *J* = 8.4 Hz, 1H), 7. 52-7. 47 (m, 2H), 7.54 -7.45 (m, 1H), 7.29-7.25 (m, 1H), 7. 13 (d, *J* = 8.4 Hz, 1H), 6.83 (s, 1H), 5.40 - 5.31 (m, 1H), 4. 52-4. 47 (m, 2H).

**Example 96:**

**Compound 96: (R,Z)-4-(1-(4-((6-chloro-7-methyl-1H-indol-3-yl)methylene)-2,5-dioxoimidazolidin-1-yl)-2-hydroxyethyl)benzonitrile**

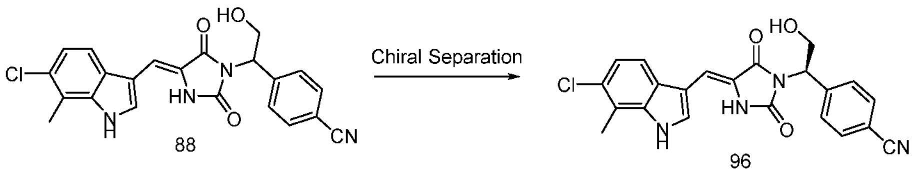


88

96

The compound **88** (60 mg, 0.14 mmol, 1.0 equiv) was separated by a chiral column: CHIRALPAKIG-34.6*50 mm 3 um; Mobile Phase A: MtBE(0.1%DEA): EtOH=93: 7; Flow rate: 1.0 mL/min mL/min; Gradient: isocratic; Injection Volume: 1.0L mL to finally obtain the yellow solid compound **96** (17.0 mg, 29.1%).

LC-MS m/z (ESI) : 419.1[M-H]- . $^1$H NMR (400 MHz, DMSO-$d_6$) δ 12.00 (s, 1H), 10. 55 (s, 1H), 8.24-8. 19 (m, 1H), 7.88-7.81 (m, 2H), 7.67-7.58 (m, 3H), 7. 19-7. 12 (m, 1H), 6. 84 (s, 1H), 5. 32-5. 22 (m, 2H), 4. 39-4. 28 (m, 1H), 4. 07-3. 97 (m, 1H), 2. 52 (s, 3H).

**Example 97:**

**Compound 97: (S,Z)-4-(1-(4-((6-chloro-7-methyl-1H-indol-3-yl)methylene)-2,5-dioxoimidazolidin-1-yl)-2-hydroxyethyl)benzonitrile**

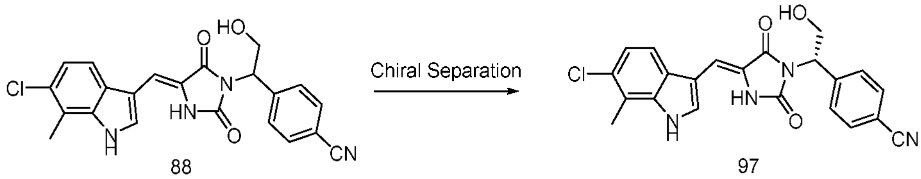


88 97

The compound **88** (60 mg, 0.14 mmol, 1.0 equiv) was separated by a chiral column: CHIRALPAKIG-34.6*50 mm 3 um; Mobile Phase A: MtBE(0. 1%DEA): EtOH=93: 7; Flow rate: 1.0 mL/min mL/min; Gradient: isocratic; Injection Volume: 1.0L mL to finally obtain the yellow solid compound **97** (23.3 mg, 39.8%).
LC-MS m/z (ESI) : 419.1[M-H]$^-$ . $^1$H NMR (400 MHz, DMSO-$d_6$) δ 12.00 (s, 1H), 10. 55 (s, 1H), 8.24-8.19 (m, 1H), 7. 88-7.81 (m, 2H), 7. 67-7. 58 (m, 3H), 7. 19-7. 12 (m, 1H), 6.84 (s, 1H), 5. 32-5. 22 (m, 2H), 4. 39-4. 28 (m, 1H), 4. 07-3. 97 (m, 1H), 2. 52 (s, 3H).

**Example 98:** Compound 98: (Z)-3-(4-chloro-2-fluorobenzyl)-5-((6-chloro-7-fluoro-1H-indol-3-yl)methylene)imidazolidine-2,4-dione

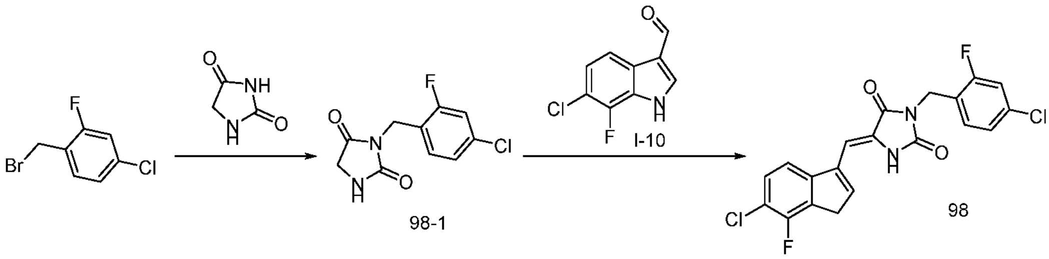


**98-1: 3-(4-chloro-2-fluorobenzyl)imidazolidine-2,4-dione**

By using the method for the compound **I-1,** the offwhite solid compound **98-1** (2.2 g, 66.1%) was prepared by using 1-(bromomethyl)-4-chloro-2-fluorobenzene and hydantoin.
LC-MS m/z (ESI): 241.0[M-H]$^-$ .

**Compound 98: (Z)-3-(4-chloro-2-fluorobenzyl)-5-((6-chloro-7-fluoro-1H-indol-3-yl)methylene)imidazolidine-2,4-dione**

By using the method for the compound **1,** the light yellow solid compound **98** (36.3 mg, 16.2%) was prepared by using **98-1** and **I-10.**
LC-MS m/z (ESI): 420.1[M-H]$^-$ . $^1$H NMR (400 MHz, DMSO-$d_6$) δ 12.55 (s, 1H), 10.60 (s, 1H), 8.26 (s, 1H), 7.68 (d, J = 8.6 Hz, 1H), 7.47-7.44 (m, 1H), 7.36-7.34 (m, 1H), 7. 30-7. 27 (m, 1H), 7. 25-7. 20 (m, 1H), 6.85 (s, 1H), 4.72 (s, 2H).

**Example 99: Compound 99: (Z)-3-(1-(4-chloro-2-fluorophenyl)-2-hydroxyethyl)-5-((6-chloro-7-fluoro-1H-indol-3-yl)methylene)imidazolidine-2,4-dione**

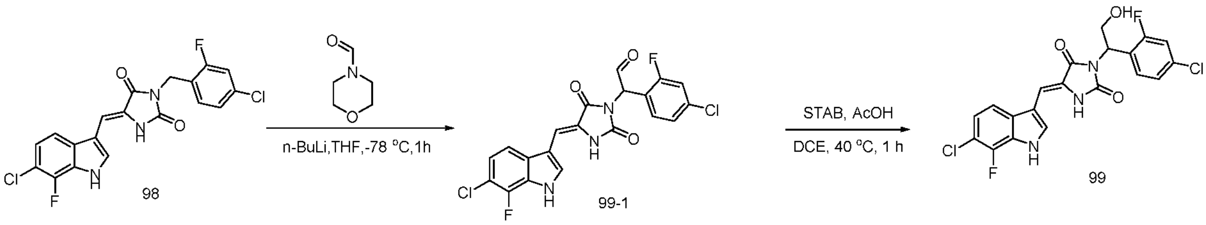

**99-1: (Z)-2-(4-chloro-2-fluorophenyl)-2-(4-((6-chloro-7-fluoro-1H-indol-3-yl)methylene)-2,5-dioxoimidazolidin-1-yl)acetaldehyde**

By using the method for the compound **33,** the brown solid compound **99-1** (120 mg, 33. 1%) was prepared by using the compound **98.**

LC-MS m/z (ESI): 450.0[M+H]+ .

**Compound 99: (Z)-3-(1-(4-chloro-2-fluorophenyl)-2-hydroxyethyl)-5-((6-chloro-7-fluoro-1H-indol-3-yl)methylene)imidazolidine-2,4-dione**

By using the method for the compound **37,** the yellow solid compound **99** (32.5 mg, 30.8%) was prepared by using the compound **99-1.**

LC-MS m/z (ESI): 450.1[M-H]- . $^1$H NMR (400 MHz, DMSO-$d_6$) δ 12.53 (s, 1H), 10. 55 (s, 1H), 8.24 (s, 1H), 7.67-7.62 (m, 2H), 7. 46-7. 44 (m, 1H), 7. 34-7. 32 (m, 1H), 7. 23-7. 21 (m, 1H), 6.80 (s, 1H), 5.45- 5.41 (m, 1H), 5. 30- 5.27 (m, 1H), 4. 40- 4. 20 (m, 1H), 3. 99-3. 81 (m, 1H).

**Example 100: Compound 100: (Z)-3-(4-chloro-2,5-difluorobenzyl)-5-((6-chloro-7-fluoro-1H-indol-3-yl)methylene)imidazolidine-2,4-dione**

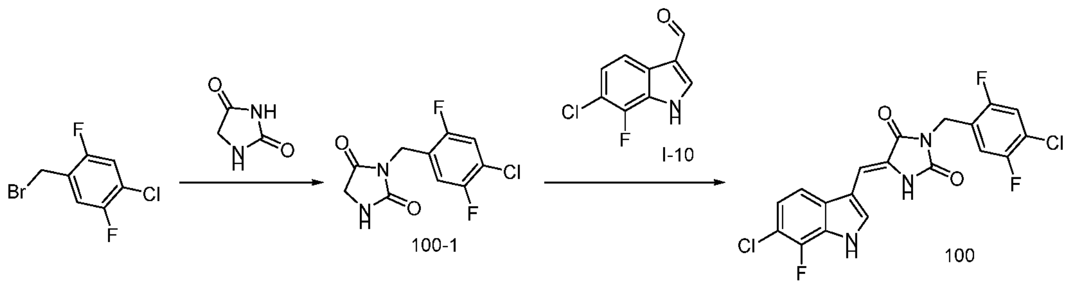

100-1 I-10 100

**100-1: 3-(4-chloro-2,5-difluorobenzyl)imidazolidine-2,4-dione**

By using the method for the compound **I-1,** the light yellow solid compound **100-1** (1.8 g, 78.5%) was prepared by using hydantoin and 1-(bromomethyl)-4-chloro-2, 5-difluorobenzene.

LC-MS m/z (ESI): 259.0[M-H]-.

**Compound 100: (Z)-3-(4-chloro-2,5-difluorobenzyl)-5-((6-chloro-7-fluoro-1H-indol-3-yl)methylene)imidazolidine-2,4-dione**

By using the method for the compound **1,** the light yellow solid compound 100 (45.6 mg, 19.5%) was prepared by using the compounds **100-1** and **I-10.**

LC-MS m/z (ESI): 438.0[M-H]- . $^1$H NMR (400 MHz, DMSO-$d_6$) δ 12.55 (s, 1H), 10.60 (s, 1H), 8.26 (s, 1H), 7.69-7.66 (m, 2H), 7.50-7.42 (m, 1H), 7.28-7.17 (m, 1H), 6.85 (s, 1H), 4.72 (s, 2H).

**Example 101: Compound 101: (Z)-3-(1-(4-chloro-2,5-difluorophenyl)-2-hydroxyethyl)-5-((6-chloro-7-fluoro-1H-indol-3-yl)methylene)imidazolidine-2,4-dione**

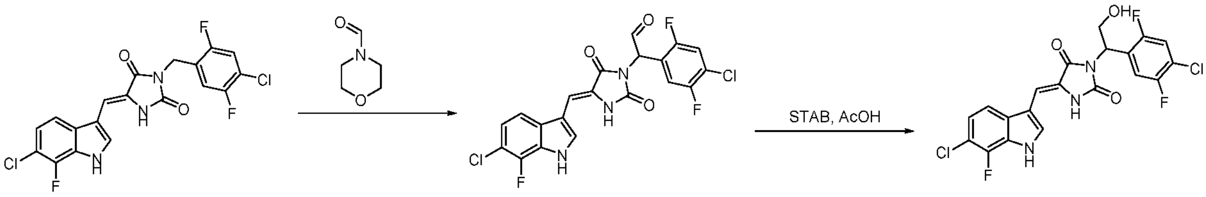


100 101-1 101

**101-1: (Z)-2-(4-chloro-2,5-difluorophenyl)-2-(4-((6-chloro-7-fluoro-1H-indol-3-yl)methylene)-2,5-dioxoimidazolidin-1-yl)acetaldehyde**

By using the method for the compound **33,** the brown solid compound **101-1** (200 mg, 56.0%) was prepared by using the compound **100.**

LC-MS m/z (ESI): 466.0[M-H]$^-$ .

**Compound 101: (Z)-3-(1-(4-chloro-2,5-difluorophenyl)-2-hydroxyethyl)-5-((6-chloro-7-fluoro-1H-indol-3-yl)methylene)imidazolidine-2,4-dione**

By using the method for the compound **37,** the yellow solid compound **101** (24.3 mg, 14.2%) was prepared by using the compound **101-1.**

LC-MS m/z (ESI) : 468.1[M-H]$^-$ . $^1$H NMR (300 MHz, DMSO-$d_6$) δ 12. 55 (s, 1H), 10. 56 (s, 1H), δ 8.24 (s, 1H), 7. 70-7. 63 (m, 3H), 7. 23-7. 18 (m, 1H), 6.81 (s, 1H), 5. 44-5. 39 (m, 1H), 5. 34-5. 20 (m, 1H), 4. 28-4. 19 (m, 1H), 3. 96-3. 85 (m, 1H).

**Example 102: Compound 102: (Z)-3-(4-chloro-2,6-difluorobenzyl)-5-((6-chloro-7-fluoro-1H-indol-3-yl)methylene)imidazolidine-2,4-dione**

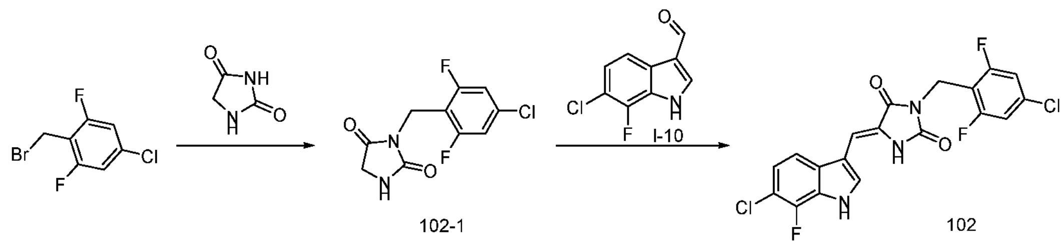


102-1

102

**102-1: 3-(4-chloro-2,6-difluorobenzyl)imidazolidine-2,4-dione**

By using the method for the compound I-1, the white solid compound **102-1** (1.3 g, 56.9%) was prepared by using hydantoin and 2-(bromomethyl)-5-1,3-difluorobenzene.

LC-MS m/z (ESI): 259.0[M-H]$^-$ .

**Compound 102: (Z)-3-(4-chloro-2,6-difluorobenzyl)-5-((6-chloro-7-fluoro-1H-indol-3-yl)methylene)imidazolidine-2,4-dione**

By using the method for the compound 1, the light yellow solid compound 102 (41.4 mg, 18.3%) was prepared by using the compounds **102-1** and **I-10.**

LC-MS m/z (ESI): 438.0[M-H]$^-$ . $^1$H NMR (400 MHz, DMSO-$d_6$) 12.52 (s, 1H), 10.50 (s, 1H), 8.23 (s, 1H), 7.65 (d, *J* = 8.5 Hz, 1H), 7.42-7.32 (m, 2H), 7.22-7.18 (m, 1H), 6.80 (s, 1H), 4.76 (s, 2H) .

**Example 103: Compound 103: (Z)-3-(1-(4-chloro-2,6-difluorophenyl)-2-hydroxyethyl)-5-((6-chloro-7-fluoro-1H-indol-3-yl)methylene)imidazolidine-2,4-dione**

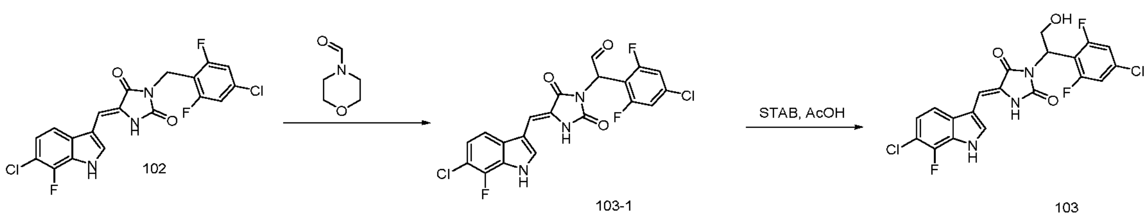


102

103-1

103

**103-1: (Z)-2-(4-chloro-2,6 difluorophenyl)-2-(4-((6-chloro-7-fluoro-1H-indol-3-yl)methylene)-2,5-dioxoimidazolidin-1-yl)acetaldehyde**

By using the method for the compound 33, the brown solid compound 103-1 (160 mg, 36.8%) was prepared by using the compound 102.

LC-MS m/z (ESI): 468.2[M+H]$^+$ .

**Compound 103: (Z)-3-(1-(4-chloro-2,6-difluorophenyl)-2-hydroxyethyl)-5-((6-chloro-7-fluoro-1H-indol-3-yl) methylene)imidazolidine-2,4-dione**

By using the method for the compound 37, the light yellow solid compound 103 (23.3 mg, 12.48%) was prepared by using the compound 103-1.

LC-MS m/z (ESI): 468.1[M-H]$^-$ . $^1$H NMR (300 MHz, DMSO-$d_6$) δ 12.51 (s, 1H), 10.48 (s, 1H), 8.22 (s, 1H), 7.64 (d, $J$ = 8.6 Hz, 1H), 7.42 -7.29 (m, 2H), 7.23-7.10 (m, 1H), 6.77 (s, 1H), 5. 50 -5.30 (m, 1H), 5.28-5.10 (m, 1H), 4. 50-4. 15 (m, 1H), 4. 10-3.90 (m, 1H)

**Example 104: Compound 104: (Z)-3-(4-chloro-2,3-difluorobenzyl)-5-((6-chloro-7-fluoro-1H-indol-3-yl)methylene)imidazolidine-2,4-dione**

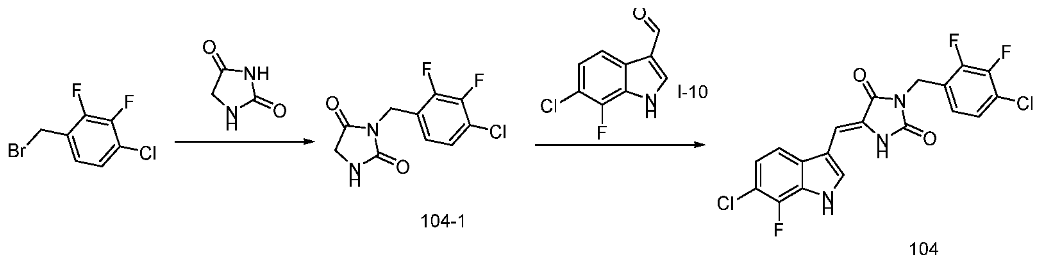


104-1

104

**104-1: 3-(4-chloro-2,3-difluorobenzyl)imidazolidine-2,4-dione**

By using the method for the compound I-1, the white solid compound 104-1 (0.9 g, 3.3 mmol, 79.0%) was prepared by using hydantoin and 1-(bromomethyl)-4-chloro-2, 3-difluorobenzene.

LC-MS m/z (ESI): 259.0[M-H]$^-$.

**Compound 104: (Z)-3-(4-chloro-2,3-difluorobenzyl)-5-((6-chloro-7-fluoro-1H-indol-3-yl)methylene)imidazolidine-2,4-dione**

By using the method for the compound **1,** the yellow solid compound **104** (41.6 mg, 16.2%) was prepared by using the compounds **104-1** and **I-10.**

LC-MS m/z (ESI): 438.0 [M-H]$^-$ . $^1$H NMR (400 MHz, DMSO-$d_6$) δ 12.55 (s, 1H), 10.59 (s, 1H), 8.25 (s, 1H), 7.69 (d, $J$ = 8.6 Hz, 1H), 7.52 -7.38 (m, 1H), 7.28-7.16 (m, 2H), 6.86 (s, 1H), 4.77 (s, 2H)

**Example 105: Compound 105: (Z)-3-(1-(4-chloro-2,3-difluorophenyl)-2-hydroxyethyl)-5-((6-chloro-7-fluoro-1H-indol-3-yl)methylene)imidazolidine-2,4-dione**

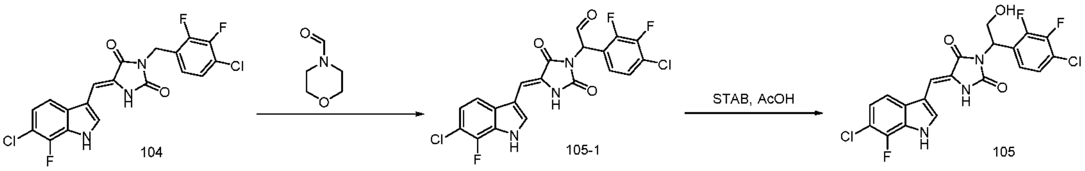


104

105-1

105

**105-1: (Z)-2-(4-chloro-2,3-difluorophenyl)-2-(4-((6-chloro-7-fluoro-1H-indol-3-yl)methylene)-2,5-dioxoimidazolidin-1-yl)acetaldehyde**

By using the method for the compound **33,** the brown solid compound **105-1** (257 mg, 55. 3%) was prepared by using the compound **104.**
LC-MS m/z (ESI): 468.0[M+H]$^+$ .

**Compound 105: (Z)-3-(1-(4-chloro-2,3-difluorophenyl)-2-hydroxyethyl)-5-((6-chloro-7-fluoro-1H-indol-3-yl) methylene)imidazolidine-2,4-dione**

By using the method for the compound **37,** the light yellow solid compound **105** (20. 5 mg, 22.8%) was prepared by using the compound **105-1.**
LC-MS m/z (ESI): 468. 1[M-H]$^-$ . $^1$H NMR (400 MHz, DMSO-$d_6$) δ 12. 54 (s, 1H), 10. 58 (s, 1H), 8.24 (s, 1H), 7.66 (d, $J$ = 8.5 Hz, 1H), 7.55-7.36 (m, 2H), 7. 34-7. 11 (m, 1H), 6.81 (s, 1H), 5.61-5.40 (m, 1H), 5. 39-5. 20 (m, 1H), 4. 50-4. 20 (m, 1H), 4.05-3.85 (m, 1H).

**Example 106: Compound 106: (Z)-3-(4-chloro-3,5-difluorobenzyl)-5-((6-chloro-7-fluoro-1H-indol-3-yl)methylene)imidazolidine-2,4-dione**

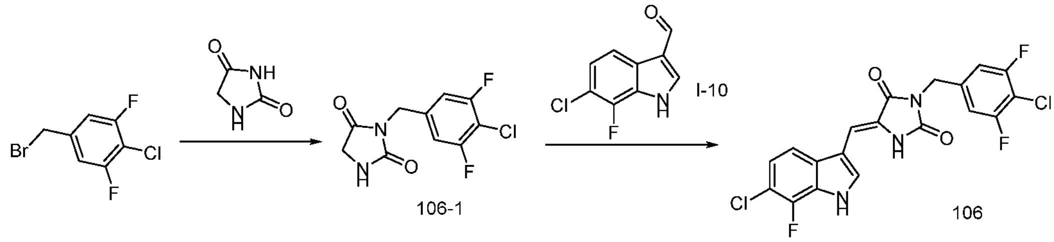

**106-1: 3-(4-chloro-3,5-difluorobenzyl)imidazolidine-2,4-dione**

By using the method for the compound I-1, the white solid compound **106-**1 (1.82 g, 83.2%) was prepared by using the hydantoin and 5-(bromomethyl)-2-chloro-1, 3-difluorbenzene.
LC-MS m/z (ESI): 259.0[M-H]$^-$.

**Compound 106: (Z)-3-(4-chloro-3,5-difluorobenzyl)-5-((6-chloro-7-fluoro-1H-indol-3-yl)methylene)imidazolidine-2,4-dione**

By using the method for the compound **1,** the gray solid compound **106** (38.0 mg, 16.5%) was prepared by using the compounds **106-1** and **I-10.**
LC-MS m/z (ESI): 438. 0[M-H]$^-$ . $^1$H NMR (400 MHz, DMSO-$d_6$) δ 12.55 (s, 1H), 10.59 (s, 1H), 8.27 (s, 1H), 7.68 (d, $J$ = 8.6 Hz, 1H), 7.40-7.29 (m, 2H), 7.28-7.10 (m, 1 H), 6.86 (s, 1H), 4.72 (s, 2H).

**Example 107: Compound 107: (Z)-3-(1-(4-chloro-3,5-difluorophenyl)-2-hydroxyethyl)-5-((6-chloro-7-fluoro-1H-indol-3-yl)methylene)imidazolidine-2,4-dione**

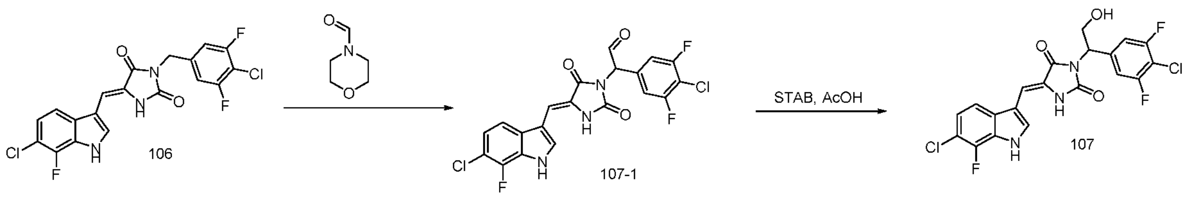

**107-1: (Z)-2-(4-chloro-3,5-difluorophenyl)-2-(4-((6-chloro-7-fluoro-1H-indol-3-yl)methylene)-2,5-dioxoimidazolidin-1-yl)acetaldehyde**

By using the method for the compound **33,** the brown solid compound **107-1** (180 mg, 34.02%) was prepared by using the compound **106.**
LC-MS m/z (ESI): 466.0[M-H]$^-$.

**Compound 107: (Z)-3-(1-(4-chloro-3,5-difluorophenyl)-2-hydroxyethyl)-5-((6-chloro-7-fluoro-1H-indol-3-yl) methylene)imidazolidine-2,4-dione**

By using the method for the compound **37,** the yellow solid compound **107** (31.1 mg, 24. 7%) was prepared by using the compound **107-1.**
LC-MS m/z (ESI) : 468. 1[M-H]$^-$ . $^1$H NMR (300 MHz, DMSO-$d_6$) δ 11. 90-10. 50 (br, 1H), 8.26 (s, 1H), 7.67 (d, J = 8.6 Hz, 1H), 7. 45-7. 35 (m, 2H), 7. 30-7. 16 (m, 1H), 6. 82 (s, 1H), 5.45- 5. 17 (m, 2H), 4. 4- 4. 12 (m, 1H), 4. 10-3. 85 (m, 1H)

**Example 108: Compound 108: (Z)-5-((6-chloro-7-fluoro-1H-indol-3-yl)methylene)-3-(1-(4-chlorophenyl)-3-hydroxypropyl)imidazolidine-2,4-dione**

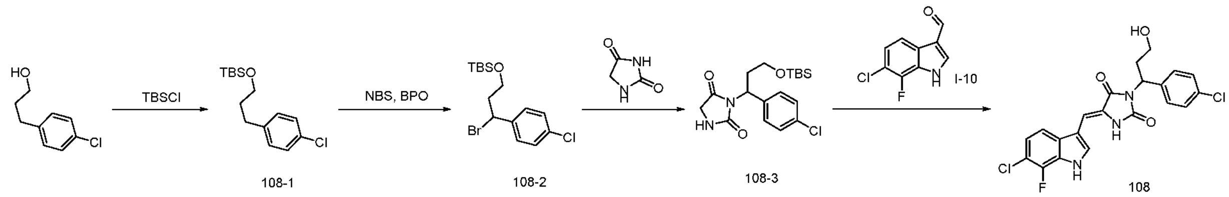


**108-1: tert-butyl (3-(4-chlorophenyl)propoxyl)dimethylsilane**

At room temperature and in a nitrogen atmosphere, TEA (8.9 g, 87.95 mmol, 3.00 eq) was added in batches into a stirred solution of 3-(4-chlorophenyl)-1-propanol (5 g, 29.30 mmol, 1.00 eq) and DMAP (0.36 g, 2.95 mmol, 0.10 eq) in DCM (50 mL), and the obtained mixture was stirred in the nitrogen atmosphere at room temperature for 16 h. The obtained mixture was concentrated at a reduced pressure. A residue was purified by silica gel column chromatography and eluted with PE/EA (10:1) to obtain the colorless liquid compound **108-1** (6.6 g, 78.5%).

**108-2: (3-bromo-3-(4-chlorophenyl)propoxyl)(tert-butyl)dimethylsilane**

At room temperature, a benzoylphenyl carbon peroxide (168 mg, 0.70 mmol, 0.03 eq) was added into a stirred mixture of the **108-1** (6.6 g, 23.17 mmol, 1.00 eq) and NBS (4.12 g, 23.15 mmol, 1.00 eq) in $CCl_4$ (120 mL), and the obtained mixture was stirred for 3 h. The mixture was cooled to room temperature. The obtained mixture was filtered. A filter cake was washed with $CCl_4$ (2x20 mL). A filtrate was concentrated at a reduced pressure. A residue was purified by silica gel column chromatography and eluted with PE/EA (15:1) to obtain the light brown liquid compound **108-2** (2.7 g, 25.8%).

**108-3: 3-(3-((tert-butyldimethylsilyl)oxyl)-1-(4-chlorophenyl)propyl)imidazolidine-2,4-dione**

At room temperature, the **108-2** (2. 7 g, 7.42 mmol, 1.00 eq) was added into a mixture of hydantoin (2.23 g, 22.28 mmol, 3.00 eq) and K2C03 (1.54 g, 11. 13 mmol, 1. 50 eq) in DMF (54 mL). In a nitrogen atmosphere, the obtained mixture was stirred at 60° C for 16 h. The mixture was cooled to room temperature. A cold saturated ammonium chloride aqueous solution (200 mL) was added to terminate the reaction. The obtained mixture was extracted with ethyl acetate (3×150 mL). Combined organic layers were washed with saline (2×100 mL) and dried with anhydrous $Na_2SO_4$. After the organic layers were filtered, a filtrate was concentrated at a reduced pressure. A residue was purified by fast reversed-phase chromatography to obtain the light yellow syrupy compound **108-3** (1.5 g, 47.0%).
LC-MS m/z (ESI): 383.1[M+H]$^+$.

**Compound 108: (Z)-5-((6-chloro-7-fluoro-1H-indol-3-yl)methylene)-3-(1-(4-chlorophenyl)-3-hydroxypropyl)imidazolidine-2,4-dione**

At room temperature, HOAc (122 mg, 2.03 mmol, 5.00 eq) was dropwise added into a stirred solution of 108-3 (186 mg, 0.49 mmol, 1.20 eq) and 6-chloro-7-fluoro-1H-indole-3-formaldehyde (80 mg, 0.41 mmol, 1.00 eq) in ethanol (10 mL). In an argon atmosphere, the obtained mixture was stirred at 100° C for 16 min and cooled to room temperature. The obtained mixture was concentrated at a reduced pressure. A residue was purified by silica gel column chromatography and eluted with PE/EA (3:1) to obtain a brown syrupy crude product. The residue was dissolved in methanol (2 mL). A methanol solution (0. 51 mL, 2.04 mmol, 5.04 eq) was dropwise added into the mixture at room temperature. The obtained mixture was then stirred at room temperature for 2 h, and the obtained mixture was concentrated in vacuum. The crude product (50 mg) was purified by HPLC to obtain the yellow solid compound **108** (12.5 mg, 6.11%).

LC-MS m/z (ESI): 446.0[M-H]$^-$ . $^1$H NMR (400 MHz, DMSO-$d_6$) δ 8.23 (s, 1H), 7.65 (d, J = 8.6 Hz, 1H), 7.51-7.40 (m, 4H), 7.30-7.16 (m, 1H), 6.79 (s, 1H), 5.50-5.22 (m, J = 9.4, 6.4 Hz, 1H), 4.64 (s, 1H), 3.46-3.42 (m, 2H), 2.71- 2. 56 (m, 1H), 2.34-2.22(m, 1H) .

**Example 109: compound 109: (Z)-5-((6-chloro-7-fluoro-1H-indol-3-yl)methylene)-3-(1-(4-chlorophenyl)-4-hydroxybutyl)imidazolidine-2,4-dione**

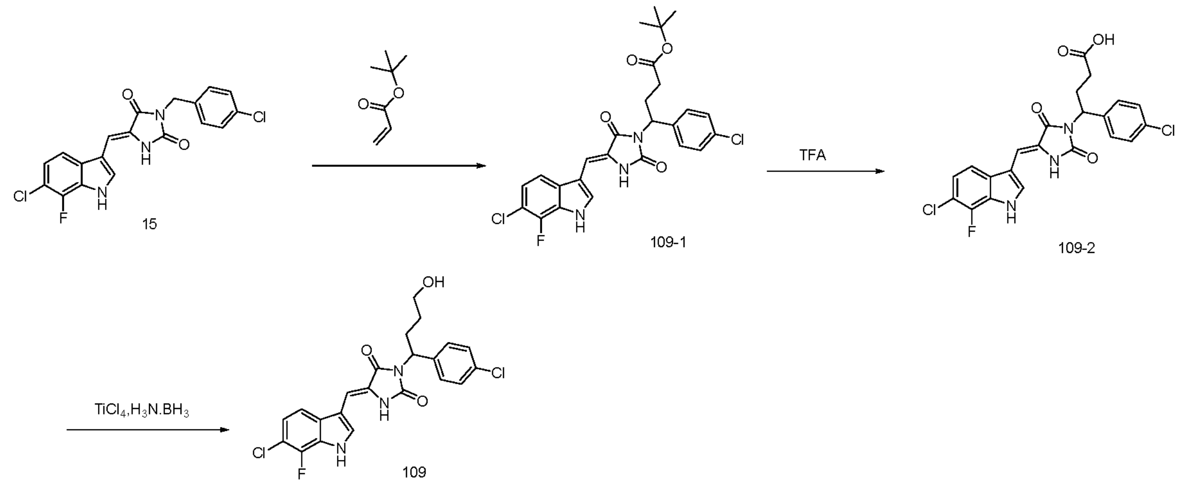

15 109-1 109-2 109

**109-1: tert-butyl (Z)-4-(4-((6-chloro-7-fluoro-1H-indol-3-yl)methylene)-2,5-dioxoimidazolidin-1-yl)-4-(4-chlorophenyl)butyrate**

At -78° C and in an argon atmosphere, n-BuLi (4.2 mL, 10.50 mmol, 10. 11 eq) was dropwise added into a stirred solution of the compound 15 (420 mg, 1.04 mmol, 1.00 eq) and tert-butyl 2-acrylate (2.66 g, 20.78 mmol, 20.00 eq) in THF (140 mL). In the argon atmosphere, the obtained mixture was stirred at -78° C for 1 h. At -82° C, the reaction was quenched with a solution of THF in acetic acid. The mixture was heated to -30° C and the obtained mixture was poured into 300 mL of water. The obtained mixture was extracted with ethyl acetate (1×300 mL). Combined organic layers was washed with saline (2×150 mL) and dried with anhydrous $Na_2SO_4$. After the organic layers were filtered, a filtrate was concentrated at a reduced pressure. A residue was purified by fast reversed-phase chromatography to obtain the light yellow solid compound **109-1** (305 mg, 53.2%).

LC-MS m/z (ESI): 530.2[M-H]$^-$.

**109-2: (Z)-4-(4-((6-chloro-7-fluoro-1H-indol-3-yl)methylene)-2,5-dioxoimidazolidin-1-yl)-4-(4-chlorophenyl)butyric acid**

At room temperature, TFA (3 mL) was dropwise added into a stirred solution of **109-1** (300 mg, 0.56 mmol, 1.00 eq) in DCM (20 mL), and the obtained mixture was stirred at room temperature for 2 h. The obtained mixture was concentrated in vacuum. A residue was purified by fast reversed-phase chromatography to obtain the yellow solid compound **109-2** (135 mg, 49.8%).

LC-MS m/z (ESI): 476.1[M+H]$^+$.

**Example 109: Compound 109: (Z)-5-((6-chloro-7-fluoro-1H-indol-3-yl)methylene)-3-(1-(4-chlorophenyl)-4-hydroxybutyl)imidazolidine-2,4-dione**

At room temperature and in a nitrogen atmosphere, $TiCl_4$ (8.4 mg, 0.04 mmol, 0.20 eq) was dropwise added into a mixture of 109-2 (105 mg, 0.22 mmol, 1.00 eq) and ammonia borane (13.7 mg, 0.44 mmol, 2.01 eq) in ether (2.8 mL), and the obtained mixture was stirred in the nitrogen atmosphere at room temperature for 2 h. A saturated ammonium chloride (20 mL) solution was added at room temperature to terminate the reaction. The obtained mixture was extracted with ethyl acetate (4×50 mL). Combined organic layers were washed with saline (2×50 mL) and dried with anhydrous $Na_2SO_4$. After the organic layers were filtered, a filtrate was concentrated at a reduced pressure. A crude product was purified by HPLC to obtain the yellow solid compound **109** (50.6 mg, 48.9%).

LC-MS m/z (ESI): 460. 0[M-H]$^-$ . $^1$H NMR (400 MHz, DMSO-$d_6$) δ 12.04 br, 1H), 10.63 (br, 1H), 8.24 (s, 1H), 7.66 (d, $J$ = 8.6 Hz, 1H), 7.55-7.35 (m, 4H), 7. 30-7. 10 (m, 1H), 6.81 (s, 1H), 5. 22-5. 10 (m, 1H), 4. 55-4. 31 (m, 1H), 3.5-3.41 (m, 2H), 2.49-2.37 (m, 1H), 2.30- 2.10 (m, 1H), 1. 51-1. 30 (m, 2H)

**Compound 110: (Z)-3-(4-((6-chloro-7-fluoro-1H-indol-3-yl)methylene)-2,5-dioxoimidazolidin-1-yl)-3-(4-chlorophenyl)propyl dihydrophosphate**

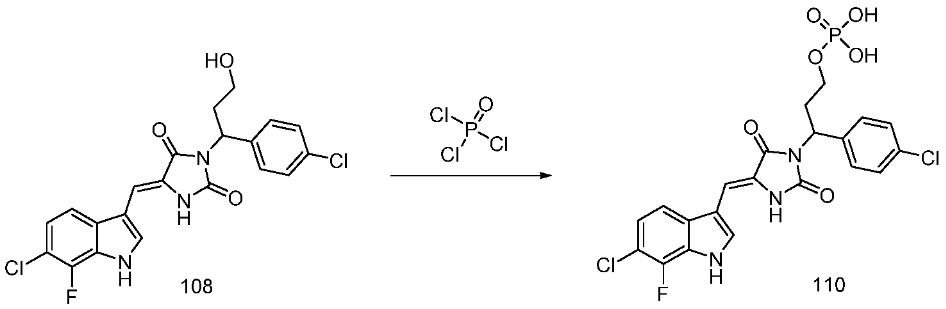

At 0° C and in a nitrogen atmosphere, phosphoryl chloride (513 mg, 3.35 mmol, 10.00 eq) was added into a stirred solution of the compound **108** (150 mg, 0.34 mmol, 1.00 eq) in THF (9 mL). In the nitrogen atmosphere, the obtained mixture was stirred at room temperature for 7 h. The reaction was quenched with a saturated $NaHCO_3$ solution at room temperature, and the obtained mixture was stirred for 30 min. The mixture was acidized with HCl (aqueous solution) till pH=2 and extracted with EtOAc(3×50 mL). Combined organic layers were washed with saline (2×50 mL) and dried with anhydrous $Na_2SO_4$. After the organic layers were filtered, a filtrate was concentrated at a reduced pressure. A residue was purified by fast reversed-phase chromatography to obtain the yellow solid compound **110** (39.2 mg, 21.9%).

LC-MS m/z (ESI): 528.0[M+H]$^+$ . $^1$H NMR (300 MHz, DMSO-$d_6$)) δ 8.24 (s, 1H), 7.63 (d, $J$ = 8.6 Hz, 1H), 7. 51-7. 35 (m, 4H), 7.25-7. 11 (m, 1H), 6.79 (s, 1H), 5.45-5.21 (m, 1H), 3.70-3.60 (m, 2H), 2. 70 - 2.51 (m, 1H), 2.45-2.40(m, 1H).

**Test example 1: activity determination for compounds**

**Test of candidate compounds on growth inhibition of cells determined by CellTiter Glo**

Candidate compounds were dissolved in DMSO, with an initial concentration of 50 mM, and were diluted to a 384-well storage plate at a triple gradient. The compounds in the storage plate were pumped into a 96-well cell culture plate (Corning 3610) with an Echo 520 instrument, with a volume of 100 nL/well, and were diluted at a triple gradient in duplicate. Blood cancer cells RS4;11 were inoculated into the 96-well cell culture plate through Multidrop 3000 cells per well, with a volume of 100 μL. The final compound concentration ranged from 50 μM to 0.01 μM at 9 concentration gradients and the DMSO final concentration was 0.1%. The cells and compounds were co-incubated in a cell incubator at 37° C for 72 h, a CellTiter-Glo Luminescent Cell Viability Assay reagent was added 100 μL/ well, the cells and compound were uniformly mixed and incubated at room temperature for 10 min, and Envision reading was performed. A sample treated with 50 μM of a positive compound was taken as a positive control and a sample treated with 100 nL of DMSO was taken as a negative control. A calculation formula was as follows: (sample reading-positive control reading)/(negative control reading-positive control reading)*100%, and $IC_{50}$ was calculated by a four-parameter formula. The cell viability $IC_{50}$ intervals of the compounds were as follows: A≤1 μM, 1<B≤10 μM, C>10 μM.

**HTRF test: test of respectively detecting inhibition of candidate compounds on MDM2-p53 and MDMX-p53 protein binding**

Candidate compounds were dissolved in DMSO, with an initial concentration of 50 mM, and were diluted to a 384-well storage plate at a triple gradient. The compounds in the storage plate were pumped into a 384-well cell culture plate (PE 6008280) with an Echo 520 instrument, with a volume of 100 nL/well, and were diluted at a triple gradient in duplicate. Proteins and/or polypeptides were respectively diluted with HTRT buffer solutions (1X PBS pH 7.4, 0.1% BSA and 1 mM DTT) as follows: An MDM2-p53 reaction system was purified 10 nM GST-MDM2 (2-138 aa) and 5 nM HIS-p53 (2-312 aa) proteins; an MDMX reaction system was 5 nM GST-MDMX (2-134 aa) proteins and 100 nM WT HIS-p53 peptide fragments (Rui Biotech). Proteins in a negative control group in the MDM2-p53 binding inhibition test were 10 nM GST proteins and 5 nM HIS-p53 (2-312 aa) proteins, proteins in a negative control group in the MDMX-p53 binding inhibition test were 10 nM GST proteins and 100 nM WT HIS-p53 peptide fragments, and positive control groups in the above two binding tests were DMSO treated groups. 10 μL of the above proteins and/or polypeptide reaction system were respectively added into the 384-well plate with the added compounds through multidrop. The final compound concentration ranged from 250 μM to 0.05 nM, they were diluted at a triple gradient, with 15 concentrations in duplicate. The DMSO final concentration was 1%, and they were incubated at room temperature for 1 h. Anti 6HIS-Tb cryptate Gold (Cisbio 61HI2TLF) and Anti GST-XL665 (Cisbio 61GSTXLF) diluted at 400 times were added into the above buffer solution, and they were uniformly mixed. The antibody mixed solution was added into the 384-well plate through multidrop 10 μL/well, and was incubated at room temperature for 1 h. Data was read by Envision. The wavelength of laser-excited light was 340 nM, readings with the wavelengths of emitted light of 665 nM and 615 nM were respectively read, and a 665/615 ratio was calculated. A known MDM2-p53 binding inhibitor Siremadlin (NVP-HDM201; MCE HY-18658) was the positive control group. 100 nL of DMSO treated group was the negative control group. A calculation formula was as follows: (sample reading-positive control reading)/(negative control reading-positive control reading)*100%, and $IC_{50}$ was calculated by a four-parameter formula. The protein binding $IC_{50}$ intervals of the compounds were as follows: A≤10 nM; 10<B≤100 nM; C>100 nM.

| Examples | HTRF test | | CellTiter Glo test |
|---|---|---|---|
| | MDM2 $IC_{50}$ (nM) | MDMX $IC_{50}$ (nM) | RS4;11 $IC_{50}$ (μM) |
| 1 | B | B | B |
| 2 | B | B | A |
| 3 | B | B | A |
| 4 | B | B | A |
| 5 | B | B | B |
| 6 | B | B | / |
| 7 | B | B | / |
| 8 | B | B | B |
| 9 | B | B | C |
| 10 | B | B | B |
| 11 | A | B | B |
| 12 | B | B | C |
| 13 | A | B | C |
| 14 | A | A | A |
| 15 | B | B | A |
| 16 | A | A | A |
| 17 | B | B | A |
| 18 | B | B | A |
| 19 | A | A | A |
| 20 | B | B | A |

(continued)

| Examples | HTRF test | | CellTiter Glo test |
|---|---|---|---|
| | MDM2 $IC_{50}$ (nM) | MDMX $IC_{50}$ (nM) | RS4;11 $IC_{50}$ (μM) |
| 21 | B | A | A |
| 22 | A | B | / |
| 23 | B | B | / |
| 24 | B | B | B |
| 25 | B | B | / |
| 26 | A | A | A |
| 27 | A | A | A |
| 28 | A | A | A |
| 29 | A | A | B |
| 30 | A | A | A |
| 31 | B | B | B |
| 32 | A | A | B |
| 33 | B | B | B |
| 34 | A | B | A |
| 35 | B | B | A |
| 36 | A | B | A |
| 37 | B | B | A |
| 38 | A | B | A |
| 39 | B | B | B |
| 40 | B | C | / |
| 41 | A | A | A |
| 43 | B | B | / |
| 44 | A | A | / |
| 45 | B | B | A |
| 48 | A | A | A |
| 49 | A | B | A |
| 50 | B | C | A |
| 53 | A | A | B |
| 54 | B | B | A |
| 56 | B | B | B |
| 57 | A | A | A |
| 58 | A | A | A |
| 59 | A | B | A |
| 60 | A | A | A |
| 61 | A | B | B |
| 62 | A | B | A |
| 64 | A | A | A |
| 66 | A | A | A |

(continued)

| Examples | HTRF test | | CellTiter Glo test |
|---|---|---|---|
| | MDM2 $IC_{50}$ (nM) | MDMX $IC_{50}$ (nM) | RS4;11 $IC_{50}$ (μM) |
| 67 | A | A | A |
| 68 | A | A | A |
| 69 | B | B | A |
| 70 | B | B | A |
| 72 | A | A | A |
| 74 | C | B | / |
| 77 | B | B | A |
| 78 | B | B | A |
| 79 | B | B | A |
| 84 | A | A | A |
| 85 | A | A | A |
| 86 | B | B | A |
| 88 | A | A | A |
| 92 | B | B | / |
| 94 | A | A | / |
| 95 | B | B | / |
| 96 | A | A | A |
| 97 | B | B | A |
| 98 | B | C | A |
| 99 | B | B | A |
| 100 | A | B | A |
| 101 | B | C | B |
| 102 | B | B | A |
| 103 | A | C | B |
| 104 | B | B | A |
| 105 | B | B | A |
| 106 | B | B | A |
| 107 | B | B | A |
| 108 | / | / | A |
| 109 | / | / | A |

**Test example 2: solubility test**

1) Preparation of a stock solution

A test substance and a control drug progesterone were prepared with DMSO into a 10 mM stock solution

2) Steps of determining solubility

30 μL 10 mM stock solution prepared from the test substance and the control drug progesterone was taken and

added into bottles in corresponding positions of 96-well sample plate in a specified order. Then, 970 μL of phosphate buffer with pH 7.4 was added into corresponding bottles of the sample plate. The experiments were two parallel experiments. A stirring rod was added into each bottle, and each bottle was covered with a polytetrafluoroethylene/silicon organic resin bottle plug. Then, a sample tray was placed in Eppendorf Thermomixer Comfort and vibrated at a rotating speed of 1100/min at 25° C for 2 h. 2 h later, the bottle plug was removed, the stirring rod was taken away with a large magnet, and then samples were transferred to a filter plate from the sample plate. A vacuum pump generated a negative pressure to filter the samples. 5 μL of a filtrate was transferred to a new sample plate. Then, 5 μL of DMSO and 490 μL of acetonitrile/water (1:1 in v/v) were added. The dilution ratio might be adjusted according to the magnitude of the solubility of the test substance or the intensity of a liquid response signal thereof.

3) Preparation of a standard product solution

15 μL of the stock solution was transferred from 10 mM DMSO stock solution to an empty plate, and 485 μL of DMSO was added to prepare a 300 μM standard product solution. 5 μL of the standard product solution was transferred from a plate of the 300 μM standard product solution to another empty plate, and then 5 μL of a phosphate buffer with pH 7.4 and 490 μL of acetonitrile/water (1:1 in v/v) were added to obtain a standard product solution with the concentration of 3 μM. The concentration of the standard product solution might be adjusted according to the intensity of the liquid response signal thereof.

4) Sample analysis step

A sample injection plate was placed in a sample injection tray of an automatic sample injection, and samples were evaluated through liquid analysis.

5) Data analysis

All calculations were performed through Microsoft Excel.

Analysis and quantification of the filter of the samples were completed by qualitatively and quantitatively analyzing the peak of a standard product with known concentration through liquid chromatography-mass spectrometry. A calculation formula for solubility values of the control drug and test substance was as follows:

$$[\text{Sample}] = \frac{\text{AREA}_{\text{Sample}} \times \text{DF}_{\text{Sample}} \times [\text{STD}]}{AREA_{Std}}$$

DF refers to the dilution ratio.

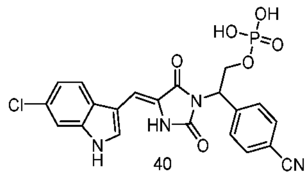
40

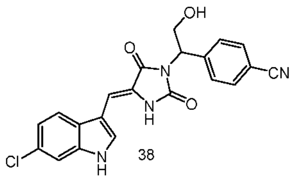
38

| Compound | Solubility (μg/mL) |
|---|---|
| Compound 38 | 0. 075 |
| Compound 40 | 145. 62 |

The embodiments of the technical solutions of the invention are exemplarily described above. It should be understood that the protection scope of the invention is not limited to the above embodiments. Any modification, equivalent replacement, improvement, etc. made by persons skilled in the art within the spirit and principle of this application should fall within the scope of claims of this application.

## Claims

1. A compound shown in formula (I), and a raceme, a stereoisomer, a tautomer, an isotopic label, a solvate, a polymorph, a pharmaceutically acceptable salt or a prodrug compound thereof:

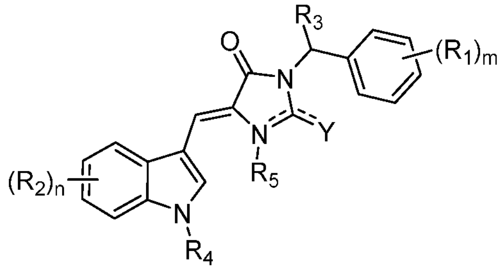

(I)

wherein

$R_1$ is selected from H, halogen, CN, $C_{1-6}$ alkyl, $C_{2-6}$ alkenyl, $C_{2-6}$ alkynyl, $C_{1-6}$ alkoxyl, halogenated $C_{1-6}$ alkyl, and halogenated $C_{1-6}$ alkoxyl;
$R_2$ is selected from H, $C_{1-6}$ alkyl, halogen, $C_{1-6}$ alkoxyl, $C_{1-6}$ alkylthio, halogenated $C_{1-6}$ alkyl, and halogenated $C_{1-6}$ alkoxyl;
$R_3$ is selected from H, $R_{3a}$-O-$C_{1-6}$ alkyl, -CH(=O), and -C(=O)-NH-$R_{3b}$; $R_{3a}$ is selected from H or -P(=0) ($OR_{31}$) ($OR_{32}$); $R_{3b}$ is selected from the following radicals which are unsubstituted or optionally substituted with one, two, three or more $R_{3c}$: $C_{1-6}$ alkyl, $C_{3-8}$ cycloalkyl, and $C_{3-8}$ cycloalkyl-$C_{1-6}$ alkyl; $R_{3c}$ is selected from OH, $C_{1-6}$ alkyl-NH-, ($C_{1-6}$ alkyl)$_2$-N-, and -O-P(=O)($OR_{31}$)($OR_{32}$); and $R_{31}$ and $R_{32}$ are same or different, and are independently selected from H and $C_{1-6}$ alkyl;
$R_4$ is selected from H and -P(=0) ($OR_{41}$)($OR_{42}$) ; and $R_{41}$ and $R_{42}$ are same or different, and are independently selected from H and $C_{1-6}$ alkyl;
$R_5$ is selected from H and -P(=O) ($OR_{51}$) ($OR_{52}$); and $R_{51}$ and $R_{52}$ are same or different, and are independently selected from H and $C_{1-6}$ alkyl;
Y is 0 or $OR_6$; $R_6$ is selected from H and -P(=0) ($OR_{61}$) ($OR_{62}$) ; and $R_{61}$ and $R_{62}$ are same or different, and are independently selected from H and $C_{1-6}$ alkyl;

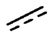 is selected from a single bond or a double bond;
m is selected from 0, 1, 2, 3, 4 or 5; and
n is selected from 0, 1, 2, 3 or 4.

2. The compound, and the raceme, the stereoisomer, the tautomer, the isotopic label, the solvate, the polymorph, the pharmaceutically acceptable salt or the prodrug compound thereof according to claim 1, **characterized in that** $R_1$ is selected from H, halogen, CN, $C_{1-3}$ alkyl, $C_{2-3}$ alkynyl, $C_{1-3}$ alkoxyl, halogenated $C_{1-3}$ alkyl, and halogenated $C_{1-3}$alkoxyl;

preferably, $R_1$ is selected from H, F, Cl, CN, acetenyl, methyl, ethyl, and propyl;
preferably, $R_2$ is selected from H, $C_{1-3}$ alkyl, halogen, $C_{1-3}$ alkoxyl, $C_{1-3}$ alkylthio, and halogenated $C_{1-3}$ alkoxyl; and
preferably, $R_2$ is selected from H, F, Cl, Br, methyl, methoxyl, fluoromethoxyl, and methylthio.

3. The compound, and the raceme, the stereoisomer, the tautomer, the isotopic label, the solvate, the polymorph, the pharmaceutically acceptable salt or the prodrug compound thereof according to claim 1 or 2, **characterized in that** $R_3$ is selected from H, $R_{3a}$-O-$C_{1-3}$ alkyl, -CH(=O), and -C(=O)-NH-$R_{3b}$; $R_{3a}$ is selected from H or -P(=O) (OH) (OH); $R_{3b}$ is selected from the following radicals which are unsubstituted or optionally substituted with one, two, three or more $R_{3c}$: $C_{1-3}$ alkyl, $C_{3-8}$ cycloalkyl, and $C_{3-8}$ cycloalkyl-$C_{1-3}$ alkyl; and $R_{3c}$ is selected from OH, $C_{1-3}$ alkyl-NH-, ($C_{1-3}$ alkyl)$_2$-N-, and -O-P(=O)(OH)(OH) ;
preferably, $R_3$ is selected from H, methyl,

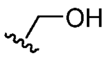, 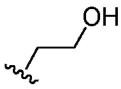, 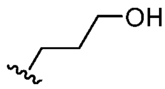,

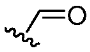, 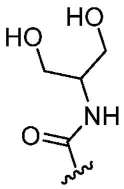, 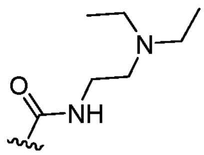, 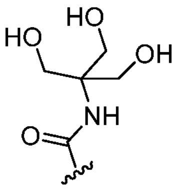, 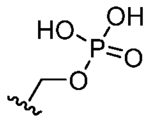, 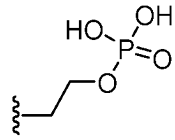,

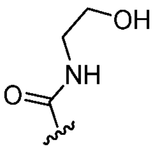, 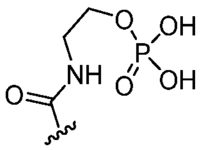, 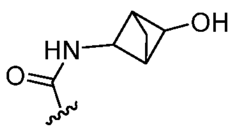, 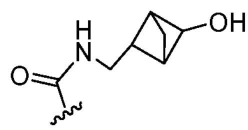,

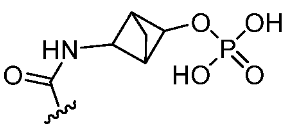, 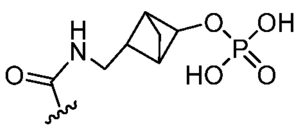, 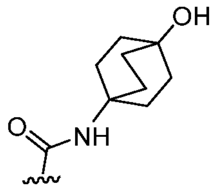, 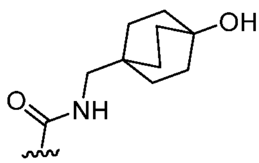,

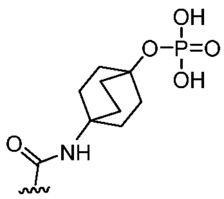, 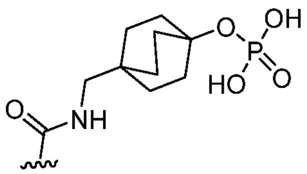, 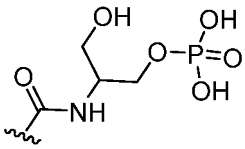, and 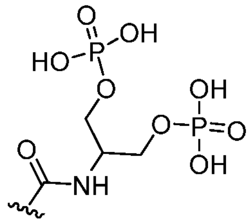;

for example, selected from

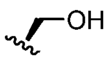, 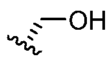, 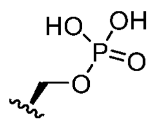, and 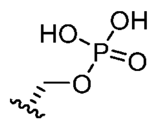.

**4.** The compound, and the raceme, the stereoisomer, the tautomer, the isotopic label, the solvate, the polymorph, the pharmaceutically acceptable salt or the prodrug compound thereof according to any one of claims 1 to 3, **characterized in that** $R_4$ is selected from H and -P(=0) $(OR_{41})$ $(OR_{41})$ ; $R_{41}$ and $R_{42}$ are same or different, and are independently selected from H and $C_{1-3}$ alkyl;

preferably, $R_4$ is selected from H and -P(=0) (OH) (OH) ;
preferably, $R_5$ is selected from H and -P(=O)$(OR_{51})(OR_{52})$; and $R_{51}$ and $R_{52}$ are same or different, and are independently selected from H and $C_{1-3}$ alkyl;
preferably, $R_5$ is selected from H and -P(=0)(OH)(OH) ;
preferably, Y is 0 or $OR_6$; $R_6$ is selected from H and -P(=O)$(OR_{61})(OR_{62})$ ; and $R_{61}$ and $R_{62}$ are same or different, and are independently selected from H and $C_{1-3}$ alkyl;
preferably, $R_6$ is selected from H and -P(=0)(OH)(OH) ;
preferably, m is selected from 1, 2 or 3; and
preferably, n is selected from 1 or 2.

**5.** The compound, and the raceme, the stereoisomer, the tautomer, the isotopic label, the solvate, the polymorph, the

pharmaceutically acceptable salt or the prodrug compound thereof according to any one of claims 1 to 4, **characterized in that** the prodrug compound has a structure shown in formula (II):

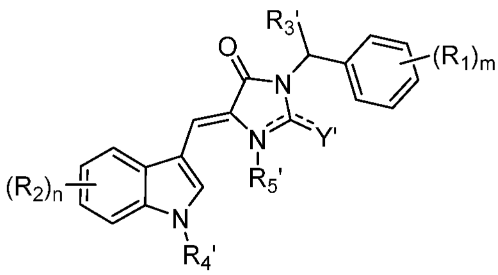

(II)

wherein $R_1$, $R_2$, m and n independently have definitions according to any one of claims 1 to 4;
$R_3$' has a definition of $R_3$ according to any one of claims 1 to 4;
$R_4$' is selected from H and -P(=O)($OR_{41}$') ($OR_{42}$'); $R_{41}$' and $R_{42}$' are same or different, and are independently selected from H and $C_{1-3}$ alkyl; preferably, $R_4$' is selected from H and -P(=0) ($OR_{41}$' ) ($OR_{42}$' ); and $R_{41}$' and $R_{42}$' are same or different, and are independently selected from H and $C_{1-6}$ alkyl;
$R_5$' is selected from H and -P(=O)($OR_{51}$' ) ($OR_{52}$' ); $R_{51}$' and $R_{52}$' are same or different, and are independently selected from H and $C_{1-3}$ alkyl; preferably, $R_5$' is selected from H and -P(=0) ($OR_{51}$' ) ($OR_{52}$' ); and $R_{51}$' and $R_{52}$' are same or different, and are independently selected from H and $C_{1-6}$ alkyl;
Y' is 0 or $OR_6$' ; $R_6$' is selected from H and -P(=0) ($OR_{61}$' ) ($OR_{62}$' ); and $R_{61}$' and $R_{62}$' are same or different, and are independently selected from H and $C_{1-3}$ alkyl; preferably, R6' is selected from H and -P(=0) ($OR_{61}$' ) ($OR_{62}$' ); and $R_{61}$' and $R_{62}$' are same or different, and are independently selected from H and $C_{1-6}$ alkyl;

is selected from a single bond or a double bond;
preferably, $R_3$' is selected from H, $R_{3a}$-O-$C_{1-6}$ alkyl, -CH(=0), and -C(=O)-NH-$R_{3b}$; $R_{3a}$ is selected from H or -P(=0)(OH)(OH) ; $R_{3b}$ is selected from the following radicals which are unsubstituted or optionally substituted with one, two, three or more $R_{3c}$ $C_{1-6}$ alkyl, $C_{3-8}$ cycloalkyl, and $C_{3-8}$ cycloalkyl-$C_{1-6}$ alkyl; $R_{3c}$ is selected from OH, $C_{1-6}$ alkyl-NH-, ($C_{1-6}$ alkyl)$_2$-N-, and -O-P(=O)(OH)(OH) ;
preferably, $R_4$' is selected from H and -P(=0) (OH)(OH) ;
preferably, $R_5$' is selected from H and -P(=O) (OH)(OH) ;
preferably, Y' is O or $OR_6$' ; and $R_6$' is selected from H and -P(=O) (OH)(OH) ;

is selected from a single bond or a double bond; and
at least one of $R_4$' , $R_5$' , $R_6$' , $R_{3a}$, and $R_{3c}$ is -P(=O)(OH)(OH).

**6.** The compound, and the raceme, the stereoisomer, the tautomer, the isotopic label, the solvate, the polymorph, the pharmaceutically acceptable salt or the prodrug compound thereof according to any one of claims 1 to 5, **characterized in that** the compound is selected from the following structures:

| Number | Structure | Number | Structure |
|---|---|---|---|
| 1 | 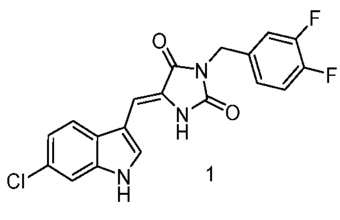 1 | 48 | 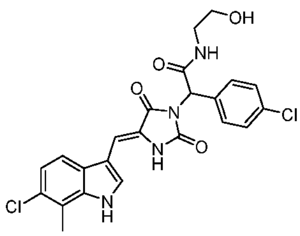 |
| 2 | 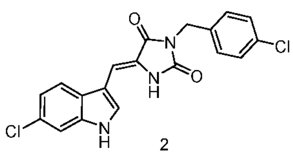 2 | 49 | 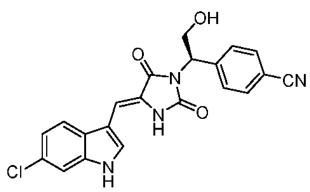 |

(continued)

| Number | Structure | Number | Structure |
|---|---|---|---|
| 3 | 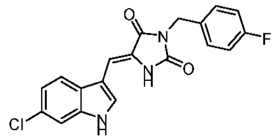 | 50 | 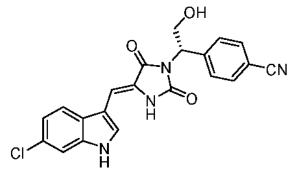 |
| 4 | 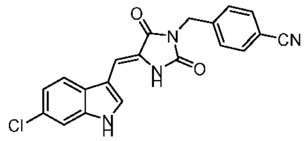 | 51 | 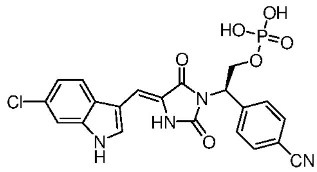 |
| 5 | 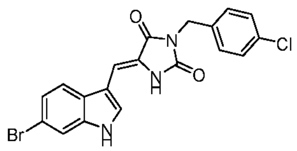 | 52 | 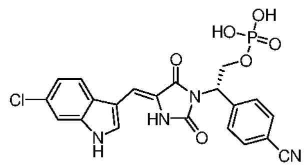 |
| 6 | 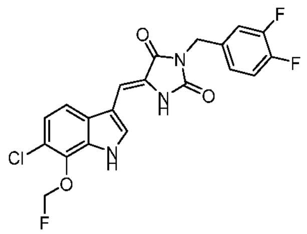 | 53 | 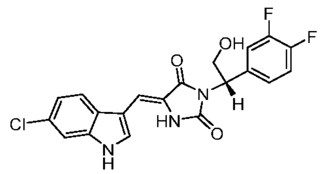 |
| 7 | 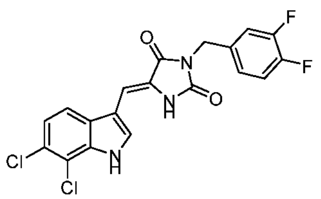 | 54 | 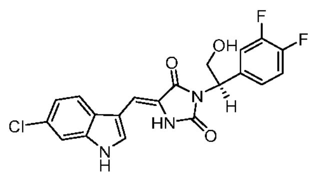 |
| 8 | 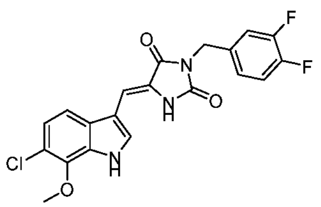 | 55 | 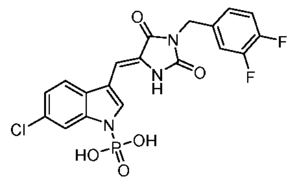 |
| 9 | 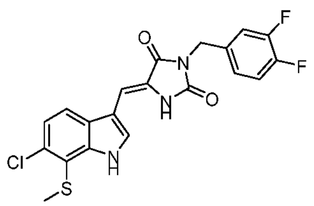 | 56 | 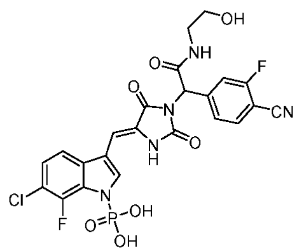 |

(continued)

| Number | Structure | Number | Structure |
|---|---|---|---|
| 10 | 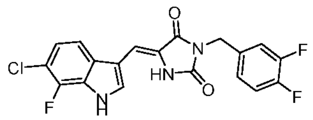 | 57 | 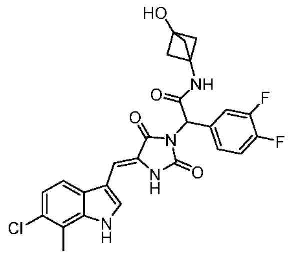 |
| 11 | 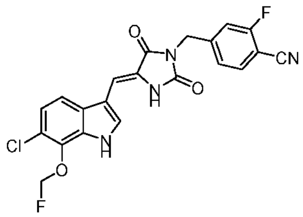 | 58 | 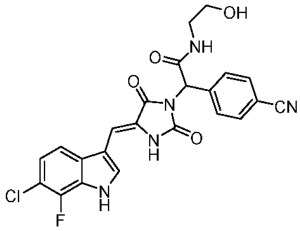 |
| 12 | 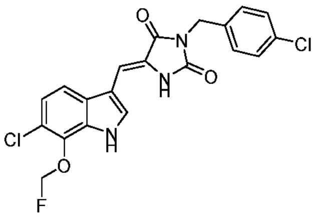 | 59 | 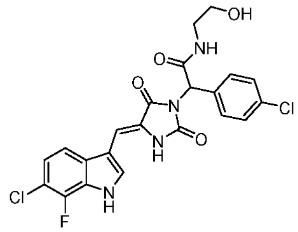 |
| 13 | 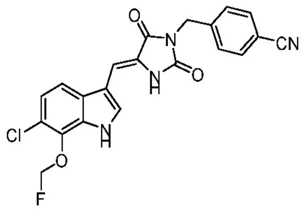 | 60 | 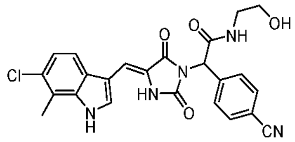 |
| 14 | 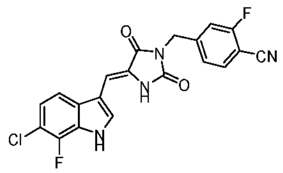 | 61 | 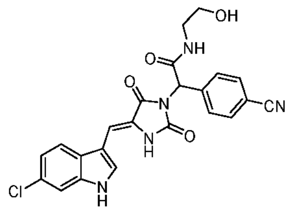 |
| 15 | 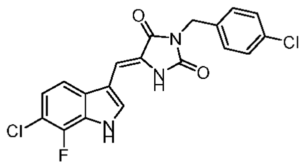 | 62 | 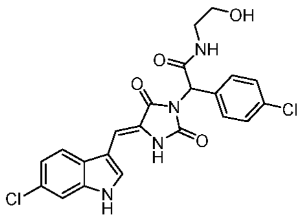 |
| 16 | 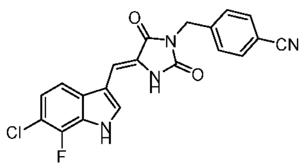 | 63 | 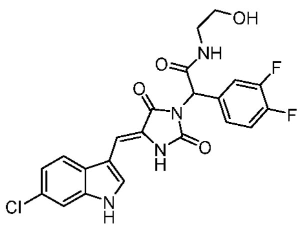 |

(continued)

| Number | Structure | Number | Structure |
|---|---|---|---|
| 17 | 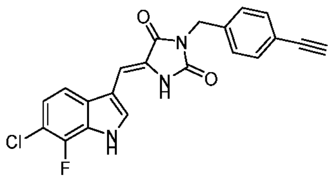 | 64 | 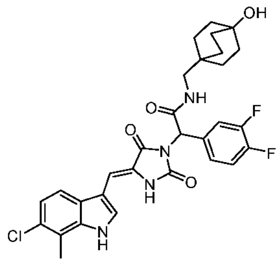 |
| 18 | 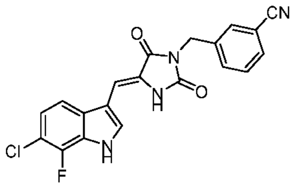 | 65 | 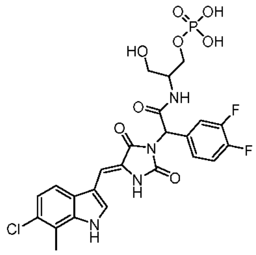 |
| 19 | 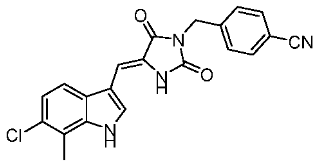 | 66 | 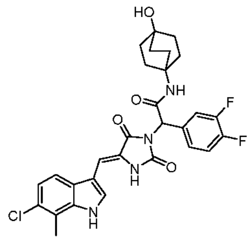 |
| 20 | 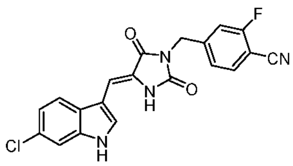 | 67 | 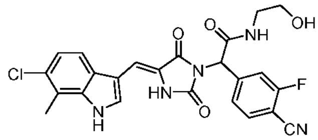 |
| 21 | 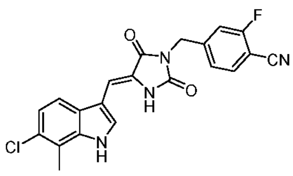 | 68 | 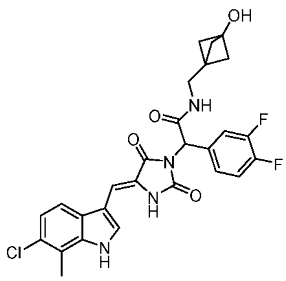 |
| 22 | 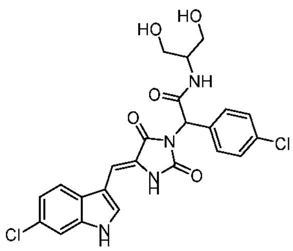 | 69 | 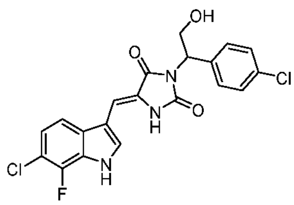 |

(continued)

| Number | Structure | Number | Structure |
|---|---|---|---|
| 23 | 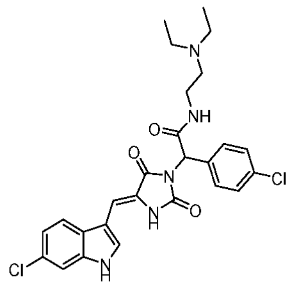 | 70 | 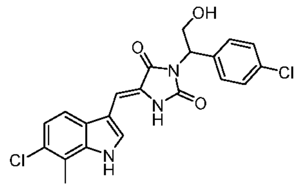 |
| 24 | 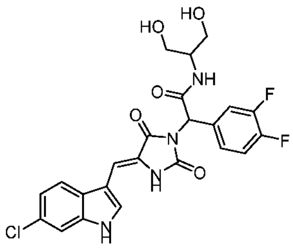 | 71 | 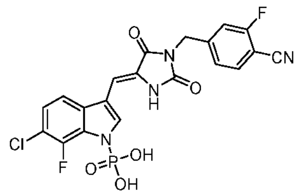 |
| 25 | 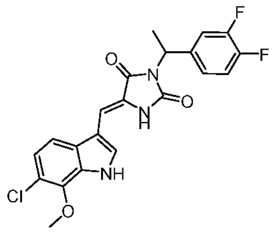 | 72 | 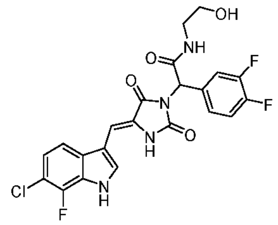 |
| 26 | 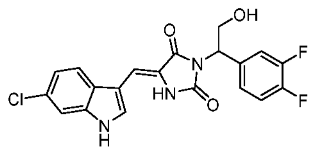 | 73 | 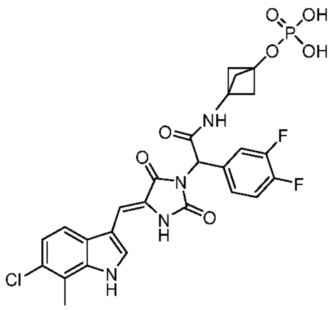 |
| 27 | 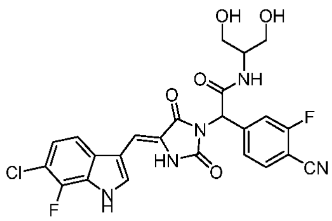 | 74 | 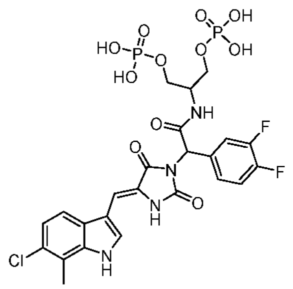 |

(continued)

| Number | Structure | Number | Structure |
|---|---|---|---|
| 28 | 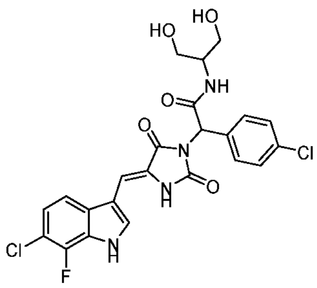 | 75 | 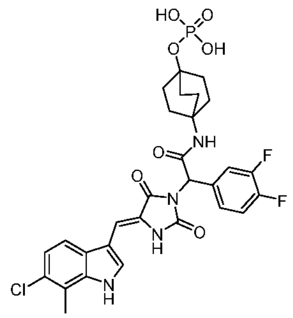 |
| 29 | 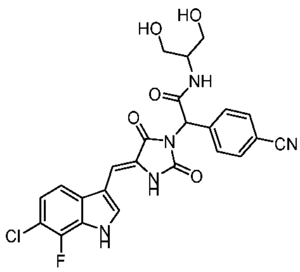 | 76 | 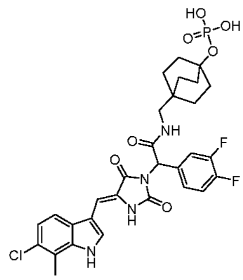 |
| 30 | 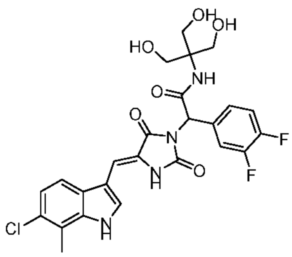 | 77 | 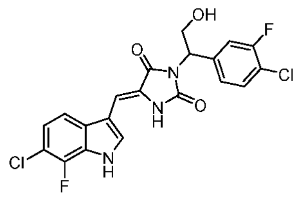 |
| 31 | 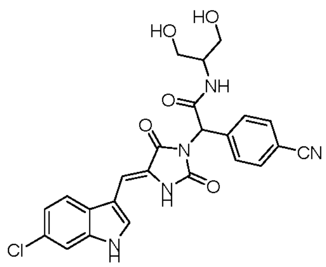 | 78 | 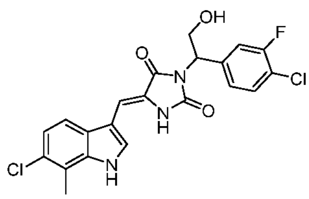 |
| 32 | 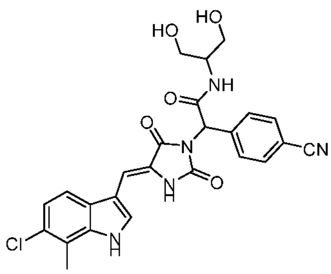 | 79 | 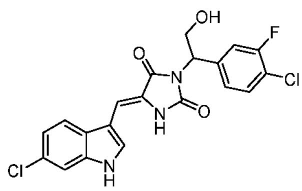 |
| 33 | 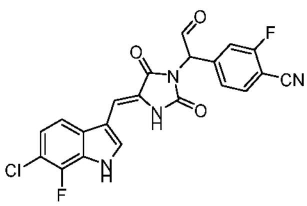 | 80 | 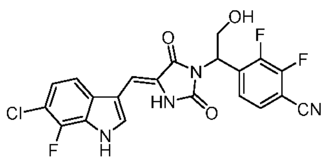 |

(continued)

| Number | Structure | Number | Structure |
|---|---|---|---|
| 34 | 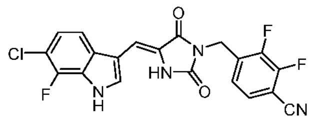 | 81 | 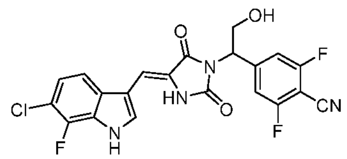 |
| 35 | 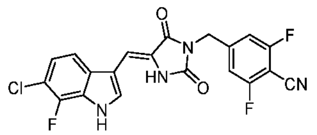 | 82 | 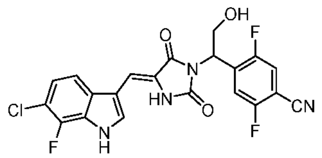 |
| 36 | 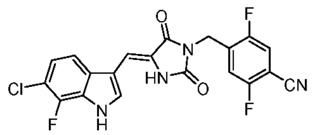 | 83 | 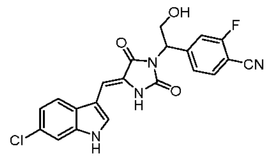 |
| 37 | 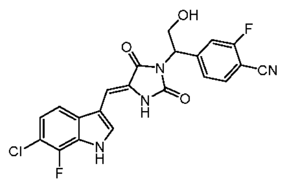 | 84 | 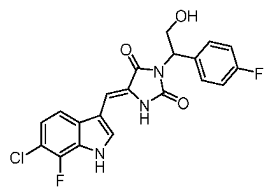 |
| 38 | 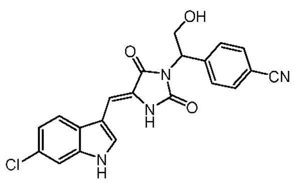 | 85 | 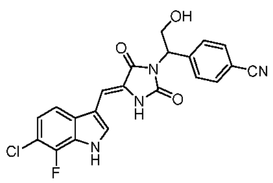 |
| 39 | 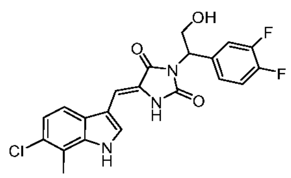 | 86 | 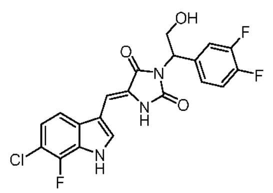 |
| 40 | 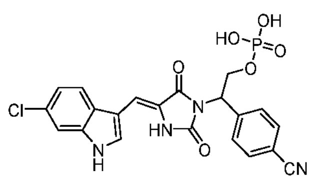 | 87 | 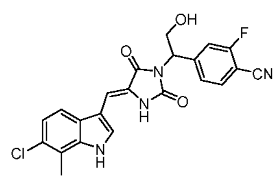 |
| 41 | 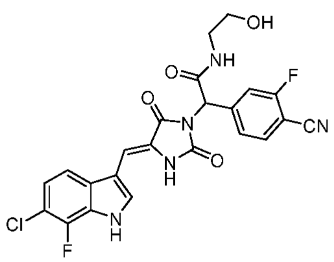 | 88 | 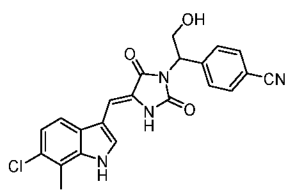 |

(continued)

| Number | Structure | Number | Structure |
| --- | --- | --- | --- |
| 42 | 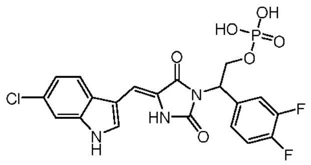 | 89 | 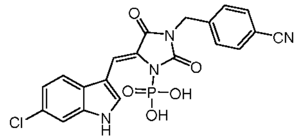 |
| 43 | 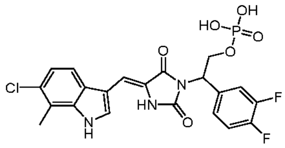 | 90 | 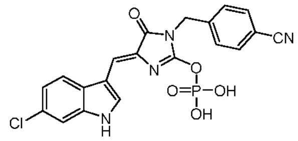 |
| 44 | 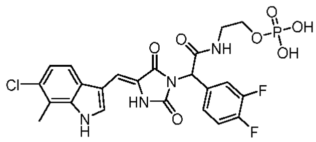 | 91 | 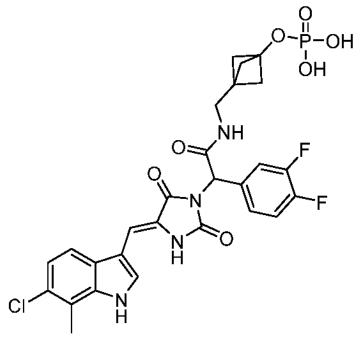 |
| 45 | 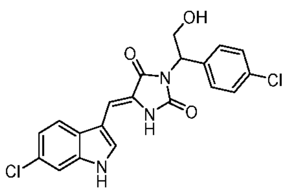 | 92 | 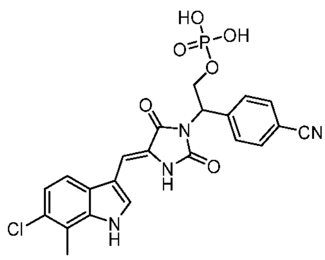 |
| 46 | 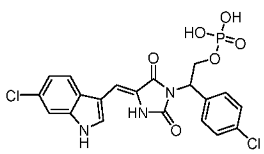 | 93 | 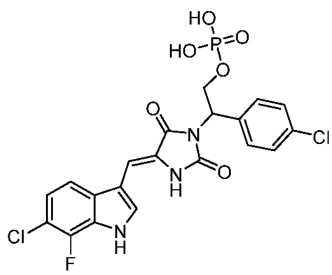 |
| 47 | 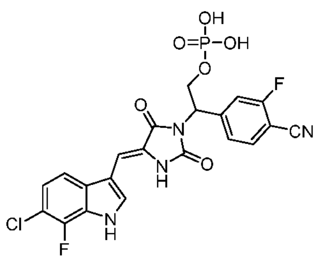 | 94 | 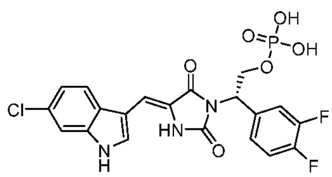 |

(continued)

| Number | Structure | Number | Structure |
|---|---|---|---|
| 95 | 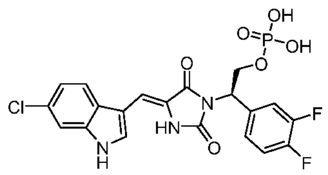 | 96 | 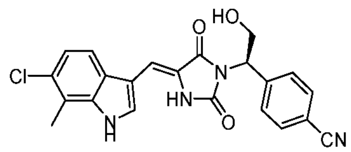 |
| 97 | 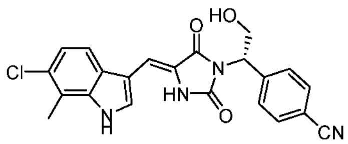 | 98 | 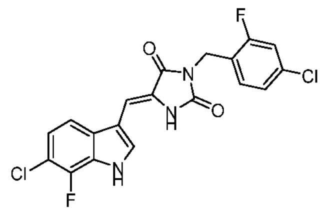 |
| 99 | 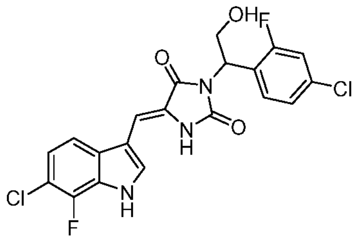 | 100 | 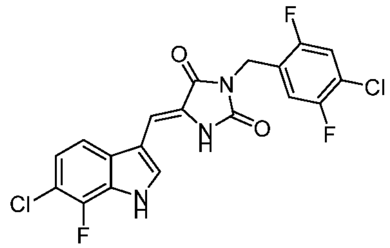 |
| 101 | 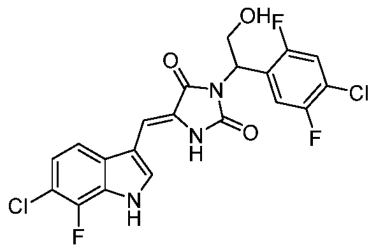 | 102 | 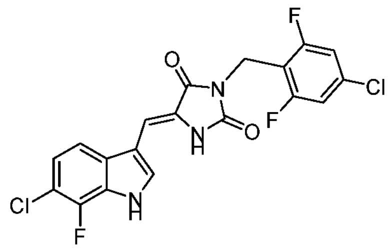 |
| 103 | 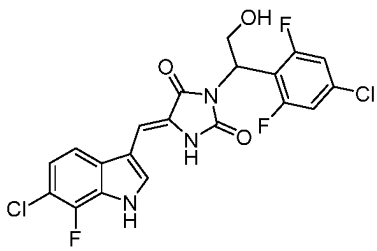 | 104 | 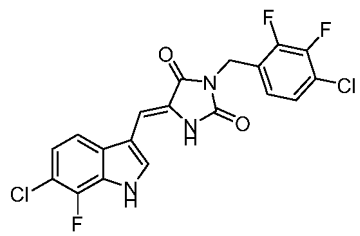 |
| 105 | 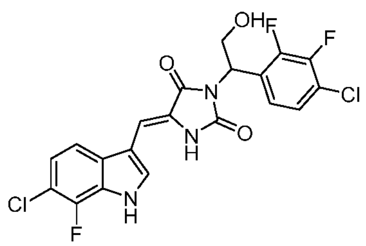 | 106 | 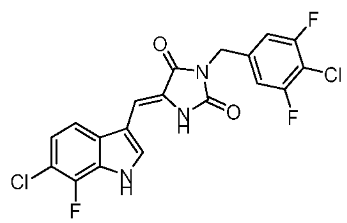 |

(continued)

| Number | Structure | Number | Structure |
|---|---|---|---|
| 107 | 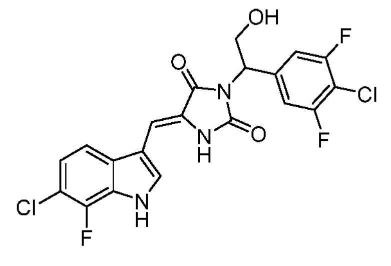 | 108 | 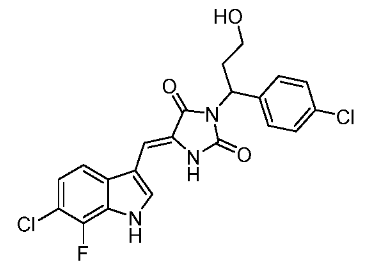 |
| 109 | 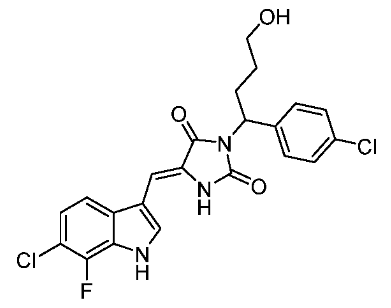 | 110 | 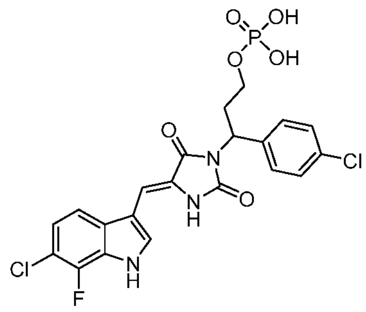 |

**7.** A method for preparing the compound according to any one of claims 1 to 6, comprising a method I and/or a method II: method I: performing a reaction on a compound a and a compound b to obtain the compound shown in formula (I);

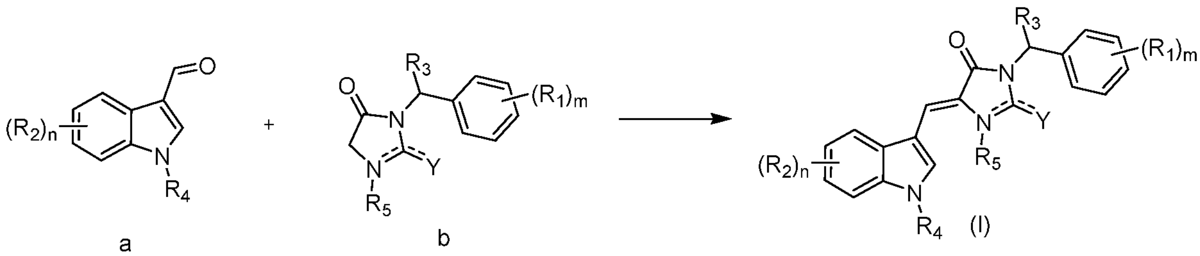

a b (I)

wherein $R_1$, $R_2$, $R_3$, $R_4$, $R_5$, Y, m, and n independently have definitions according to any one of claims 1 to 6; method II: performing a reaction on a compound c and $R_{3b}$-$NH_2$ to obtain the compound shown in formula (I);

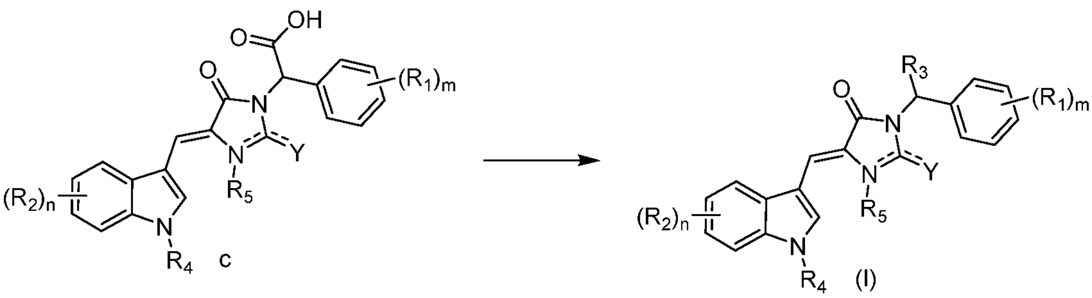

c (I)

wherein $R_1$, $R_2$, $R_3$, $R_4$, $R_5$, $R_{3b}$, Y, m, and n independently have definitions according to any one of claims 1 to 6.

**8.** A pharmaceutical composition, comprising a therapeutically effective amount of at least one of the compound, and the raceme, the stereoisomer, the tautomer, the isotopic label, the solvate, the polymorph, the pharmaceutically acceptable salt or the prodrug compound thereof according to any one of claims 1 to 6.

**9.** Use of at least one of the compound, and the raceme, the stereoisomer, the tautomer, the isotopic label, the solvate, the polymorph, the pharmaceutically acceptable salt or the prodrug compound thereof according to any one of claims

1 to 6 in preparation of a drug, wherein

preferably, the use is use in preparation of a drug for treating an MDM2 and/or MDMX-mediated symptom and/or disease, for example, use in preparation of an MDM2 and/or MDMX inhibitor drug; and
preferably, the use is use in preparation of a drug for treating idiopathic pulmonary fibrosis.

**10.** The use according to claim 9, **characterized in that** the disease is a neoplastic disease, and the neoplastic disease comprises at least one of leukemia, chronic myeloid leukemia, acute myelocytic leukemia, lymphocytic leukemia, hairy cell leukemia, T lymphoblastic leukemia, lymphoma, B-cell lymphoma, burkitt lymphoma, hodgkin lymphoma, chondrosarcoma, fibrosarcoma, ewing sarcoma, rhabdomyosarcoma, small cell lung cancer, non-small cell lung cancer, myeloma, glioma, prostatic cancer, breast cancer, bone cancer, neuroblastoma, gastric cancer, ovarian cancer, intestinal cancer, renal cancer, medulloblastoma, pancreatic cancer, thyroid cancer, mesothelioma, cervical cancer, bladder cancer, liver cancer, skin cancer, and tumors in the central system or the peripheral nervous system.

**INTERNATIONAL SEARCH REPORT**

International application No.
**PCT/CN2023/131362**

**A. CLASSIFICATION OF SUBJECT MATTER**

C07D403/06(2006.01)i; A61K31/4178(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D403/-, A61K31/-

Documentation searched other than minimum documentation to the extent that such documents are included in the fields searched

Electronic data base consulted during the international search (name of data base and, where practicable, search terms used)

CNTXT, CNKI, VEN, STN(REG, CAPLUS): 咪唑烷, 吲哚, 苯基, 二酮, MDM2, MDMX, imidazol, dione, indol, phenyl, 根据权利要求1中的式(I)进行了子结构检索, substructure search performed on the basis of formula (I) in claim 1

**C. DOCUMENTS CONSIDERED TO BE RELEVANT**

| Category* | Citation of document, with indication, where appropriate, of the relevant passages | Relevant to claim No. |
|---|---|---|
| X | CN 105985324 A (CHINA PHARMACEUTICAL UNIVERSITY) 05 October 2016 (2016-10-05)<br>embodiments 19, 20, and 24-26, and description, pages 2, 3, and 6-9 | 1-10 |
| X | Rezk, Mohamed Salah et al. "Synthesis and optimization of new 3,6-disubstitutedindole derivatives and their evaluation as anticancer agents targeting the MDM2/MDMx complex" *Chemical & Pharmaceutical Bulletin (2016),*<br>Vol. 64, No. 1, 31 December 2016 (2016-12-31), 34-41<br>table 1 | 1-10 |
| X | WO 2017117430 A1 (SCRIPPS RESEARCH INSTITUTE) 06 July 2017 (2017-07-06)<br>claim 26 | 1-5 |
| X | CN 110623956 A (BUCK INSTITUTE FOR RESEARCH ON AGING et al.) 31 December 2019 (2019-12-31)<br>claim 12 | 1-5, 9 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 February 2024** | **04 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/131362**

| Patent document cited in search report | | | Publication date (day/month/year) | Patent family member(s) | | | Publication date (day/month/year) |
|---|---|---|---|---|---|---|---|
| CN | 105985324 | A | 05 October 2016 | None | | | |
| WO | 2017117430 | A1 | 06 July 2017 | US | 2021393557 | A1 | 23 December 2021 |
| | | | | EP | 3397256 | A1 | 07 November 2018 |
| | | | | EP | 3397256 | A4 | 11 December 2019 |
| | | | | US | 2019008809 | A1 | 10 January 2019 |
| | | | | US | 11135186 | B2 | 05 October 2021 |
| CN | 110623956 | A | 31 December 2019 | AU | 2022256167 | A1 | 19 January 2023 |
| | | | | WO | 2015116740 | A1 | 06 August 2015 |
| | | | | JP | 2020105213 | A | 09 July 2020 |
| | | | | RU | 2016134429 | A | 01 March 2018 |
| | | | | RU | 2016134429 | A3 | 01 October 2018 |
| | | | | RU | 2716256 | C2 | 11 March 2020 |
| | | | | US | 2018303828 | A1 | 25 October 2018 |
| | | | | US | 10478433 | B2 | 19 November 2019 |
| | | | | US | 2017326136 | A1 | 16 November 2017 |
| | | | | US | 10130628 | B2 | 20 November 2018 |
| | | | | JP | 2021138712 | A | 16 September 2021 |
| | | | | KR | 20160117519 | A | 10 October 2016 |
| | | | | KR | 102496146 | B1 | 06 February 2023 |
| | | | | HK | 1231392 | A1 | 22 December 2017 |
| | | | | US | 2019343832 | A1 | 14 November 2019 |
| | | | | US | 2018250296 | A1 | 06 September 2018 |
| | | | | US | 10517866 | B2 | 31 December 2019 |
| | | | | US | 2021169876 | A1 | 10 June 2021 |
| | | | | EP | 3099380 | A1 | 07 December 2016 |
| | | | | EP | 3099380 | A4 | 27 September 2017 |
| | | | | EP | 3099380 | B1 | 11 August 2021 |
| | | | | AU | 2015211021 | A1 | 01 September 2016 |
| | | | | AU | 2015211021 | B2 | 02 July 2020 |
| | | | | JP | 2019142933 | A | 29 August 2019 |
| | | | | AU | 2020244600 | A1 | 05 November 2020 |
| | | | | MX | 2016009753 | A | 07 July 2017 |
| | | | | US | 2016339019 | A1 | 24 November 2016 |
| | | | | US | 2017224680 | A2 | 10 August 2017 |
| | | | | US | 9993472 | B2 | 12 June 2018 |
| | | | | US | 2021030752 | A1 | 04 February 2021 |
| | | | | US | 2018256568 | A1 | 13 September 2018 |
| | | | | US | 10413542 | B2 | 17 September 2019 |
| | | | | EP | 3669881 | A1 | 24 June 2020 |
| | | | | EP | 3669881 | B1 | 30 March 2022 |
| | | | | CA | 3100140 | A1 | 06 August 2015 |
| | | | | CA | 3100140 | C | 24 October 2023 |
| | | | | JP | 2023126918 | A | 12 September 2023 |
| | | | | US | 2017209435 | A1 | 27 July 2017 |
| | | | | US | 9855266 | B2 | 02 January 2018 |
| | | | | US | 2017198253 | A1 | 13 July 2017 |
| | | | | US | 10213426 | B2 | 26 February 2019 |
| | | | | IL | 286427 | A | 31 October 2021 |
| | | | | US | 2017196857 | A1 | 13 July 2017 |
| | | | | US | 9849128 | B2 | 26 December 2017 |

Form PCT/ISA/210 (patent family annex) (July 2022)

**INTERNATIONAL SEARCH REPORT**
**Information on patent family members**

International application No.
**PCT/CN2023/131362**

| Patent document cited in search report | Publication date (day/month/year) | Patent family member(s) | | | Publication date (day/month/year) |
|---|---|---|---|---|---|
| | | US | 2020030323 | A1 | 30 January 2020 |
| | | US | 11351167 | B2 | 07 June 2022 |
| | | US | 2019022090 | A1 | 24 January 2019 |
| | | US | 10328073 | B2 | 25 June 2019 |
| | | NZ | 723035 | A | 01 July 2022 |
| | | JP | 2017532285 | A | 02 November 2017 |
| | | JP | 6688224 | B2 | 28 April 2020 |
| | | IL | 246954 | A0 | 29 September 2016 |
| | | IL | 246954 | B | 01 February 2022 |
| | | US | 2017196858 | A1 | 13 July 2017 |
| | | CA | 2939121 | A1 | 06 August 2015 |
| | | CA | 2939121 | C | 24 November 2020 |
| | | SG | 10201805670 | QA | 30 August 2018 |
| | | BR | 112016017564 | A2 | 03 October 2017 |
| | | BR | 112016017564 | A8 | 17 April 2018 |
| | | MX | 2021012547 | A | 12 November 2021 |
| | | US | 2021169875 | A1 | 10 June 2021 |
| | | SG | 11201606239 | UA | 30 August 2016 |
| | | US | 2018117038 | A1 | 03 May 2018 |
| | | US | 10010546 | B2 | 03 July 2018 |
| | | US | 2018235956 | A1 | 23 August 2018 |
| | | US | 10258618 | B2 | 16 April 2019 |
| | | US | 2017348307 | A1 | 07 December 2017 |
| | | US | 9980962 | B2 | 29 May 2018 |
| | | US | 2018235957 | A1 | 23 August 2018 |
| | | US | 10478432 | B2 | 19 November 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211419976